# EUROPEAN PATENT APPLICATION

(11) **EP 2 199 797 A1**
(43) Date of publication of application: **23.06.2010**
(21) Application number: 08172133.4
(22) Date of filing: 18.12.2008
(51) Int. Cl.: G01N 33/569

(54) **Antigenic polypeptides for the detection of chlamydia-related bacteria and tests of diagnosis**

(71) Applicant: University of Lausanne, 1005 Lausanne (CH)
(72) Inventor: Greub, Gilbert, 1073 Mollie-Margot (CH); Raoult, Didier, 13008 Marseille (FR); Kebbi Beghdadi, Carole, 1007 Lausanne (CH); Riederer, Beat, 1066 Epalinges (CH)
(74) Representative: Schneiter, Sorin

(57) **Abstract**

The present invention relates to the disclosed transgenic peptides for use in the diagnosis of an infection by intracellular *Chlamydia-like* bacteria. The invention also relates to a serological diagnostic test. The transgenic peptides are selected from the proteome of *Parachlamydia acanthamoebae* properties of binding to antibodies of infected human and animals. The test may give further insight in the role of this microorganism in pulmonary diseases and possibly in miscarriage.

## Description

### Technical Field

The present invention generally relates to the fields of diagnosis, microbiology, immunology and more specifically to the use of recombinant and/or isolated peptides of the invention in the diagnosis of an infection by a *Chlamydia*-like microorganism, to a test for diagnosis, and to a method of diagnosis.

### Prior Art and the Problem Underlying the Invention

Novel chlamydiae, amoebae-resisting bacteria, *Chlamydia*-like organisms, and *Chlamydia-*related bacteria are various acronyms used to refer to a large variety of strict intracellular bacteria belonging to the *Chlamydiales* order, but exhibiting enough biological differences with *Chlamydiaceae* to be assigned to other families. The biodiversity of these *Chlamydia-*related bacteria, as evidenced by both molecular-based studies and culture-based studies, led to the proposal of several new families, genus, species and *Candidatus* species.

*Parachlamydia acanthamoebae* strains have been first identified within *Acanthamoeba* amoebae isolated from the nasal mucosa of healthy volunteers. The role of these free-living amoebae as a widespread aquatic reservoir has thus been suspected early on.

By analogy with *Legionella, P. acanthamoebae* may have used amoebae as an evolutionary crib to select virulence traits, explaining its adaptation to survive within macrophages. Moreover, amoebae have likely played a pivotal role in the exchange of genes between *Rickettsiales* and *Chlamydiales,* as suggested by the occurrence of a similar *tra* operon in both clades.

The life cycle of *P. acanthamoebae* in *Acanthamoeba* has been thoroughly studied, demonstrating that *Parachlamydia* elementary bodies generally enter passively by phagocytosis before differentiating in reticulate bodies that may divide by binary fission, before re-differentiating in elementary bodies. Bacteria are then released from amoebae within expelled vesicles or after amoebal lysis. Crescent bodies of *P. acanthamoebae,* another infectious stage, may be observed (i) outside of the amoebae in the process of being internalized by phagocytosis or (ii) late in the intra-amoebal cycle, at a time when the amoeba is mainly filled with elementary bodies. These crescent bodies have also been observed among other *Parachlamydiaceae,* among *Waddliaceae* and to a lesser extend among *Criblamydiaceae* and *Simkaniaceae.* A peculiar composition of the cell membrane of these novel chlamydiae likely explains this special cell morphology.

The prevalence of human lung infections due to *Parachlamydia* is currently unknown. It may well be underestimated since these fastidious intracellular bacteria can only be cultivated from clinical samples using amoebal culture, a procedure not routinely performed in diagnostic laboratories. Human exposure to these *Parachlamydiaceae* is supported by the amplification of parachlamydial DNA from nose and/or throat swabs and recovery of two *Parachlamydia* strains from amoebae isolated from the nasal mucosa.

There are, however, several lines of evidence supporting the medical importance of P. *acanthamoebae* (Greub G, Raoult D. Parachlamydiaceae: potential emerging pathogens. Emerg Infect Dis 2002 Jun;8(6):625-30). The first hint was the identification of P. *acanthamoebae* strain Hall's coccus within an amoeba isolated from the water of a humidifier linked to an outbreak of fever (Birtles RJ, Rowbotham TJ, Storey C, Marrie TJ, Raoult D. Chlamydia-like obligate parasite of free-living amoebae. Lancet 1997 Mar 29;349(9056):925-6). This humidifier was only investigated for the presence of amoebae given the high attack rate observed and given the recognized role of amoebae in the epidemiology of *Legionella,* whose pathogenic role was also initially documented in a similar fashion during the original outbreak in 1976.

Several serological studies have suggested a role for *P. acanthamoebae* in community-acquired pneumonia. In one of these studies, 8 (2.2%) of 371 patients with community-acquired pneumonia exhibited a positive *Parachlamydia* serology as compared to 0 of 511 healthy controls (p<0.01) (Marrie TJ, Raoult D, La Scola B, Birtles RJ, de Carolis E. Legionella-like and other amoebal pathogens as agents of community-acquired pneumonia. Emerg Infect Dis 2001 Nov-Dec;7(6):1026-9). In this study, 2 patients presented serological evidence of acute parachlamydial infection: a 68 year-old renal transplant recipient and a 21 year-old man with adult-onset Kawasaki disease. Greub G, Boyadjiev I, La Scola B, Raoult D, and Martin C. (Serological hint suggesting that Parachlamydiaceae are agents of pneumonia in polytraumatized intensive care patients. Ann N Y Acad Sci 2003 Jun;990:311-9) also identified a possible role of *Parachlamydia* in aspiration pneumonia. Indeed, while investigating patients hospitalized on a neurosurgical ward for a possible association of *P. acanthamoebae* infection with nosocomial infection, we unexpectedly identified 3 seroconversions, which all occurred in patients with aspiration pneumonia.

The identification of 16SrRNA gene sequences of *Parachlamydiaceae* from bronchoalveolar lavage fluid and sputum are additional hints for a pathogenic role of *P. acanthamoebae* in lower respiratory tract infections. Parachlamydial DNA was also amplified from monocytes taken from a patient with bronchitis (Ossewaarde JM, Meijer A. Molecular evidence for the existence of additional members of the order Chlamydiales. Microbiology 1999 Feb;145 ( Pt 2):411-7). Finally, *P. acanthamoebae* was amplified from nasopharyngeal swabs taken from children suffering from bronchiolitis of unexplained etiology (Casson N, Posfay-Barbe KM, Gervaix A, Greub G. A new diagnostic real-time PCR for the specific detection of P. acanthamoebae DNA in clinical samples. J Clin Microbiol 2008 Jan 30).

In summary, a possible role is emerging for *Parachlamydia* as agent of bronchitis, bronchiolitis, community-acquired pneumonia and aspiration pneumonia.

When further investigating the pathogenic role of *P. acanthamoebae,* its ability to enter within human macrophages was found. This strict intracellular bacterium is able to enter and replicate within human monocyte-derived macrophages (Greub G, Mege JL, Raoult D. P. acanthamoebae enters and multiplies within human macrophages and induces their apoptosis Infect Immun 2003 Oct;71(10):5979-85), representing a good example of the adaptation of an amoebae-resisting *Chlamydiae* to macrophages. However, contrary to *Chlamydiaceae* that mainly survive within the target cells by relocalizing in vacuoles associated with the exocytic pathway, *P. acanthamoebae* was able to survive within endocytic acidic vacuoles, by preventing the acquisition of cathepsin, a lysosomal hydrolase (Greub G, Mege JL, Gorvel JP, Raoult D, Meresse S. Intracellular trafficking of Parachlamydia acanthamoebae. Cell Microbiol 2005 Apr;7(4):581-9). Moreover, *P. acanthamoebae* did not induce significant cytokine production and remain partially unrecognized by human macrophages (Greub G, Desnues B, Raoult D, Mege JL. Lack of microbicidal response in human macrophages infected with Parachlamydia acanthamoebae. Microbes Infect 2005 Apr;7(4):714-9). However, parachlamydial macrophage infection was only partially successful since this strict intracellular bacteria only replicated to a low extent and induced the apoptosis of the macrophage (Greub et. al, Infect Immun 2003), thereby preventing its use as a replicative niche.

The permissiveness of lung fibroblasts and pneumocytes, and the absence of cytopathic effect on these cells were consequently of importance, since these lung cells may allow sustained parachlamydial viability for prolonged periods (Casson N, Medico N, Bille J, Greub G. Parachlamydia acanthamoebae enters and multiplies within pneumocytes and lung fibroblasts. Microbes Infect 2006 Apr;8(5): 1294-300).

Based on these *in vitro* studies, it was hypothesized that (i) *P. acanthamoebae* escape innate immune defenses by inducing macrophage apoptosis and via an unknown mechanism preventing cytokines release by these pivotal phagocytic cells, (ii) *P. acanthamoebae* may disseminate to other organs when internalized in monocytes, and (iii) *P. acanthamoebae* may cause lung infections by replicating within pneumocytes.

The etiological role of *P. acanthamoebae* in human pneumonia based on clinical observations and *in vitro* studies has been further investigated with a mouse model of pneumonia (Casson N, Entenza JM, Borel N, Pospischil A, Greub G. Murine model of pneumonia caused by Parachlamydia acanthamoebae. Microbial Pathogenesis 2008 Aug;45(2):92-7. In this work, Balb-C mice inoculated intratracheally with 2.5 x 10⁸ bacteria lost approx. 20% of their body weight in 48 hours concomitant with the development of severe pneumonia. In the absence of therapy, mortality due to the *P. acanthamoebae* pneumonia was 80% after 6 days. Pneumonia was initially purulent with a predominantly neutrophilic alveolar infiltrate, which later contained mainly macrophages. Of note, infection of both macrophages and pneumocytes could be documented *in vivo.* Moreover, pneumonia was also observed after intranasal administration of a similar inoculum (Casson N, Klos, A.;Stehle, J-C.;Pusztaszeri M.; Greub G.;. Intranasal murine model of infections by Parachlamydia acanthamoebae. Proceedings of the Annual Meeting of the Swiss Society for Infectious Diseases 2008:17) These results demonstrate that *P. acanthamoebae* may cause a severe pneumonia in experimentally infected mice, thus fulfilling the 3^{rd} and 4^{th} Koch criteria for a pathogenic role of this intracellular bacterium.

In view of the above, it is an objective of the present invention to provide diagnostic tools to further precise the role of the obligatory intracellular bacteria of the genus *Parachlamydia* as human and/or animal pathogens.

It is a further objective to determine more accurately if *Parachlamydia* strains are agents of one or more lower respiratory tract diseases, and if they can cause one or more of bronchitis, bronchiolitis, community-acquired pneumonia and aspiration pneumonia.

It is a further objective of the present invention to provide a diagnostic tool for determining the presence or absence of an infection by a *Parachlamydia* strain. Preferably, such a diagnostic tool is specific to the genus *Parachlamydia* and/or even specific for *P. acanthamoebae.* Accordingly, the diagnostic tool is able to detect infection with *Parachlamydia* and to allow the conclusion that the infection is not due to another organism, in particular another Chlamydia-like organism outside the *Parachlamydiacae* family. There is an objective of avoiding false-positives in a method of diagnosis and/or a diagnostic tool.

It is a further objective of the invention to provide a diagnostic tool and/or a method of diagnosis that is sensitive and thus also detects low infection levels.

It is noted that the development of a diagnostic tool is complicated by the fact that it has so far not been possible to isolate parachlamydial strains from human samples, including samples from which parachlamydial DNA was amplified. This is likely due to the obligatory intracellular live of these strains, which is specific to species of amoeba and/or to certain types of cells. *Parachlamydia* does not or only slowly grow in most available model cells, such as eucaryotic cell lines Vero, McCoy and HeLa, or other cells, such as A549 pneumocytes and lung fibroblasts.

It is an objective of the present invention to provide a serological test for the determination of an infection by *Parachlamydia.* More specifically, it is an objective to provide an Enzyme-linked Immunosorbent Assay (ELISA) or a similar serological test.

It is a further objective of the present invention to provide an immunohistochemic assay, for example an epifluorescence immunoassay, and a method of detecting *Parachlamydia* or any *Chlamydia*-related bacteria in tissue samples.

It is also an objective of the present invention to provide antigen to which polyclonal and/or monoclonal antibodies may be raised and used to detect *Parachlamydia* and/or related bacteria in animal and/or humans tissues thanks to immunochemistry or similar approaches.

It is an objective of the present invention to provide immunogenic proteins that may be used in a vaccine.

### Summary of Invention

Remarkably, the present inventors identified immunogenic proteins of *Chlamydia*-like intracellular microorganisms. The proteins are useful in the diagnosis of an infection by *Parachlamydia,* advantageously by way of a serological test or using immunohistochemistry. For serological approaches, it is possible to rapidly test an individual for the presence of infection with *Parachlamydia* on the basis of a small blood sample, for example.

Accordingly, the present invention provides, in a first aspect, the use of at least one peptide in the diagnosis of an infection by an intracellular microorganism.

In a second aspect, the present invention provides the use of at least one recombinant immunogenic peptide in the diagnosis of an infection by an intracellular *Chlamydia*-like microorganism.

In a third aspect, the present invention provides the use of at least one isolated immunogenic peptide in the diagnosis of an infection by an intracellular *Chlamydia*-like microorganism.

In a fourth aspect, the present invention provides a test of diagnosis comprising one or more isolated and/or recombinant immunogenic peptides of *Parachlamydia acanthamoebae.*

In a fifth aspect, the present invention provides a method of producing an antibody, the method comprising the steps of exposing a mammal to a peptide comprising: (a) an amino acid sequence according to any one of SEQ ID NO: 1-55; (b) a variant of an amino acid sequence as defined under (a); and/or (c) a fragment of an amino acid sequence as defined under (a) and/or (b); and, harvesting said antibody from serum of said mammal.

In a sixth aspect, the present invention provides a method of diagnosing infection of an individual by intracellular *Chlamydia*-like organisms, the method comprising the steps of contacting a blood or serum sample of an individual with one or more recombinant and/or isolated immunogenic peptide of the *Chlamydia*-like organism; determining the presence or absence in said sample of an antibody specifically binding to at least one of said immunogenic peptides; and diagnosing an infection if an antibody is detected in the previous step.

In a seventh aspect, the present invention provides (a) a peptide comprising an amino acid sequence according to any one of SEQ ID NO: 1-55; (b) of a variant of a peptide as defined under (a); and/or (c) a fragment of a peptide as defined under (a) or (b).

In another aspect, the present invention provides a nucleotide sequence encoding a peptide, variant peptide or fragment peptide as described herein. For examples, the nucleotide sequence may be a DNA or an RNA sequence.

In an aspect, the present invention further relates to the use of peptides disclosed herein in the diagnosis of diseases of the lungs and the respiratory tract (pulmonary diseases), such as bronchitis, bronchiolitis, and pneumonia, in particular community-acquired and/or aspiration pneumonia.

In yet another aspect, the present invention further provides a test designed to assess a risk of miscarriage. In particular, in a further aspect, the present invention provides a test for identifying a risk of adverse pregnancy outcomes such as miscarriage.

Further aspects and preferred embodiment of the present invention are as provided in the appended claims.

In the drawings,

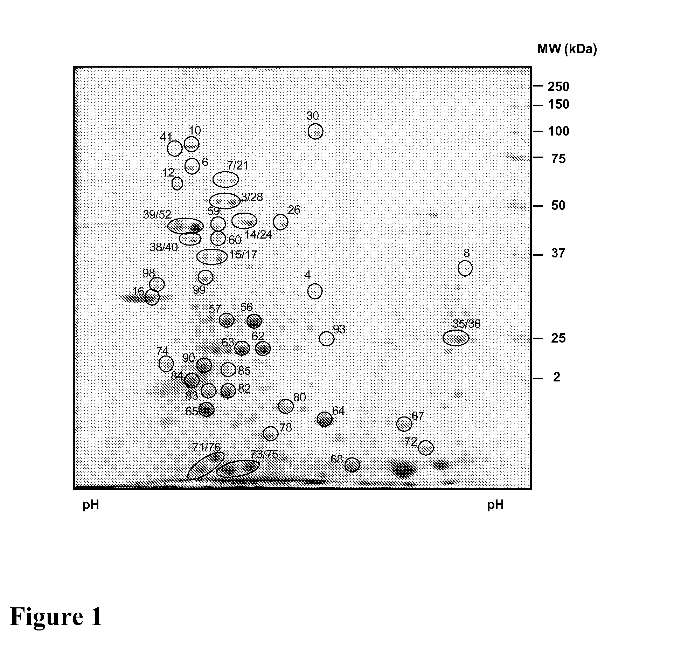

**Figure 1** shows a 2-dimensional, Coomassie blue stained SDS PAGE of a crude extract of *Parachlamydia acanthamoebae.* The extract was separated using a pH 3-11 NL IPG strip in the first dimension followed by a 12.5% SDS PAGE in the second dimension. Specific spots of antigenic peptides are encircled and numbered as described in the examples.

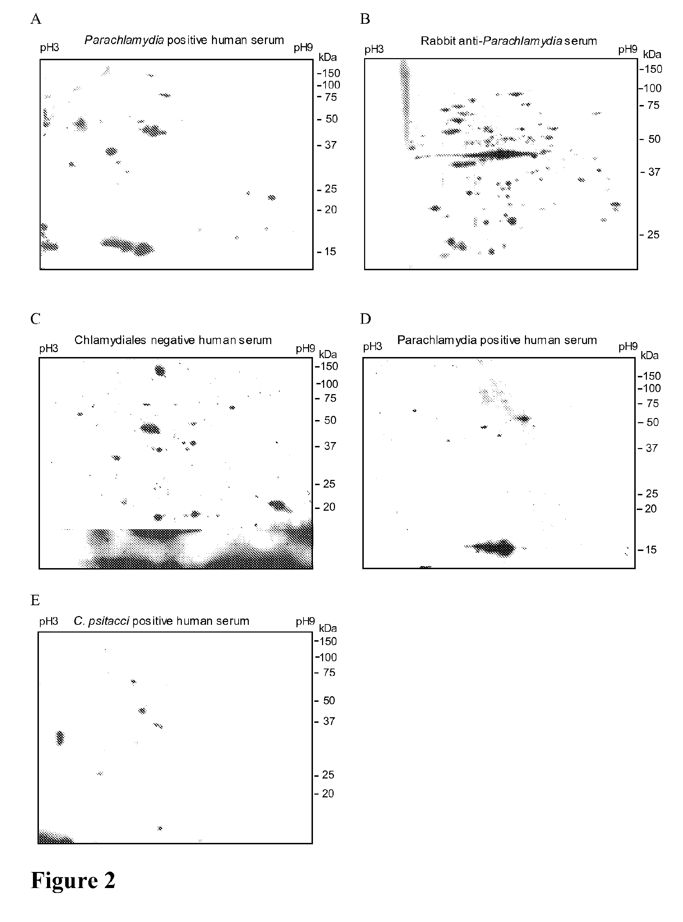

**Figures 2A-E** show western blots of parachlamydial proteins exposed to (A) and (D) sera of two human patients tested positive for *Parachlamydia;* (B) serum of a rabbit immunized with *Parachlamydia;* (C) serum of a human individual tested negative for Chlamydiales, and (E) serum of a human patient tested positive for infection by *Chlamydia psitacci.*

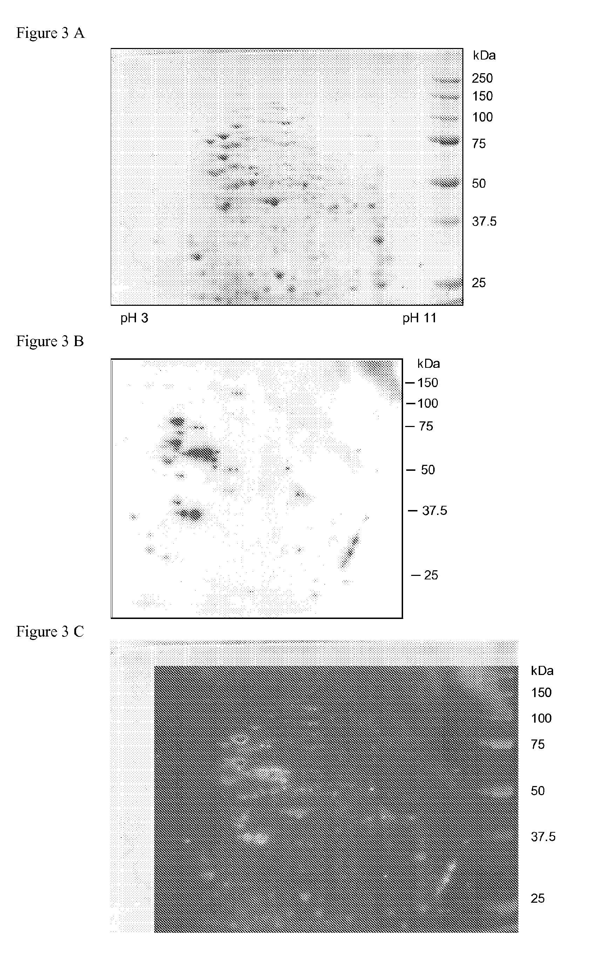

**Figures 3A-C** illustrate the method for detecting immunogenic proteins from a *Chlamydia-*like organism. Figure 3A is a 2D Coomassie blue stained SDS PAGE of a *Parachlamydia acanthamoebae* proteome (similar to Figure 1 but with spots not yet being numbered); Figure 3B is a western blot obtained with human *Parachlamydia* positive serum; and Figure 3C is the superposition of A and B, which allows to identify immunogenic proteins.

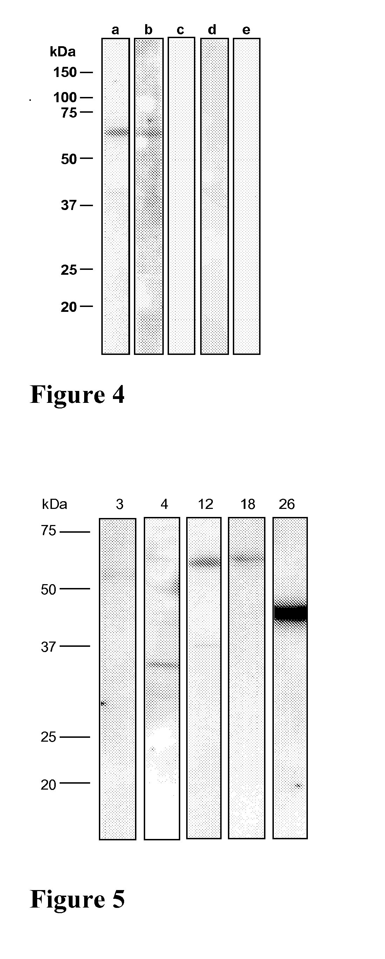

**Figure 4** shows western blots recognizing a recombinant protein according to the present invention. The protein corresponds to spot Pac12 in Table 1 and Figure 1 (SEQ ID NO: 9) and is blotted on a nitrocellulose membrane and probed, in lane a: with a serum from an immunized rabbit, in lane b: with a *Parachlamydia* positive serum (patient 3 in Table 1), in lane c: with *Chlamydiales* negative serum (individual 12 (N-C9) in Table 1), in lane d: C. *psitacci* positive serum (individual 18 in Table 1), in lane e: a *C. pneumoniae* positive serum (patient 15 in Table 1). Molecular mass standards (in kDa) are indicated on the left side.
**Figure 5** shows western blots recognizing recombinant proteins according to the present invention. The proteins correspond to spots 3, 4, 12, 18 and 26 in Table 1 and Figure 1 (SEQ ID NO:1, 2, 9, 16, and 17) and are blotted on a nitrocellulose membranes probed with a serum from an immunized rabbit.

### Detailed Description of the Preferred Embodiments

The present invention relates to the use of at least one peptide in diagnosis. Preferably, diagnosis is the diagnosis for infection by a *Chlamydia*-like microorganism. As indicated above, the term "*Chlamydia*-like" or *"Chlamydia-related"* microorganism, for the purpose of the present specification, encompasses a large variety of strict intracellular bacteria belonging to the *Chlamydiales* order, but which exhibit enough biological differences with *Chlamydiaceae* to be assigned to other families.

According to a preferred embodiment, the *Chlamydia*-like microorganism belongs to the family of *Prachlamydiaceae,* more preferably to the genus of *Parachlamydia,* and most preferably it is *Parachlamydia acanthamoebae.*

The present invention relates to uses, tests, diagnosis and methods. These are all interrelated such that reference herein to a specific test or use, for example, automatically also refers also to the methods and diagnosis disclosed herein, and *vice versa.*

According to an embodiment, the diagnosis and or tests disclosed herein are specific. "Specificity", for the purpose of the present specification, can be at the order level, at the family level, at the genus level and at the level of the species.

In one embodiment, the methods, tests and/or diagnosis as disclosed herein are specific at the level of the order, meaning that they allow diagnosis of infection by a given clade (i.e. any *Chlamydiales*), but does not yield a positive result when there is an infection, for example, of other Eubacteria. In other words, when the result of the diagnosis and/or the test is positive, it can be said that the positivity relates to an infection by a bacterial species of the order of the Chlamydiales, and not to an infection by a bacterium from another order. This does not exclude the possibility that an infection by such a bacterium of another order is also present, but it specifies that an infection by a bacterium that is part of this order is present.

According to an embodiment, the methods, tests and/or diagnosis disclosed herein are specific at the level of the family of the *Parachlamydiaceae.* In analogy of the before mentioned specificity with respect to the order, this means that the present invention allows the diagnosis of infection by any *Parachlamydiaceae,* but does not yield a positive result when there is an infection, for example, by a species belonging to the same order (*Chalmydiales*) but to another family than *Parachlamydiaceae.*

According to an embodiment, the methods, tests and/or diagnosis disclosed herein are specific at the level of the genus. In analogy to the paragraphs above, this means that the present invention allows the diagnosis of infection by any *Parachlamydia,* but does not yield a positive result when there is an infection, for example, of another genus of the family of the *Parachlamydiaceae.*

According to an embodiment, the methods, tests and/or diagnosis disclosed herein are specific at the level of the species any *P. acanthamoebae.* In analogy to the paragraphs above, this means that the present invention allows the diagnosis of infection by any strain belonging to the species of *P. acanthamoebae,* but does not yield a positive result when there is an infection, for example, of another species of the genus of *Parachlamydia*.

According to an embodiment, the diagnosis and/or the test disclosed herein are specific for an intracellular bacterial species.

Due to the possible role of *Chlamydia*-like microorganisms as agents of bronchitis, bronchiolitis, and pneumonia, in particular aspiration and/or community-acquired pneumonia, the present invention also relates to the diagnosis of these conditions and/or in the assessment of a risk and/or possibility of imminent or future contracting of one or more of these conditions.

A "peptide", for the purpose of the present specification, refers to a genus of peptide or peptide fragments that encompass the amino acid sequences identified herein, as well as smaller fragments. A peptide is preferably defined in terms of its antigenic relatedness to any peptide disclosed herein. Thus, in one embodiment, a peptide within the scope of the invention is defined as an amino acid sequence comprising a linear or 3-dimensional epitope shared with any amino acid sequence disclosed herein.

According to a preferred embodiment, a peptide within the scope of the present invention is recognized by an antibody that specifically recognizes and/or binds to any peptide having an amino acid sequence as disclosed in the present specification. Antibodies are defined to be specifically binding if they bind peptides of the invention with Kₐ of greater than or equal to about 10⁷ M⁻¹, such as greater than or equal to 10⁸ M⁻¹.

The peptide may be a polypeptide (protein) or an oligopeptide, having from 3-10 amino acids. The size of the peptide is not crucial, as long as it has the minimum size for providing an antigenic entity and/or an epitope, which will be specifically targeted by the immune system of a human or animal subject. Preferably, a peptide comprises a continuous amino acid sequence of at least 10 amino acids, more preferably at least 15 and most preferably at least 20 amino acids.

The term "to comprise" or "comprising", for the purpose of the present specification, is intended to mean "includes amongst other". It is not intended to mean "consists only of".

According to the invention, at least one peptide, as characterised by a specific amino acid sequence, is needed for the purpose of diagnosis, for example in the test of the present invention. Preferably, however, two or more different peptides are used, preferably three and most preferably four or more peptides. The use of a combination of a certain number of different, selected peptides reduces cross-reactivity and thereby enables to reduce the number of false positive and/or false negative diagnostic outcomes.

The present invention also relates to the production of an antibody by using the peptides of the invention as antigens. The antibody may be produced according to any currently known method. The antibody preferably specifically binds to a peptide of the present invention.

The peptide of the present invention is preferably a recombinant and/or an isolated peptide. A recombinant peptide can be produced by any suitable organism, for example a bacterium, an eucaryotic cell, a multicellular organism such as a transgenic plant or animal, for example. The recombinant peptide may be isolated or may not be. The recombinant peptide may, for example, be used in the form of an unpurified bacterial lysate, together with other cellular components.

The isolated peptide may be recombinant or may not be recombinant, for example isolated from the original organism. The peptide may be isolated according to any peptide and/or protein isolation and/or purification procedure.

The peptide is preferably selected from proteins with the amino acid sequences of SEQ ID NO: 1-55. Amino acid sequences SEQ ID NO:1-51 were selected on the basis of their antigenic properties. Epitopes present on these proteins are recognised by individuals tested positive for an infection by *Parachlamydia.* SEQ ID NO: 52-55 are determined from DNA sequences selected from the genome of the *P. acanthamoebae* Hall coccus strain established for the purpose of the present invention (Example 5). The sequences SEQ ID NO: 52-55 are thus selected by their sequence homology with proteins known as possible antigens in other organisms.

According to a preferred embodiment, the peptide comprises (a) an amino acid sequence selected from any one of SEQ ID NO:1-51, (b) a functional variant of an amino acid sequence selected from any one of SEQ ID NO: 1-55; and/or (c) an immunogenic fragment of an amino acid sequence of (a) and/or (b).

According to an embodiment, the peptide comprises (a) an amino acid sequence selected from any one of SEQ ID NO: 1, 2, 6, 7, 9, 13, 14, 15, 17, 18, 19, 22, 23, 21, 35, 39, (b) a variant thereof, or (c) a fragment of (a) and/or (b).

According to preferred embodiment, the peptide comprises (a) an amino acid sequence selected from any one of SEQ ID NO: 2, 6, 7, 9, 13, 14, 18, 23, 35, 39, (b) a variant thereof, or (c) a fragment of (a) and/or (b).

According to another preferred embodiment, the peptide comprises (a) an amino acid sequence selected from any one of SEQ ID NO: SEQ ID NO: 2, 6, 9, 18, 39, (b) a variant thereof, or (c) a fragment of (a) and/or (b). Preferably, the peptide comprises an amino acid sequence selected from SEQ ID NO: 2 and 9, a variant thereof, and/or a fragment of the sequence or of the variant sequence.

The embodiments detailed above related to preferred peptides as detailed in the examples, which are selected in order to make a diagnosis more specific with respect to the microorganism and/or more sensitive. They are also selected so as to avoid cross-reactivity.

The embodiments detailed above related to the preferred peptides relate to all uses, diagnosis, tests, assays and methods to disclosed herein to which such peptides apply. For example, the preferred peptides are also preferred with respect to the method of preparing an antibody, as disclosed further below.

Instead of the peptides as defined by an amino acid sequences according to any one of SEQ ID NO: 1-55, the peptide may comprising an amino acid sequence, which is a variant sequence of a sequence according to a any one of SEQ ID NO: 1-55 and/or the preferred peptides as detailed above.

A peptide comprising a variant amino acid sequence, also referred to as variant peptide herein, means a peptide having a substantially identical amino acid sequence as the native and/or originally defined peptide, but which amino acid sequence is different from that disclosed herein because of one or more amino acids are removed, are substituted by one or more other amino acids and/or because further and/or other amino acids are added. Any combinations of substituted, deleted and added amino acids are possible for the purpose of defining a variant peptide.

Variants can comprise conservatively substituted sequences, meaning that a given amino acid residue is replaced by a residue having similar physiochemical characteristics. Examples of conservative substitutions include substitution of one aliphatic residue for another, such as Ile, Val, Leu, or Ala for one another, or substitutions of one polyar residue for another, such as between Lys and Arg; Glu and Asp; or Gln and Asn. See Zubay, Biochemistry, Addison-Wesley Pub. Co., (1993). The effects of such substitutions can be calculated using substitution score matrices such as PAM-120, PAM-200, and PAM-250 as discussed in Altschul (J. Mol. Biol. 219:555-65, 1991). Other such conservative substitutions, for example, substitutions of entire regions having similar hydrophobicity characteristics, are well known. Naturally peptide variants are proteins that result from alternate mRNA splicing events or from proteolytic cleavage of the peptides described herein. Variants attribuatable to proeolysis include, for example, differences in the N- or C-termini upon expression in different types of host cells, due to proteolytic removal of one or more terminal amino acids from the polypeptides encoded by the sequences of the invention.

Fusion peptides and/or proteins are also considered as variant peptides for the purpose of the present invention. Fusions of additional peptide sequences at the amino and/or carboxyl terminal ends of the polypeptides of the invention may be used to enhance expression and/or extracellular secretion and/or may aid in the purification of the protein, for example. For example, peptides as defined herein further comprising a signal peptide and/or a his-tag or a different tag fulfilling any specific function are also encompassed by the present invention.

It is also possible to create a fusion protein containing a non-antigenic amino acid sequence combined with an amino acid sequence or fragment thereof as defined herein.

According to an embodiment, a variant peptide preferably is a at least 50%, 60%, 70%, 75%, 80%, 85%, 90%, 91%, 92%, 93%, 94%, 95%, 96%, 97%, 98% or 99% identical to an amino acid sequence selected from any one of SEQ ID NO: 1-55. The length of comparison sequences will generally be at least 10, 30, 30, 50, 100 or more amino acids. For example, the entire sequence can be compared.

The peptide of the present invention may be a fragment of a polypeptide according to anyone of SEQ ID NO: 1-55 disclosed herein, or of a variant peptide as defined above. A fragment peptide preferably has at least 10, 30, 30, 50, 100 or more amino acids. In principle, any fragment of said sequences or variants is encompassed by the present invention. Since the peptide is used in diagnosis and preferably in an antibody-based, serological test, the fragment is preferably sufficiently large to be recognized by an antibody that specifically recognizes and/or binds to the fragment having an amino acid sequence as defined herein. The fragment of the invention may be used as such or as a stretch of amino acids in a larger polypeptide and/or amino acid sequence. For example, the antigenic fragment may be combined with, for example fused to, a non-antigenic amino acid sequence. According to an embodiment, the fragment has 30 or more, preferably 40 or more and most preferably 50 or more amino acids, wherein "or more" includes lengths of up to the entire protein.

The present invention also relates to a test of diagnosis. The test is preferably a serological test, which means that a diagnosis can be made on the basis of a blood and/or serum sample of an individual. Preferably, a serum sample is used. Preferably, a sample comprises 5ml or less of blood and/or serum of an individual, for example 0.5ml or less. Preferably, the sample is diluted, the test being sufficiently sensitive to provide an accurate result with the diluted sample. For example, the dilution is at least 1 / 2, more preferably at least 1 / 4, and most preferably at least 1 / 8.

The test is preferably an antibody-based test, which means that the presence of antibodies specifically binding to a peptide as defined herein, said antibody being contained in the blood and/or serum sample of an individual, is detected, and from the presence of a sufficient amount of such antibody. The test may also contain one or more antibodies that specifically binds to a peptide as defined herein.

An example for a diagnostic test of the present invention is an Enzyme-Liked Immunosorbent Assay (ELISA). Another example are immunofluorescent strips as marketed by the company inoDiag in 83 870 Signes, France, under the trademark InoMu.S.T.®. These glass strips contain a spot, which is incubated with a diluted blood sample and with further reactants generally comprising an antibody selective for the constant domains of a specific human antibody type. Subsequently, the strip is fluorescence analysis in an automated process and the diagnosis is accomplished.

The use, test and the method of the present invention preferably comprises one or more isolated and/or recombinant immunogenic peptides of an intracellular bacterial strain of the genus *Parachlamydium*, preferably of the species *P. acanthamoebae,* and most preferably of the Hall *P. acanthamoebae* coccus strain.

The present invention also relates to a method of producing an antibody, the method comprising the steps of exposing a mammal to a peptide as disclosed herein, and, following exposure, harvesting polyclonal antibodies from the serum of said mammal. The peptide may be administered to the mammal as an isolated peptide, as a recombinant peptide, as an unpurified bacterial extract containing the peptide, for example, an unpurified lysate and/or bacterial extract of recombinant bacteria expressing said peptide. Preferably, one, two, three, four, five or more recombinant peptides are used for immunisation of the mammal. Preferably, the peptides or any composition comprising the peptides are administered by injection, for example subcutaneous injection. The peptide may be administered in the form of a composition, for example an aqueous solution comprising the peptide or a mixture of different peptides as defined and disclosed herein.

The present invention also provides a monoclonal antibody, wherein said antibody specifically binds to a peptide as defined herein, and also to a hybridoma producing the antibody. Methods for producing a hybridoma from myeloma cells and cells isolated from immunized individuals, such humans or animals, in particular mammals, in particular mice, rabbits, goats, for example, are known. With respect to the present invention, a mammal is immunized with an isolated and/or recombinant peptide as defined herein, or composition of two or more different peptides as defined herein. Specifically binding, for the purpose of the present invention, may be determined by immunofluorescence, western blotting, EPLISA and/or immunohistochemistry, while using the corresponding antigen.

The polyclonal and/or monoclonal antibodies referred to herein are preferably isolated antibodies.

The antibodies of the present invention may be used in immunohistochemical methods, assays and/or tests for detecting a *Chlamydia*-like microorganism in tissue, in particular tissue samples. These methods, assays and/or tests may comprise primary and secondary antibodies, wherein the primary antibody is the antibody of the present invention, and a secondary antibody is provided that specifically binds to said primary antibody and the presence of which secondary antibody can be visualized, for example by linking an enzyme to said secondary antibody and/or a fluorescent dye, as is conventional in immunohistochemistry.

The tissues and/or tissue samples mentioned herein are preferably prepared and/or treated so as to disrupt cell membranes, of the tissue and thereby expose antigens possibly present in the tissue. The tissues may also be treated so as to unmask the antigens in order to allow for better antibody-antigen recognition.

The peptides and test disclosed herein may be used in the diagnosis of an infection of *Chlamydia*-related organisms and/or in the diagnosis of pulmonary diseases. Preferably, a pulmonary disease, for the purpose of the present invention, is an infectious pulmonary disease. Since infection with *Parachlamydia* has been associated with miscarriage, the present invention is useful for assessing the risk of adverse pregnancy outcomes such as miscarriage.

The invention is now illustrated by way of the examples below, which are not intended do limit the scope of the present invention.

### Examples

### Patients

In the publication of G. Greub, I. Boyadjiev, B. La Scola, D. Raoult and C. Martin, "Serological Hint Suggesting That Parachlamydiaceae Are Agents of Pneumonia in Polytraumatized Intensive Care Patients", Ann.N.Y.Acad.Sci. 990: 330-319 (2003) sera taken of intensive-care patients and from healthy blood donors were tested for reactivity against *Parachlamydia* by immunofluorescence. On pages 312-313 the determination of infection by detection of specific antibodies against *Parachlamydia* is described in detail under "Serology". In five patients, infection by *Parachlamydia* was found. Sera of these five patients, and of two additional patients, in which anti-*Parachlamydia* antibodies could be identified in the same manner, were used in the examples below. In addition, sera were also taken from women with miscarriage and at term uneventful pregancy (Baud et al. Emerg. Infect. Dis. 2007, 13 (8), 1239-1243). Using a similar approach than that used in the above mentionned publication by Greub et al., infection by *Parachlamydia acanthamoebae* was documented in 7 patients. No cross-reactivity was found in sera taken from both work mentioned above.

### Example 1: Cultivation and purification of Parachlamydia acanthamoebae

A procedure for purifying *P. acanthamoebae* is described by G. Greub, J.-L. Mege and D. Raoult "Parachlamydia acanthamoebae Enters and Multiplies within Human Macrophages and Induces Their Apoptosis", Infection and Immunity, Oct. 2003, p. 5979-5985, more particularly on page 5979 under the title "Materials and methods" the paragraph "P. *acanthamoebae* culture and purification". This procedure was used with the exception that instead of *A. polyphaga,* the *A. castelanii* strain ATCC 30010 was used as a host. Furthermore, there was no tittering, lysis test and freezing conducted. Instead, the large lower band of *Parachlamydia* resuspended twice in PBS was subjected to 2D gel electrophoresis as reported below.

### Example 2: Crude extract sample preparation and 2-D gel electrophoresis

Purified bacteria (mostly elementary bodies, EB) were washed twice in 10mM Tris, 5mM MgAc, pH 8.0 and then lysed by 5 cycles of short-pulse sonication in lysis buffer ( 30 mM Tris, 7M urea, 2M Thiourea, 4% CHAPS, pH 8.5). Proteins were recovered by centrifugation at 8'0000 rpm and quantified using a Bradford assay (Quick Start^{™} Bradford Protein Assay, Bio-Rad laboratories, Hercules, USA). Routinely about 4 mg of total parachlamydial proteins were obtained from 60 T75 flasks of amoebal co-culture. Aliquots of 1.2 mg were stored at minus 80°C for subsequent electrophoretic analysis.

Two dimensional gel electrophoresis was performed as described by Centeno et al (Centeno et al., Cell Death and Differentiation, 2007, 14, p.240-253) using approximately 150 µg (mini gels) or 600 µg (midi-gels) of total EB proteins for each electrophoretic run. Proteins were visualized by Coomassie Blue staining or transferred to nitrocellulose. **Figure 1** shows the 2D gel obtained.

### Example 3: Immunoblot Analysts

Nitrocellulose membranes were blocked by 2 hours incubation with 5% non-fat dry-milk in Tris-buffered saline with 0.05% Tween 20 (TBS) , washed 3 times with TBS, 0.5% milk and incubated overnight at 4°C with sera diluted in TBS, 0.5% milk. Membranes were probed either with human sera (see "Patients" above (dilution 1/64) or with sera (dilution 1/25) of rabbits immunized 4 times with purified and heat-inactivated bacteria (Eurogentec standard protocol). After 3 subsequent washes with TBS, 0.5% milk, the membranes were probed with horseradish peroxidase-conjugated goat anti-human IgG (Chemicon,Temecula,CA, 1:5000), or anti-rabbit IgG (Cell Signaling, Allschwill, Switzerland, 1:1000). Membranes were then washed 3 more times with TBS and immunoreactive spots were detected with a chemiluminescence-based kit (LiteAblot™, Euroclone SpA, Pero, Italy).

**Figure 2** shows the result of immunoblot analysis obtained with sera as indicated. Serum of a patient negative by IF for all Chlamydiae tested: This blot shows cross-reactions with similar proteins in other organisms (par ex: ribosomal protein L7/L12, DnaK, HSP 60)

### Example 4: Selection of immunogenic proteins (antigens)

The Coomassie Blue stained gel and the immunoblots obtained after incubation with P. *acanthamoebae* positive sera or rabbit anti-P. *acanthamoebae* sera were overlapped using the Adobe Photoshop program to select spots corresponding to immunoreactive proteins.

This process is illustrated in **Figures 3A-C**, with Figure 3A being the 2D PAGE with *Parachlamydia* Comassie blue stained proteins and Figure 3B is a western blotting obtained with human *Parachlamydia* positive serum. Figure 3C is the overlap of Figs 3A and B, wherein, in the overlap, the spots obtained with human serum appear in blue.

The overlap procedure allows to differentiate sports of interest of the *Parachlamidia* proteome from non-antigenic spots. The spots of interest (antigen spots) were marked and numerated, as illustrated in Figure 1.

**Table 1,** annexed further below, contains the result of the overlap analysis of immunoblotting obtained with serum of different patients and immunized rabbits exposed to the western blot of the *P. acanthamoebae* proteome. The black filled cells in Table 1 indicate that the respective patient and/or rabbit produced an antibody against the respective protein, referred to by its spot number. Table 1 also lists results obtained with serum of patients that were not tested positive for *P. acanthamoebae*, but which were positive for other Chlamydiales, in particular *Chlamydia trachomatis* and/or *Chlamydia pneumoniae*. Furthermore, also the serum of an individual being negative for any *Chlamydia*-like organism was tested. The results of Table 1 thus allow identifying antigenic proteins of *P. acanthamoebae* that avoid cross-reactivity when exposed to serum of an individual infected with *C. trachomatis* and *C. pneumoniae*.

**Table 2** lists a selection of spots and the corresponding proteins as identified according to the procedure below. The listed antigenic proteins were recognized by serum of a rabbit immunized with *P. acanthamoebae.* For a test specific to *P. acanthamoebae,* spots are considered as "best/good candidates" when they were not recognized by serum of patients infected with *C. pneumoniae* or *C. psittacci* and are also not recognized by two control sera of patients negative for all *Chlamydiales* tested. These spots are listed on top in Table 2. These proteins are thus particularly useful in a serological test for infection by *Parachlamydia.*

**Table 2: Evaluation of some identified P. acanthamoebae immunogenic proteins for use in a serological diagnostic test.**

| Comments | Spot | SEQ ID NO: | Protein description |
|---|---|---|---|
| Best candidates | | | |
| | 12 | 9 | Protein of unknown function |
| | 4 | 2 | Protein of unknown function |
| Other good candidates | | | |
| | 30 | 18 | Putative manganese and iron superoxide dismutase |
| | 8 | 6 | Putative NAD(P)H-dependent glycerol-3-phosphate dehydrogenase |
| | 74 | 39 | Putative yciF protein |

| Similarity with proteins of other species | | | |
|---|---|---|---|
| | 10 | 7 | Molecular chaperone DnaK |
| | 41 | 23 | 30S ribosomal protein S1 |
| | 6 | 4 | Chaperonin GroEL |
| | 73 | 37 | Probable 50S ribosomal protein L7/L12 |
| | 14 | 13 | Elongation factor Tu |
| | 15 | 14 | Elongation factor Ts |
| | 71 | 35 | Co-chaperonin GroEs |

| Cross reaction with *C. pneumoniae, C. psitacci* or negative controls | | | |
|---|---|---|---|
| | 10 | 7 | Molecular chaperone DnaK |
| | 41 | 23 | 30S ribosomal protein S1 |
| | 6 | 4 | Chaperonin GroEL |
| | 73 | 37 | Putative 50S ribosomal protein L7/L12 |
| | 3 | 1 | Putative serine proteinase |
| | 14 | 13 | Elongation factor Tu |
| | 26 | 17 | Protein of unknown function |
| | 52 | 21 | Protein of unknown function |
| | 40 | 22 | Protein of unknown function |
| | 15 | 14 | Elongation factor Ts |
| | 16 | 15 | Putative mip |
| | 36 | 19 | Protein of unknown function |

### Example 5: Raw genome sequences data and ORFing

Recent advances in DNA sequencing such as 454 pyrosequencing and Solexa technologies provide much faster, and much better sequencing strategies. We thus use the GS20 sequencer (454 Life Sciences, Brandford, USA) to sequence the genome of *Parachlamydia acanthamoebae* strain Hall coccus. Two independent runs were performed to increase the coverage. In order to correct possible frame shifts due to homopolymers, we also sequenced the *Parachlamydia* genomic DNA using the Solexa technology in Illumina Genome analyzer (Illumina Inc, San Diego, USA). The assembly of all GS20 sequences was done using the Newbler software (Roche, Basel) with default parameters except for overlap size (45 nucleotides) and identity score (95%). Solexa sequences were assembled using the Edena software (Hernandez et al. Genome research 2008). Both assemblies were then merged by reassembling the GS20 sequences with contigs of Solexa'assembly with Newbler. Remaining differences were manually inspected and corrected when necessary.

Then, ORFing was performed using Glimmer v3.02 trained on published sequences of all published genomes of *Chlamydiales.* These ORFs were then used as input in the Mascot program to look for mass spectrometry hits (see below).

### Example 6: Protein identification by MALDI-MS/MS

The antigen spots were excised from the 2-D gel and subjected to mass spectrometry (MS) to definitively identify the proteins.

Spots excised from the 2-D gel were transferred to special 96-well plates (Perkin Elmer Life Sciences). In-gel proteolytic cleavage with sequencing-grade trypsin (Promega, Madison, WI, USA) was performed automatically in the robotic workstation Investigator ProGest (Perkin Elmer Life Sciences) according to the protocol of Shevchenko et al. (Shevchenko A, Wilm M, Vorm O, Mann M. Mass spectrometric sequencing of proteins silver-stained polyacrylamide gels. Anal Chem. 1996 Mar 1;68(5):850-8.). Digests were evaporated to dryness and resuspended in 3 ul alpha-cyano-hydroxycinnamic acid matrix (5 mg / ml in 60% (v :v) acetonitrile :water), of which 0.7 µl were deposed in duplicate on a target plate.

MALDI-MS-MS analysis was performed on a 4700 Proteomics Analyser (Applied Biosystems, Framingham, MA, USA). After MALDI-TOF MS analysis, internal calibration on trypsin autolysis peaks and subtraction of matrix peaks, the 10 most intense ion signals were selected for MS/MS analysis. Non-interpreted peptide tandem mass spectra were used for direct interrogation of *P. acanthamoebae* contig database (3533 ORFs) using Mascot 2.0 (http://www.matrixscience.com). The mass tolerance for database searches was 50 ppm. MASCOT was set up to only report peptide matches with a score above 14. With the parameters used, the threshold for statistical significance (p<0.05) corresponded to a total (protein) MASCOT score of 17, but we considered only scores greater than 30. Proteins scoring above 80 were considered automatically as valid, while all protein identifications with a total MASCOT score between 30 and 80 were manually validated. Validation included examination of the peptide rms mass error of individual peptide matches. MS/MS Peptide matches were validated only if at least an ion series of 4 consecutive y ions were matched, in addition to ions belonging to other series.

### Example 7: Cloning of selected immunogenic proteins

The ORFs corresponding to the immunogenic proteins Pac1, Pac4, Pac12, Pac18, Pac26, Pac35 and Pac123 were amplified by PCR using *P. acanthamoebae* DNA as template. The following primers were used :
Pac1 forward: 5'cagcatatgaattttaaatcttcctttaca3' (SEQ ID NO: 56)
Pac1 reverse: 5'gatgagctcttaatcgactttaatagaaacgaag3' (SEQ ID NO: 57)
Pac4 forward: 5'gatcatatggctataagtttatattcaaatcaa3' (SEQ ID NO: 58)
Pac4 reverse: 5'gatggatccttaggtattagacgttgcaggttt3' (SEQ ID NO: 59)
Pac12 forward: 5'gatcatatgacttacaataataatatcaatgtat3' (SEQ ID NO: 60)
Pac12 reverse: 5'gatggatccctacggaatttgatcggaacgtt3' (SEQ ID NO: 61)
Pac18 forward: 5'gattcatgagtccagatccaatcaaaggtttcg3' (SEQ ID NO: 62)
Pac18 reverse: 5'gatctcgagctgtgtaagtccttggaacatct3' (SEQ ID NO: 63)
Pac26 forward: 5'gaacatatgaatattaatagtactccacctg3' (SEQ ID NO: 64)
Pac26 reverse: 5'gtcggatccttagtgaatacgataattaaatgcgc3' (SEQ ID NO: 65)
Pac35 forward: 5'gatcatatgcaacgatcccttgggggt3' (SEQ ID NO: 66)
Pac35 reverse: 5'gatggatccttagtattggctaccgcagcaa3' (SEQ ID NO: 67)
Pac123 forward: 5'gactcatgagtaaaaaatggcatgtgattttatacg3' (SEQ ID NO: 68)
Pac123 reverse: 5'gacctcgagaatgcgtttaagatgctgttgca3' (SEQ ID NO: 69)

PCR products were cloned into the pET28 vector (Novagen EMD Chemicals Inc, San Diego, USA). This vector system allows the protein of interest to be expressed as a fusion protein with a 6 His tail fused to its N-terminus (Pac1, Pac4, Pac12, Pac18, Pac 26, Pac35) or C-terminus (Pac123).

### Example 8: Expression and purification of selected proteins

Protein expression in *E.coli* DE-3 is induced with isopropyl-β-D-thiogalactopyranoside (IPTG, Qbiogen, Basel, Switzerland), in most of the cases with 1 mM IPTG during 2.5 hours at 37°C, but induction conditions must be adapted for each individual protein. The P. *acanthamoebae* proteins can be used as antigens in detection or diagnostic assays either as unpurified *E. coli* lysate or, for increased sensitivity and reduced background, as a purified product.

For purification, the bacterial pellet is resuspended in lysis buffer (100 mM NaH₂PO4 H₂O, 10 mM Tris, 8M urea, pH 8.0 (denaturing conditions) or 50 mM NaH₂PO4 H₂O, 300 mM NaCl and 10 mM imidazole, pH 8.0 (non denaturing conditions)) containing 1 mg/ml lysozyme, 1x Halt Protease Inhibitor (Pierce Biotechnology Inc, Rockford, USA), 5 µg/ml DNAseI and 8 µg/ml RNAseA and lysed by short pulses of sonication on ice. Protein samples are applied on a Ni-NTA column (HIS-Select^{™} Spin Columns, Sigma-Aldrich, Missouri, USA). The column is washed 2 times with washing buffer (100 mM NaH₂PO4 H₂O, 10 mM Tris, 8M urea, pH 6.3 (denaturing conditions) or 50 mM NaH₂PO4 H₂O, 300 mM NaCl and 20 mM imidazole, pH 8.0 (non denaturing conditions)). And finally, the protein of interest is eluted from the column either by decreasing the pH of the solution to pH 5.9 and then to pH 4.5 (denaturing conditions) or by adding 250 mM imidazole to the last solution (non denaturing conditions). The most pure protein fractions are pooled and if necessary (denaturing conditions) dialysed to remove urea against 1 × PBS containing 4M, 2M, and no urea at 4° C. Some recombinant proteins precipitate when urea concentration decreases. In these cases, urea concentration was kept high enough to ensure solubility of the protein (usually 2M).

The purified recombinant proteins are submitted to SDS PAGE and transferred to nitrocellulose membrane before probing with relevant mouse, rabbit or human sera or are used directly as antigens in serological tests.

### Example 9: Enzyme-Linked Immunsorbent Assay (ELISA)

96 wells ELISA plates are coated with one purified recombinant proteins diluted in carbonate/bicarbonate buffer (or with a mixture of different proteins, i.e. we combined recombinant proteins 1, 4 and 12 in one assay for example) and blocked with 1% non-fat dry-milk in PBS, 0.1% Tween 20. After one washing step with PBS 0.1% Tween 20, the plate is incubated with human sera diluted 1:8 (in PBS, 0.1% Tween 20, 1% non-fat dry-milk), washed 5 times with PBS 0.1% Tween 20 and incubated with horseradish peroxidase-conjugated goat anti-human IgG (Chemicon,Temecula,CA,) diluted 1:5000 (in PBS, 0.1% Tween 20, 1 % non-fat dry-milk). After 5 washing steps with PBS 0.1% Tween 20, the plate is incubated with OPD (O_Phenylendiamine dihydrochloride, 1 mg/ml in citrate buffer) and the absorbance measured at 490 nm.

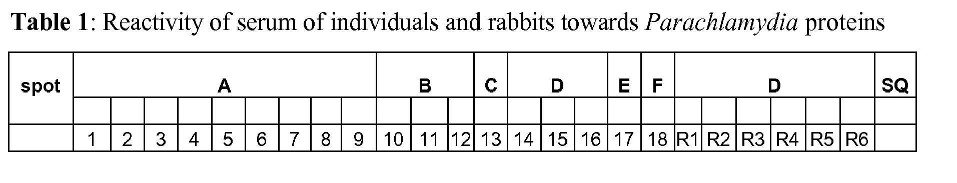

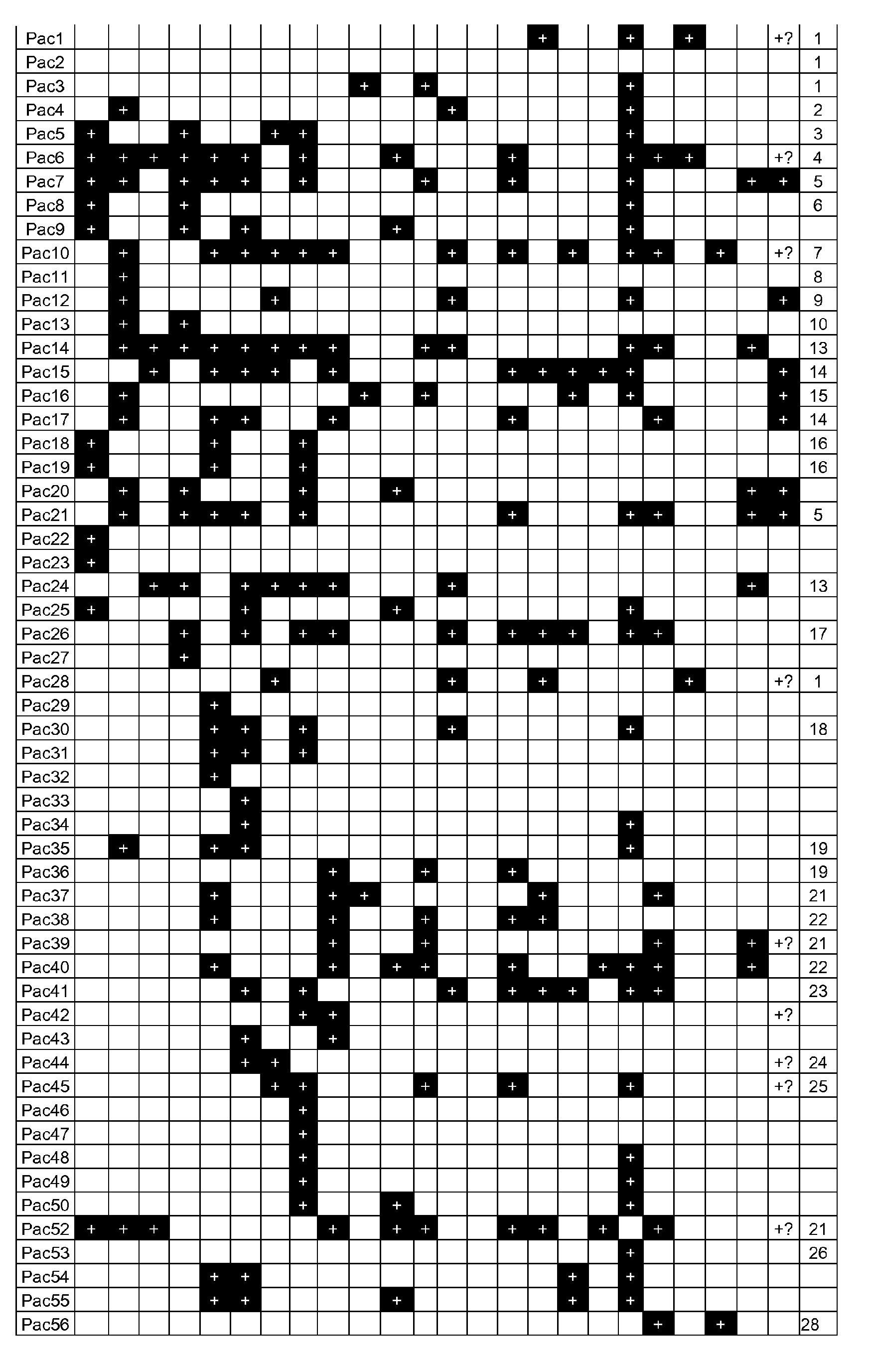

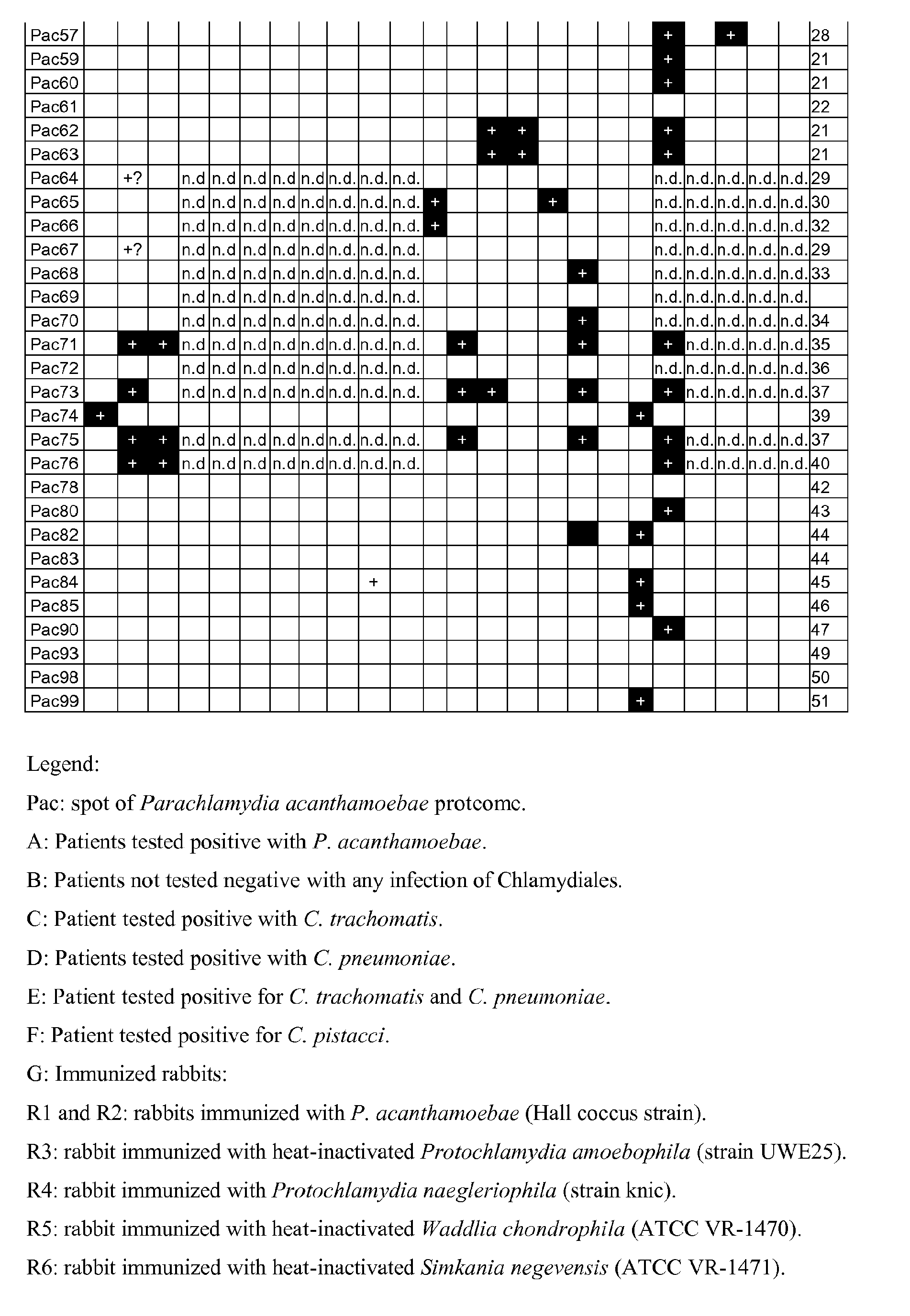

All the organisms used in R1-R6 are *Chlamydia*-like and these sera are tested in western blots in order to evaluate the possible cross-reactions.
SQ: SEQ ID NO (see attached official sequence listing).

## Claims

1. Use of at least one recombinant immunogenic peptide in the diagnosis of an infection by an intracellular *Chlamydia*-like microorganism.

2. Use of at least one isolated immunogenic peptide in the diagnosis of an infection by an intracellular *Chlamydia*-like microorganism.

3. The use of any one of the preceding claims in the diagnosis of an infection by an bacterial strain of the genus *Parachlamydia.*

4. The use of any one of the preceding claims, wherein the diagnosis is specific for an intracellular bacterial species.

5. The use of any one of the preceding claims, for diagnosis in a serological test.

6. The use of any one of the preceding claims, for diagnosis by an antibody-based test.

7. The use of any one of the preceding claims, wherein the peptide comprises:
(a) an amino acid sequence selected from the amino acid sequences according to any one of SEQ ID NO: 1-55;
(b) a functional variant of an amino acid sequence selected from any one of SEQ ID NO: 1-55; and/or
(c) an immunogenic peptide fragment of an amino acid sequence according to any one of SEQ ID NO: 1-55 and/or of the variant (b).

8. The use of any one of the preceding claims, wherein the peptide comprises (a) an amino acid sequence selected from any one of SEQ ID NO: 1-51, (b) a functional variant of an amino acid sequence selected from any one of SEQ ID NO: 1-51; (c) an immunogenic fragment sequence selected from any one of SEQ ID NO: 1-51 or from the variant sequence of (b).

9. The use of any one of claims 7-8, wherein the amino acid sequence is selected from any one of SEQ ID NO: 1, 2, 6, 7, 9, 13, 14, 15, 17, 18, 19, 22, 23, 21, 35, 39.

10. The use of any one of claims 7-9, wherein the amino acid sequence is selected from any one of SEQ ID NO: 2, 6, 7, 9, 13, 14, 18, 23, 35, 39.

11. The use of any one of claims 7-10, wherein the amino acid sequence is selected from any one of SEQ ID NO: 2, 6, 9, 18, 39, in particular SEQ ID NO: 2 and 9.

12. The use of any one of the preceding claims, wherein at least two different immunogenic peptides are used.

13. A test of diagnosis comprising one or more isolated and/or recombinant immunogenic peptides of an intracellular bacterial strain of the family *Parachlamydiaceae.*

14. The test of claim 13, wherein the peptide comprises:
(a) an amino acid sequence according to any one of SEQ ID NO: 1-55;
(b) an amino acid sequence comprising a functional variant of an amino acid sequence selected from any one of SEQ ID NO: 1-55; and/or
(c) an immunogenic peptide fragment of an amino acid sequence according to any one of SEQ ID NO: 1-55 and/or of the variant of (b).

15. The test of any one of claims 13 and 14, wherein the test in as an ELISA (Enzyme-Linked Immunosorbent Assay.

16. The test of any one of claims 13-15, which detects the presence of an antibody specifically binding to a peptide expressed by an intracellular bacterial strain of the family *Parachlamydiaceae..*

17. The test of any one of claims 13-15, wherein the test comprises a combination of more than one of said immunogenic peptides.

18. A method of producing an antibody, the method comprising the steps of:
(1) exposing a mammal to a peptide comprising:
(a) an amino acid sequence according to any one of SEQ ID NO: 1-55;
(b) a functional variant of an amino acid sequence selected from any one of SEQ ID NO: 1-55; and/or
(c) an immunogenic fragment of an amino acid sequence of (a) and/or (b); and,
(2) harvesting said antibody from serum of said mammal.

19. The antibody obtained by the method of claim 18.

20. A method of diagnosing infection of an individual by an intracellular *Chalmydia*-like organisms, the method comprising the steps of:
- contacting the serum and/or a blood sample of an individual with one or more recombinant and/or isolated immunogenic peptides of the *Chalmydia*-like organism;
- determining the presence or absence in said sample of an antibody specifically binding to at least one of said recombinant and/or isolated immunogenic peptide; and
- diagnosing an infection if an antibody is detected in the previous step.

21. The method of claim 20, wherein said recombinant and/or isolated immunogenic peptide are selected from peptides comprising:
(a) an amino acid sequence according to any one of SEQ ID NO: 1-55;
(b) a functional variant of an amino acid sequence selected from any one of SEQ ID NO: 1-55; and/or
(c) an immunogenic fragment of an amino acid sequence of (a) and/or (b).

22. The method of any one of claims 20 and 21, wherein the presence of two or more antibodies against a combination of two or more different recombinant and/or isolated immunogenic antibodies, respectively, is determined.

23. The use of an antibody specifically binding to a peptide selected from peptides comprising:
(a) an amino acid sequence according to any one of SEQ ID NO: 1-55;
(b) a functional variant of an amino acid sequence selected from any one of SEQ ID NO: 1-55; and/or
(c) an immunogenic fragment of an amino acid sequence of (a) and/or (b); in the diagnosis of an infection by an intracellular *Chlamydia*-like microorganism.

24. A method of diagnosing infection by an intracellular *Chlamydia*-like microorganism, the method comprising the steps of exposing tissue to an antibody specifically binding to an immunogenic peptide selected from peptides comprising:
(a) an amino acid sequence according to any one of SEQ ID NO: 1-55;
(b) a functional variant of an amino acid sequence selected from any one of SEQ ID NO: 1-55; and/or
(c) an immunogenic fragment of an amino acid sequence of (a) and/or (b); detecting if said antibody binds to said tissue; and, diagnosing, if there is such binding that there is an infection by said *Chlamydia*-like microorganism.

25. The method of claim 24, wherein said tissue is a tissue sample taken from an individual.

26. The method of claim 24 and 25, wherein said step of detecting if said antibody binds to said tissue comprises the step of detecting, preferably visualising, an antibody-antigen interaction, wherein the antigen is the said immunogenic peptide.

27. The use of an antibody specifically binding to a peptide selected from peptides comprising:
(a) an amino acid sequence according to any one of SEQ ID NO: 1-55;
(b) a functional variant of an amino acid sequence selected from any one of SEQ ID NO: 1-55; and/or
(c) an immunogenic fragment of an amino acid sequence of (a) and/or (b); in a immunohistochemical test for detecting the presence of a *Chlamydia*-like microorganism in tissue, in particular samples of tissue.


SEQUENCE LISTING

<110>  University of Lausanne

<120>  Antigenic Polypeptides for the Detection of Chlamydia-related
       Bacteria and Serological Tests for Diagnosis

<130>  B-6019-EP

<160>  69

<170>  PatentIn version 3.3

<210>  1
<211>  493
<212>  PRT
<213>  Parachlamydia acanthamoebae

<400>  1

Lys Ser Ile Gly Ala Asn Ser Ala Met Asn Phe Lys Ser Ser Phe Thr
1               5                   10                  15

Gln Phe Glu Lys Ile Phe Cys Ser Leu Leu Leu Thr Thr Phe Ser Leu
            20                  25                  30

Tyr Val His Pro Ala Phe Ser Ala Thr Thr Thr Glu Ile Gln Gln Gln
        35                  40                  45

His Glu Ser Pro Lys Ile Met Ala Leu Asp Phe Val Asp Val Ala Lys
    50                  55                  60

Lys Ala Thr Pro Ala Val Val Ser Ile Arg Val Lys Ile Ala Pro Ser
65                  70                  75                  80

Ser Pro Phe Gln Lys Ser Glu Arg Asp Glu Thr Asp Leu Phe Asn Glu
                85                  90                  95

Asn Phe Trp Gln Gln Phe Phe Gly Leu Pro Lys Ser Asn Glu Lys Lys
            100                 105                 110

Ser Ser Gln Asp Gln Val Gly Gln Ala Ser Gly Phe Ile Val Ser Asp
        115                 120                 125

Asn Gly Tyr Ile Leu Thr Asn Asn His Val Ile Thr Asp Ala Lys Glu
    130                 135                 140

Ile Thr Ala Met Leu Val Asp Gly Arg Glu Phe Pro Ala Lys Val Val
145                 150                 155                 160

Gly Lys Asp Lys Asn Thr Asp Ile Ala Val Leu Lys Ile Glu Ala Glu
                165                 170                 175

Ser Leu Pro Tyr Leu Lys Leu Ala Asp Ser Asp Glu Leu Gln Pro Gly
            180                 185                 190

Gln Trp Ala Ile Ala Ile Gly Asn Pro Leu Gly Leu Gln Ala Ser Leu
        195                 200                 205

Thr Val Gly Val Ile Ser Ala Thr Gly Arg Asp Asn Leu Asp Ile Ala
    210                 215                 220

Thr Ile Glu Asp Phe Ile Gln Thr Asp Ala Ala Ile Asn Arg Gly Asn
225                 230                 235                 240

Ser Gly Gly Pro Leu Leu Asp Met Lys Gly Glu Val Val Gly Ile Asn
                245                 250                 255

Thr Ala Ile Val Ser Asn Gln Gly Gly Tyr Met Gly Ile Gly Phe Ala
            260                 265                 270

Ile Pro Ser Asn Ile Ala Gln Asn Ile Met Asp Gln Leu Ile Ser Ser
        275                 280                 285

Gly Ser Ala Thr Arg Gly Phe Ile Gly Val Thr Leu Gln Lys Ile Asp
    290                 295                 300

Gln Asn Leu Ala Gln Ala Phe Gly Leu Thr Lys Met Glu Gly Ala Leu
305                 310                 315                 320

Ile Ser Asp Ile Ser Lys Gly Ser Pro Ala Glu Lys Ala Gly Leu Arg
                325                 330                 335

Gln Gly Asp Ile Val Leu Lys Tyr Asp Asn His Pro Val Ala His Ile
            340                 345                 350

Ser Ala Leu Arg Lys Ala Val Ser Phe Met Lys Pro Gly Thr Lys Leu
        355                 360                 365

Asn Leu Thr Ile Leu Arg Glu Gly Lys Thr Leu Glu Ile Pro Ile Asp
    370                 375                 380

Val Gly Thr Phe Pro Glu His Pro Ser Gln Leu Ile Ala Ala Asp Asn
385                 390                 395                 400

Lys Ile Gly Leu Glu Val Glu Asn Ile Thr Pro Glu Thr Ala Gln Lys
                405                 410                 415

Leu Gly Ile Ser Gly Leu Lys Gly Gly Leu Leu Ile Thr Gln Val Arg
            420                 425                 430

Pro Gly Ser Pro Ala Tyr Ile Ala Gly Ile Arg Pro Gly Ala Ile Leu
        435                 440                 445

Val Ala Val Asn Gln Asn Thr Val Thr Thr Val Ala Glu Phe Gln Gln
    450                 455                 460

Ile Leu Lys Asp Ser Asp Pro Ser Lys Pro Val Leu Leu Leu Ile Lys
465                 470                 475                 480

Gln Gly Gly Phe Thr Arg Phe Val Ser Ile Lys Val Asp
                485                 490

<210>  2
<211>  264
<212>  PRT
<213>  Parachlamydia acanthamoebae

<400>  2

Ile Arg Arg Asn Phe Phe Met Ala Ile Ser Leu Tyr Ser Asn Gln Gln
1               5                   10                  15

Pro Phe Ala Lys Leu Ile Glu Glu Ala Ser Lys Gln Leu Thr Gly His
            20                  25                  30

Ser Glu Leu Arg Leu Ser Ser Gly Leu Lys Val Arg Lys Ala Gly Tyr
        35                  40                  45

Gly Phe Ile Glu Trp Leu Arg Ser Ile Phe Gly Ala Asp Ser Thr Arg
    50                  55                  60

Gly Tyr Lys Lys Thr Ala Ala Lys Leu Glu Ala Leu Ala Leu Lys Arg
65                  70                  75                  80

Ser Ala Glu Ile Pro Phe Glu Gly Asp Leu Arg Asp Gln Phe Asn Glu
                85                  90                  95

His Tyr Lys Asn Ile Leu Gly Lys Val Leu His Ile Thr Glu Lys Asp
            100                 105                 110

Asp Gly Ser Leu Glu Val Asp Ala Lys Leu Thr Lys Asn Val Lys Lys
        115                 120                 125

Thr Glu Ala Ile Lys Lys Arg Phe Thr Asp Ser Leu Asp Lys Val Lys
    130                 135                 140

Lys Ile Glu Val Thr Glu Glu Ala Lys Glu Ile Gln Arg Lys Gln Leu
145                 150                 155                 160

Asn Ile Leu Asn Glu Leu Glu Asp Leu Lys Ala Asp Lys Ala Ala Lys

165                 170                 175

Ala Gln Glu Ile Thr Gly Lys His Glu Glu Ile Ser Gln Gln Gln Lys
            180                 185                 190

Ala Ile Glu Asp Leu Gln Ala Leu Glu Asp Lys Asp Glu Asp Ala Ile
        195                 200                 205

Ala Ala Ala Lys Glu Thr Leu Lys Glu Leu Ser Asp Gln Leu Ser Arg
    210                 215                 220

Leu Glu Glu Glu Asn Asp Ala Leu Ala Ile Ser Gln Lys Thr Lys Glu
225                 230                 235                 240

Glu Glu Arg Thr Lys Asn His Ala Ala Leu Ile Ala Phe Gln Val Lys
                245                 250                 255

Lys Lys Pro Ala Thr Ser Asn Thr
            260

<210>  3
<211>  696
<212>  PRT
<213>  Parachlamydia acanthamoebae

<400>  3

Arg Met Ala Arg Gly Glu Lys Asn Gln Leu Lys Asn Ile Arg Asn Ile
1               5                   10                  15

Gly Ile Met Ala His Ile Asp Ala Gly Lys Thr Thr Thr Thr Glu Arg
            20                  25                  30

Ile Leu Tyr Tyr Ser Gly Arg Val His Arg Met Gly Glu Val His Glu
        35                  40                  45

Gly Ala Ala Thr Met Asp Trp Met Glu Gln Glu Gln Glu Arg Gly Ile
    50                  55                  60

Thr Ile Thr Ser Ala Ala Thr Thr Val Phe Trp Lys Asp Ala Lys Ile
65                  70                  75                  80

Asn Ile Ile Asp Thr Pro Gly His Val Asp Phe Thr Ile Glu Val Glu
                85                  90                  95

Arg Ser Leu Arg Val Leu Asp Gly Ala Val Ala Val Phe Cys Ser Val
            100                 105                 110

Ser Gly Val Glu Pro Gln Ser Glu Thr Val Trp Arg Gln Ala Asp Arg
        115                 120                 125

Tyr Gly Val Pro Arg Ile Ala Phe Val Asn Lys Met Asp Arg Val Gly
    130                 135                 140

Ala Asp Phe Ala Asp Ala Val Arg Thr Met Arg Glu Lys Leu His Ala
145                 150                 155                 160

Asn Ala Ile Pro Val His Cys Pro Ile Gly Ala Glu Ala Asp Phe Lys
                165                 170                 175

Gly Met Val Asp Leu Val Ser Met Arg Ala Leu Phe Phe His Asp Glu
            180                 185                 190

Thr Leu Gly Ala Gln Trp Glu Glu Thr Asp Ile Pro Glu Asp Leu Leu
        195                 200                 205

Glu Lys Cys Lys Gln Met Arg Thr Glu Leu Leu Asp Glu Leu Ala Thr
    210                 215                 220

Ile Asp Asp Ser Asp Asp Glu Phe Met Thr Lys Val Leu Glu Asp Pro
225                 230                 235                 240

Asp Ala Leu Thr Val Asp Glu Ile Asn Ala Val Ile Arg Lys Gly Val
                245                 250                 255

Cys Ala Asn Lys Phe Asn Pro Val Leu Cys Gly Ser Ala Phe Lys Asn
            260                 265                 270

Lys Gly Ile Gln Gln Leu Leu Asp Ala Val Val Ser Trp Met Pro Ser
        275                 280                 285

Pro Leu Asp Arg Gly Ala Ile Lys Ala His Asp Leu Asn Thr Asp Glu
    290                 295                 300

Glu Ile Met Leu Gln Pro Ser Asp Asp Ala Pro Phe Ser Ala Leu Ala
305                 310                 315                 320

Phe Lys Ile Met Thr Asp Pro Tyr Val Gly Arg Leu Thr Phe Ile Arg
                325                 330                 335

Ile Tyr Ser Gly Met Leu Thr Lys Gly Met Asn Leu Ile Asn Ser Thr
            340                 345                 350

Lys Asp Ser Lys Glu Arg Ile Ser Arg Leu Leu Glu Met His Ala Asn
        355                 360                 365

Lys Arg Glu Glu Arg Asp Glu Phe Tyr Thr Gly Asp Ile Gly Ala Cys
    370                 375                 380

Ile Gly Leu Lys Lys Ala Ser Thr Gly Asp Thr Leu Cys Glu Ala Glu
385                 390                 395                 400

Arg Pro Phe Val Leu Glu Lys Met Glu Phe Pro Glu Pro Val Ile Ser
                405                 410                 415

Met Ala Ile Glu Pro Lys Ser Lys Ala Asp Arg Glu Lys Leu Ser Gly
            420                 425                 430

Ala Leu Ser Ala Leu Ser Glu Glu Asp Pro Thr Phe Arg Val Thr Thr
        435                 440                 445

Asn Glu Glu Thr Gly Gln Thr Ile Ile Ala Gly Met Gly Glu Leu His
    450                 455                 460

Leu Glu Ile Leu His Asp Arg Met Lys Arg Glu Phe Gly Val Glu Ala
465                 470                 475                 480

Asn Val Gly Lys Pro Gln Val Ser Tyr Lys Glu Thr Ile Thr Ile Pro
                485                 490                 495

Gly Ala Ser Gln Thr Lys Phe Val Lys Gln Ser Gly Gly Arg Gly Gln
            500                 505                 510

Tyr Ala His Val Glu Leu Glu Val Gln Pro Asn Glu Lys Gly Lys Gly
        515                 520                 525

Asn Glu Val Val Ser Lys Ile Val Gly Gly Val Ile Pro Arg Glu Tyr
    530                 535                 540

Ile Pro Ala Val Ile Ala Gly Val Asn Glu Gly Leu Ala Thr Gly Val
545                 550                 555                 560

Leu Ala Gly Tyr Asn Leu Val Asp Val Lys Val Ala Ile Val Phe Gly
                565                 570                 575

Ser Tyr His Asp Val Asp Ser Ser Glu Met Ala Phe Lys Ile Cys Gly
            580                 585                 590

Ser Met Ala Ile Lys Asp Ala Ala Arg Lys Cys Lys Pro Ile Ile Leu
        595                 600                 605

Glu Pro Ile Met Lys Val Asp Val Thr Thr Pro Glu Ala Ser Leu Gly
    610                 615                 620

Asp Val Ile Gly Asp Leu Asn Arg Arg Arg Gly Lys Ile Leu Gly Gln
625                 630                 635                 640

Glu Asn His Lys Gly Ser Val Ile Val Ser Ala Glu Val Pro Leu Ser
                645                 650                 655

Glu Met Phe Gly Tyr Ser Thr Gln Leu Arg Ser Leu Ser Ser Gly Arg
            660                 665                 670

Ala Thr Tyr Thr Met Glu Pro Ser His Phe Glu Lys Val Pro Ala Lys
        675                 680                 685

Ile Gln Glu Glu Ile Thr Lys Lys
    690                 695

<210>  4
<211>  552
<212>  PRT
<213>  Parachlamydia acanthamoebae

<400>  4

Glu Thr Thr Thr Lys Arg Ser Leu Arg Ser Met Ile Met Ala Ala Lys
1               5                   10                  15

Asn Ile Lys Phe Lys Glu Asp Ala Arg Gln Asn Ile Leu Lys Gly Val
            20                  25                  30

Arg Thr Leu Ala Ser Ala Val Lys Val Thr Leu Gly Pro Lys Gly Arg
        35                  40                  45

Asn Val Ile Ile Asp Lys Ala Tyr Gly Ala Pro His Ile Thr Lys Asp
    50                  55                  60

Gly Val Thr Val Ala Lys Glu Ile Glu Leu Glu Asp Lys His Glu Asn
65                  70                  75                  80

Met Gly Ala Gln Met Val Lys Glu Val Ala Ser Lys Thr Ala Asp Lys
                85                  90                  95

Ala Gly Asp Gly Thr Thr Thr Ala Thr Val Leu Ala Glu Ala Ile Phe
            100                 105                 110

Ser Glu Gly Leu Arg Asn Val Ala Ala Gly Ala Asn Pro Met Asp Leu
        115                 120                 125

Lys Arg Gly Met Glu Lys Ala Val Lys Thr Ile Ala Lys Glu Leu Glu
    130                 135                 140

Ala Leu Ser Lys Pro Ile Lys Asn Gln His Glu Ile Ala Gln Val Ala
145                 150                 155                 160

Thr Ile Ser Ala Asn Asn Asp Ala Glu Ile Gly Gln Ile Ile Ala Asp
                165                 170                 175

Ala Met Glu Lys Val Gly Lys Asp Gly Thr Ile Thr Val Glu Glu Ala
            180                 185                 190

Lys Gly Phe Glu Thr Thr Leu Asp Val Val Lys Gly Met Asn Phe Asp
        195                 200                 205

Arg Gly Tyr Leu Ser Ala Tyr Phe Met Thr Asn Pro Glu Thr Gln Glu
    210                 215                 220

Cys Ile Leu Glu Asn Pro Tyr Ile Leu Ile Tyr Glu Lys Lys Ile Ser
225                 230                 235                 240

Val Val Lys Glu Leu Ile Pro Leu Leu Gln Thr Val Ala Glu Ser Gly
                245                 250                 255

Arg Pro Leu Leu Ile Ile Ala Glu Asp Val Glu Gly Glu Ala Leu Ala
            260                 265                 270

Thr Leu Val Val Asn Arg Leu Arg Ala Gly Leu Lys Val Cys Ala Val
        275                 280                 285

Lys Ala Pro Gly Phe Gly Asp Arg Arg Lys Ala Ile Leu Glu Asp Leu
    290                 295                 300

Ala Ile Leu Thr Gly Ala Gln Val Ile Ser Glu Glu Leu Gly Met Lys
305                 310                 315                 320

Leu Glu Gln Ala Thr Ile Glu Met Leu Gly Lys Ala Lys Lys Val Ile
                325                 330                 335

Val Lys Lys Asp Asp Thr Thr Leu Ile Glu Gly Gln Gly Asp Lys Glu
            340                 345                 350

Arg Leu Arg Asp Arg Val Ala Gln Ile Lys Arg Gln Ile Glu Glu Thr
        355                 360                 365

Glu Ser Asp Tyr Asp Arg Glu Lys Leu Gln Glu Arg Leu Ala Lys Leu
    370                 375                 380

Ala Gly Gly Val Gly Val Ile Arg Val Gly Ala Ala Thr Glu Val Glu
385                 390                 395                 400

Met Lys Glu Lys Lys Asp Arg Val Asp Asp Ala Gln His Ala Thr Ala
                405                 410                 415

Ala Ala Ile Glu Glu Gly Ile Leu Pro Gly Gly Gly Val Ala Phe Ile
            420                 425                 430

Arg Cys Ile Pro Ala Leu Asn Arg Leu Ala Thr Thr Leu Glu Gly Asp
        435                 440                 445

Glu Lys Thr Gly Val Leu Ile Ile Ala Arg Ala Leu Ser Ala Pro Leu
    450                 455                 460

Arg Gln Ile Ala Glu Asn Ala Gly Gln Glu Gly Ser Ile Ile Leu Gln
465                 470                 475                 480

Glu Val Glu Ser Arg Pro Val Thr Glu Gly Tyr Asn Ala Leu Thr Gly
                485                 490                 495

Glu Tyr Val Asp Met Phe Glu Ser Gly Ile Leu Asp Pro Lys Lys Val
            500                 505                 510

Ala Arg Cys Ala Leu Glu Asn Ala Val Ser Ile Ala Ala Leu Leu Leu
        515                 520                 525

Thr Thr Glu Ala Thr Val Val Glu Ile Pro Glu Glu Lys Ala Ala Pro
    530                 535                 540

Ala Met Pro Ala Gly Met Asp Tyr
545                 550

<210>  5
<211>  561
<212>  PRT
<213>  parachlamydia acanthamoebae

<400>  5

Leu Met Ser Lys Leu Leu Gln Phe Asn Glu Glu Ala Leu Lys Ser Ile
1               5                   10                  15

Ser Lys Gly Leu Lys Thr Leu Ala Lys Ala Val Lys Val Thr Leu Gly
            20                  25                  30

Pro Lys Gly Arg Asn Val Val Ile Asn Arg Gly Phe Gly Ser Pro Leu
        35                  40                  45

Ser Thr Lys Asp Gly Val Thr Val Ala Lys Glu Val Ser Leu Lys Asp
    50                  55                  60

Lys Phe Glu Asn Ile Gly Ala Gln Leu Val Lys Glu Val Ala Ser Lys
65                  70                  75                  80

Thr Ser Asp Ile Ala Gly Asp Gly Thr Thr Thr Ala Ile Val Leu Ala
                85                  90                  95

Glu Ala Ile Tyr Ser Ala Gly Leu Lys Ser Val Thr Ala Gly Ala Asn
            100                 105                 110

Pro Met Ser Leu Lys Lys Gly Ile Glu Arg Ala Val Glu Val Leu Asn
        115                 120                 125

Lys Glu Leu Thr Ala Leu Ala Ser Pro Val Asn Thr Ser Gln Glu Ile
    130                 135                 140

Gln Gln Ile Ala Cys Ile Ser Ala Asn Asn Asp Pro Glu Ile Gly Glu
145                 150                 155                 160

Ile Ile Ala Lys Ala Met Glu Lys Val Gly Lys Asp Gly Ile Ile Thr
                165                 170                 175

Val Ala Glu Ala Lys Gly Ile Asp Thr Thr Leu Asp Val Val Glu Gly
            180                 185                 190

Met Gln Phe Asp Lys Gly Tyr Val Ser Pro Tyr Phe Ile Thr Asn Pro
        195                 200                 205

Glu Asn Met Ser Val Glu Leu Gln Asn Ala Leu Val Leu Val Thr Asp
    210                 215                 220

Lys Lys Leu Ser Ser Ala Lys Asp Leu Ile Pro Ile Leu Glu Lys Thr
225                 230                 235                 240

Met Glu Lys Gly Ala Arg Pro Ile Leu Ile Ile Ala Glu Asp Ile Asp
                245                 250                 255

Gly Glu Ala Leu Ala Thr Leu Val Val Asn Lys Leu Lys Ala Gly Leu
            260                 265                 270

Pro Val Cys Ala Val Lys Ala Pro Gly Phe Gly Asp Arg Arg Lys Ala
        275                 280                 285

Leu Leu Gln Asp Ile Ala Ile Leu Thr Gly Ala Thr Val Val Ser Glu
    290                 295                 300

Glu Val Gly Leu Gln Ile Glu Glu Val Asp Val Ser Val Leu Gly Arg
305                 310                 315                 320

Ala Lys Thr Ile Lys Ile Thr Lys Glu Glu Thr Thr Ile Ile Asp Gly
                325                 330                 335

Met Gly Asp Ala Gly Leu Ile Gln Asp Arg Ile Asn Gln Ile Lys Ala
            340                 345                 350

Glu Ile Ala Asn Pro Ser Thr Ser Asn Tyr Asp Arg Glu Lys Leu Glu
        355                 360                 365

Glu Arg Leu Ala Lys Met Val Gly Gly Val Ala Ile Ile Asn Ile Gly
    370                 375                 380

Ala Ala Thr Glu Thr Glu Leu Lys Glu Lys Lys Ala Arg Val Glu Asp
385                 390                 395                 400

Ala Leu His Ala Thr Arg Ala Ala Val Ala Glu Gly Ile Val Pro Gly
                405                 410                 415

Gly Gly Val Ala Leu Leu Arg Ala Val Lys Ser Leu Asp Asn Leu Lys
            420                 425                 430

Ala Ser Gly Asp Glu Ala Thr Gly Ile Ala Ile Ile Lys Ser Ala Ala
        435                 440                 445

Phe Ala Pro Ala Thr Ala Ile Ala Asn Asn Cys Gly Lys Gln Gly Asn
    450                 455                 460

Leu Ile Ala Glu Lys Ile Phe Glu Ala Gln Gly Ala His Gly Tyr Asn
465                 470                 475                 480

Gly Leu Thr Asp Glu Phe Cys Asp Leu Val Gln Ala Gly Val Ile Asp
                485                 490                 495

Pro Val Arg Val Thr Lys Ser Ala Leu Thr Asn Ala Ala Ser Ile Ala
            500                 505                 510

Ala Leu Leu Leu Thr Thr Ala Ala Val Ile Thr Asp Lys Pro Glu Pro
        515                 520                 525

Lys Ser Lys Gln Pro Asp Met His Ala Met Gly Gly Met Gly Gly Met
    530                 535                 540

Gly Gly Met Gly Gly Met Gly Met Gly Gly Met Gly Gly Met Gly Met
545                 550                 555                 560

Met

<210>  6
<211>  342
<212>  PRT
<213>  Parachlamydia acanthamoebae

<400>  6

Ile Leu Lys Ile Glu Arg Leu Arg Lys Arg Met Lys Ile Gly Tyr Leu
1               5                   10                  15

Gly Met Gly Ala Trp Gly Phe Cys Leu Ala Ser Leu Leu Ala Ala Lys
            20                  25                  30

Gly Tyr Glu Leu Thr Cys Trp Thr Thr Lys Gln Ala Leu Ala Asp Arg
        35                  40                  45

Leu Asn Gln Thr Arg Glu His Pro Ser Leu Pro Gly His Ile Ala Lys
    50                  55                  60

Gly Asn Leu His Phe Thr Thr Asp Leu Lys Ala Ala Leu Ile Gln Ser
65                  70                  75                  80

Glu Leu Leu Ile Glu Ser Val Thr Ser Ala Gly Leu Arg Pro Val Leu
                85                  90                  95

Glu Lys Val Lys Ala Val Gly Pro Leu Asn Ile Pro Leu Ile Met Thr
            100                 105                 110

Ser Lys Gly Ile Glu Gln Gly Ser Gly Leu Ile Leu Pro Asp Val Ala
        115                 120                 125

Ile Gln Thr Leu Gly Pro Gln Val Lys Ser Gln Val Gly Phe Leu Ser
    130                 135                 140

Gly Pro Gly Phe Ala Glu Glu Val Ile Arg Asn Leu Pro Thr Ser Val
145                 150                 155                 160

Val Gly Ser Ala Tyr Asp Pro Asn Leu Leu Met Thr Val Cys Gln Ile
                165                 170                 175

Phe Thr Thr Pro Thr Phe Arg Val Tyr Pro Asn Ala Asp Ile His Gly
            180                 185                 190

Val Ala Phe Gly Gly Ala Leu Lys Asn Ile Ile Ala Ile Ala Cys Gly
        195                 200                 205

Ile Cys Glu Gly Leu Gly Leu Gly Asn Ser Ser Lys Ala Ala Leu Met
    210                 215                 220

Thr Arg Gly Leu His Glu Ile Arg Lys Leu Ala Val Ala Gln Gly Cys
225                 230                 235                 240

Lys Ala Glu Thr Leu Asn Gly Leu Ser Gly Met Gly Asp Leu Phe Leu

                245                 250                 255

Thr Cys Ser Ser Phe Ile Ser Arg Asn Phe Arg Phe Gly His Leu Met
            260                 265                 270

Thr Gln Gly Leu Thr Pro Lys Glu Ala Gln Asp Lys Ile Gly Met Val
        275                 280                 285

Val Glu Gly Ala Tyr Thr Cys Val Ser Ala Leu Gln Leu Ser Glu Lys
    290                 295                 300

Leu Asn Val Ser Met Pro Ile Thr Lys Ile Ile His Glu Ile Ile Tyr
305                 310                 315                 320

Lys Asp Met Lys Pro Gln Ile Ala Val Asn Ala Leu Met Glu Arg Ala
                325                 330                 335

Ile Lys Glu Glu His Leu
            340

<210> 7
<211> 607
<212> PRT
<213> Parachlamydia acanthamoebae

<400> 7

Lys Glu Gln Glu Ile Lys Gly Asp His Met Asn Lys Lys Lys Gly Lys
1               5                   10                  15

Ile Ile Gly Ile Asp Leu Gly Thr Thr Asn Ser Cys Val Ala Val Met
            20                  25                  30

Glu Gly Gly Val Pro Lys Val Ile Ala Ser Ala Glu Gly Ser Arg Thr
        35                  40                  45

Thr Pro Ser Val Val Ala Phe Lys Gly Asn Glu Arg Pro Glu Glu Ile
    50                  55                  60

Ala Ala Gln Ile Leu Ile Lys Met Lys Glu Thr Ala Glu Ala Tyr Leu
65                  70                  75                  80

Gly Glu Lys Val Thr Glu Ala Val Ile Thr Val Pro Ala Tyr Phe Asn
                85                  90                  95

Asp Ser Gln Arg Gln Ser Thr Lys Asp Ala Gly Arg Ile Ala Gly Leu
            100                 105                 110

Asp Val Lys Arg Ile Ile Pro Glu Pro Thr Ala Ala Ala Leu Ala Tyr
        115                 120                 125

Gly Leu Asp Lys Glu Lys Thr Glu Lys Lys Ile Ala Val Phe Asp Leu
    130                 135                 140

Gly Gly Gly Thr Phe Asp Ile Ser Ile Leu Glu Ile Gly Glu Gly Val
145                 150                 155                 160

Phe Glu Val Leu Ala Thr Asn Gly Asp Thr His Leu Gly Gly Asp Asp
                165                 170                 175

Phe Asp His Ala Ile Leu Asn Trp Met Leu Glu Thr Phe Lys Gln Glu
            180                 185                 190

Thr Gly Ile Asp Leu His Asn Asp Lys Met Ala Leu Gln Arg Leu Arg
        195                 200                 205

Asp Ala Ala Glu Lys Ala Lys Ile Glu Leu Ser Gly Thr Gln Ser Thr
    210                 215                 220

Glu Ile Asn Gln Pro Phe Ile Thr Met Asp Ala Thr Gly Pro Lys His
225                 230                 235                 240

Leu Ser Leu Asn Leu Thr Arg Ala Lys Leu Glu Ser Leu Thr Ala Glu
                245                 250                 255

Leu Ile Asp Arg Thr Arg Glu Pro Cys Ile Lys Ala Leu Lys Asp Ser
            260                 265                 270

Gly Leu Ser Lys Asp Asp Ile Gly Glu Val Ile Leu Val Gly Gly Met
        275                 280                 285

Thr Arg Met Pro Ala Val Gln Glu Val Val Lys Ser Ile Phe Gly Lys
    290                 295                 300

Glu Gly His Lys Gly Val Asn Pro Asp Glu Val Val Ala Val Gly Ala
305                 310                 315                 320

Ala Ile Gln Gly Gly Val Leu Ala Gly Asp Val Lys Asp Val Leu Leu
                325                 330                 335

Leu Asp Val Thr Pro Leu Thr Leu Gly Ile Glu Thr Met Gly Gly Val
            340                 345                 350

Met Thr Pro Leu Val Glu Arg Asn Thr Thr Ile Pro Thr Gln Lys Lys
        355                 360                 365

Gln Val Phe Ser Thr Ala Ala Asp Asn Gln Pro Ala Val Thr Ile Arg
    370                 375                 380

Val Leu Gln Gly Glu Arg Lys Met Ala Asn Asp Asn Lys Glu Ile Gly
385                 390                 395                 400

Arg Phe Asp Leu Ala Asp Ile Pro Pro Ala Pro Arg Gly Val Pro Gln
                405                 410                 415

Ile Glu Val Ala Phe Asp Ile Asp Ala Asp Gly Ile Leu His Val Ser
            420                 425                 430

Ala Lys Asp Asn Ser Ser Gly Lys Glu Gln Lys Ile Arg Ile Glu Ala
        435                 440                 445

Gln Ser Gly Leu Arg Glu Glu Asp Ile Gln Asn Met Leu Lys Asp Ala
    450                 455                 460

Glu Leu His Ser Glu Glu Asp Lys Lys Arg Lys Glu Glu Val Glu Ile
465                 470                 475                 480

Arg Asn Glu Ala Asp Ser Gln Ala Phe Arg Ala Ser Lys Ala Leu Asp
                485                 490                 495

Glu Tyr Lys Asp Lys Leu Pro Ala Glu Ile Val Ser Glu Val Gln Gly
            500                 505                 510

Lys Ile Asp Ala Val Lys Lys Ala Leu Glu Gly Thr Asp Ser Ala Arg
        515                 520                 525

Ile Lys Ser Ala Lys Glu Asp Leu Glu Lys Ser Met Gln His Ile Gly
    530                 535                 540

Glu Ala Met Ala Lys Ala Gly Ala Ala Gly Gly Ala His Ala Ser Ala
545                 550                 555                 560

Ala Ala His Glu Gly Gln Ala His Gln Gln Ser Ser Ser Phe Glu Gly
                565                 570                 575

Gly Gln Ser His Gly Gly His His His Glu His Ser Lys Asp Asp Asp
            580                 585                 590

Gln Ile Glu Glu Ala Glu Val Glu Ile Ile Asp Asp Lys Asp Lys
        595                 600                 605

<210>  8
<211>  624
<212>  PRT
<213>  Parachlamydia acanthamoebae

<400>  8

Lys Ile Arg Ser Met Ile Met Val Thr Lys Thr Leu Glu Ile His Ser
1               5                   10                  15

Glu Asn Ile Leu Pro Ile Ile Lys Arg Trp Leu Tyr Ser Asp Lys Asp
            20                  25                  30

Ile Phe Ile Arg Glu Leu Val Ser Asn Ala Cys Asp Ala Ile His Lys
        35                  40                  45

Val Lys Ile Leu Gln Asp Lys Gly Glu Leu Ser Ala Ser Ser Asp Asp
    50                  55                  60

Pro Phe Arg Ile Glu Ile His Ile Asp Lys Glu Asn Lys Thr Leu Thr
65                  70                  75                  80

Phe Ser Asp Asn Gly Ile Gly Met Asp Ala Glu Glu Val Gln Lys Tyr
                85                  90                  95

Ile Ala Gln Ile Ala Phe Ser Ser Ala Glu Glu Phe Met Glu Lys Tyr
            100                 105                 110

Lys Ser His Asn Glu Thr Asp Gln Phe Ile Gly His Phe Gly Leu Gly
        115                 120                 125

Phe Tyr Ser Ala Tyr Met Val Ala Ser Lys Val Glu Ile Asn Thr Leu
    130                 135                 140

Ser Tyr Lys Glu Gly Ala Glu Pro Val Arg Trp Ile Cys Asp Gly Ser
145                 150                 155                 160

Ala Gln Tyr Glu Leu Glu Val Gly Thr Arg Thr Lys Arg Gly Thr Glu
                165                 170                 175

Val Ile Leu Tyr Val Asp Lys Asp Ser Glu Glu Phe Leu Asp Pro Ala
            180                 185                 190

Arg Ile Arg Gln Ile Leu Asn His Tyr Cys Ser Phe Leu Pro Tyr Pro
        195                 200                 205

Val Tyr Leu Glu Asp Ser His Ile Asn His Glu Glu Pro Leu Trp Ile
    210                 215                 220

Lys Ser Pro Ser Glu Cys Thr Ser Glu Asp Tyr Leu Arg Phe Tyr Arg
225                 230                 235                 240

His Leu Tyr Pro Met Asp Pro Asp Pro Leu Phe Trp Val His Leu Asn
                245                 250                 255

Val Asp Tyr Pro Phe His Leu Lys Gly Ile Leu Tyr Phe Pro Lys Leu
            260                 265                 270

Asn Arg Asp Phe Asp Ile Ser Lys Asn Thr Val Lys Leu Phe Cys Asn
        275                 280                 285

Arg Val Phe Val Ser Asp Asn Cys Lys Asp Val Ile Pro Asn Tyr Leu
    290                 295                 300

Met Ala Leu Arg Gly Val Ile Asp Ser Pro Asp Ile Pro Leu Asn Val
305                 310                 315                 320

Ser Arg Ser Tyr Leu Gln Met Asp Arg Thr Val Arg Gln Leu Gly Asn
                325                 330                 335

His Ile Ser Lys Lys Val Ala Asp Ser Leu Ala Thr Leu Tyr Arg Thr
            340                 345                 350

Glu Arg Glu Arg Phe Leu Glu Cys Trp Ser Asp Val Gly Gln Val Val
        355                 360                 365

Lys Leu Gly Ile Leu Glu Asp Glu Lys Phe Tyr Asp Lys Ala Lys Ser
    370                 375                 380

Phe Leu Val Trp Lys Asn Val Asn Gly Glu Trp Ile Thr Val Glu Glu
385                 390                 395                 400

Tyr Leu Glu Arg Asn Glu Ser Lys Ile Lys Asp Lys Val Phe Tyr Thr
                405                 410                 415

Ile Asp Gly Glu His Leu Ser His Phe Ala Glu Val Tyr His Lys Gln
            420                 425                 430

Gly Ile Glu Ile Leu Val Ala Asn Ser Pro Phe Asp Pro Tyr Leu Ile
        435                 440                 445

Gln Phe Leu Glu Arg Lys Leu Thr Pro Val Val Phe Lys Arg Val Asp
    450                 455                 460

Ser Asp Ile Asp Glu Ser Ile Leu Asp Lys Glu Lys Glu Lys Thr Leu
465                 470                 475                 480

Leu Asp Ala Glu Gly Arg Thr Glu Ala Gly Lys Ile Ala Asp Phe Val
                485                 490                 495

Arg Ser Lys Leu Glu Asn Glu Asn Val Glu Val Glu Ala Lys Ser Leu
            500                 505                 510

Ala Ala Glu Ser Val Pro Gly Val Val Val Ile Asp Glu Gln Gln Arg
        515                 520                 525

Arg Met Arg Asp Tyr Met Arg Ser Leu Asp Pro Lys Asp Ala Ser Arg
    530                 535                 540

Val Lys Gly Leu Met Asp Lys Arg Lys Leu Val Ile Asn Thr Asn Ser
545                 550                 555                 560

Pro Phe Val Ser Leu Val Gln Lys Leu Asp Gln Ile Gln Pro Glu Leu
                565                 570                 575

Thr Gly Asp Leu Val Lys Gln Ile Tyr Glu Met Ala Leu Leu Ser Gln
            580                 585                 590

Lys Glu Met Asp Pro Ala Glu Leu Lys Glu Phe Leu Glu Arg Asn Ala
        595                 600                 605

Arg Leu Leu Glu Lys Leu Thr Glu Lys Leu Ile Asp Ala Lys Gln Gln
    610                 615                 620

<210>  9
<211>  520
<212>  PRT
<213>  Parachlamydia acathamoebae

<400>  9

Phe Asn Lys Ile Arg His Phe Met Thr Tyr Asn Asn Asn Ile Asn Val
1               5                   10                  15

Leu Gln Ala Lys Ser Phe Gly Lys Asp Leu Ile Ala Ile Glu Lys Asp
            20                  25                  30

Gly Asn Thr Leu Lys Ser Val Gly Phe Leu Gly Arg Phe Ile Arg Arg
        35                  40                  45

Leu Lys Lys Met Ile Gly Lys Asn Ser Tyr Glu Asp Cys Lys Ile Thr
    50                  55                  60

Lys Ile Ala Ser Thr Met Lys Asn Met Val Asp Glu His Pro Ser Asp
65                  70                  75                  80

Glu Val Gly Lys Lys Ile Val Gln Asp Lys Phe Lys Glu Ile Leu Gly
                85                  90                  95

Leu Lys Lys Ile Lys Pro Glu Asn Lys Asp Leu Val Gln Gln Ile Phe
            100                 105                 110

Phe Glu Val Phe Pro Lys Glu Ser Leu Lys His Asp Leu Leu Glu Lys
        115                 120                 125

Gly Phe Gly Ala Leu Asp Arg Arg Ser Phe Asp Glu Ile Lys Glu Leu
    130                 135                 140

Ile Asp Leu Leu Thr Asp Arg Glu Leu Asn Glu Val Leu Leu Lys Glu
145                 150                 155                 160

Asp Ser Leu Asn Pro Asp Asn Lys Asp Ala Leu Pro Leu Ile Ser Thr
                165                 170                 175

Ala Ile Met Asp Ile Ser Asp Pro Ala Lys Ala Leu Ser Ile Val Gln
            180                 185                 190

Glu Leu His Ala Arg Gly Ile Glu Leu Asn Ser Lys Ala Val Glu His
        195                 200                 205

Gly Glu Ala Ser Thr Leu Tyr Glu Leu Ala Leu Arg Lys Asn Tyr Arg
    210                 215                 220

Glu Val Ala Glu Phe Ile Ala Ala Glu Leu Gly Phe Pro Pro Asn Ala
225                 230                 235                 240

Asn Val Ser Thr Asp Ala Glu Leu Gln Arg Ile Asn Leu Met Leu Asp
                245                 250                 255

Arg Leu Asn Asp Ala Lys Thr Glu Ala Glu Glu Glu Arg Ile Phe Lys
            260                 265                 270

Asp Ile Asn Asp Leu Thr Glu Glu Gly Lys Leu Asn Asn Val Phe Ser
        275                 280                 285

Leu Thr Thr Lys Asp Gly Ser Val Glu Asn His Thr Leu Leu Thr Ala
    290                 295                 300

Ile Tyr Thr Thr Leu Asp Asp Asp Glu Lys Lys Trp Lys Tyr Val Ser
305                 310                 315                 320

Tyr Leu Ile Glu Lys Gly Ala Asp Leu Asn Met Lys Ile Gly Glu Pro
                325                 330                 335

Lys Glu Ser Ile Gly Ala Leu Leu Cys Arg Asp Phe Ile Asp Glu Phe
            340                 345                 350

Ala Ser Phe Ser Pro Thr Gln Ile Ser Asn Asn Val Asp Ile Phe Gly
        355                 360                 365

Glu Tyr His Ala Asp Gly Glu Ala Gly Leu Lys Phe Glu Leu Leu Glu
    370                 375                 380

Lys Ala Ile His Asp Gln Gly Asp Arg Lys Trp Asp Ile Ile Ser Arg
385                 390                 395                 400

Leu Phe Asp Arg Asn Val Asp Leu Asn Arg Glu Ile Gly Pro Glu Asp
                405                 410                 415

Ser Lys Glu Lys Ile Gly His Leu Val Leu Lys Asp Leu Val Ser Glu
            420                 425                 430

Ile Asp Asn Ile Pro Thr Ala Gln Ile Leu Ser Met Ile Gln Asp Leu
        435                 440                 445

Lys Asn Tyr Asn Leu Ile Asn Leu Pro Val Gln Ile Gln Asp Glu Lys
    450                 455                 460

Asn Ile Leu Ile Thr Thr Leu Leu Val Glu Ala Asn Lys Lys Leu Asn
465                 470                 475                 480

Lys Asp Gly Lys Ser Ile Val Val Lys Thr Leu Leu Glu Lys Gly Ala
                485                 490                 495

Asp Pro Ala Phe Arg Pro Ile Ile Val Ser Pro Leu Thr Gly Val Asn
            500                 505                 510

Thr Gln Arg Ser Asp Gln Ile Pro
        515                 520

<210>  10
<211>  121
<212>  PRT
<213>  Parachlamydia acanthamoebae

<400>  10

Lys Ile Gln Ile Val Gly Asp Asp Ile Phe Val Thr Asn Pro Lys Phe
1               5                   10                  15

Leu Gln Lys Gly Phe Glu Glu Lys Ile Ala Asn Ser Ile Leu Val Lys
            20                  25                  30

Val Asn Gln Ile Gly Thr Leu Thr Glu Thr Leu Glu Thr Ile Arg Leu
        35                  40                  45

Ala Gln Thr His Ala Tyr Ser Ala Val Ile Ser His Arg Ser Gly Glu
    50                  55                  60

Thr Glu Asp Ser Ile Ile Ala Asp Ile Cys Val Ala Thr Asn Ser Gly

65                  70                  75                  80

Gln Ile Lys Thr Gly Ser Leu Cys Arg Thr Asp Arg Val Ala Lys Tyr
                85                  90                  95

Asn Arg Leu Leu Ser Ile Glu Ala Glu Leu Gly Ser Ile Ala Arg Tyr
            100                 105                 110

Ala Asp Ser His Ser Ala Lys Lys Ile
        115                 120

<210>  11
<211>  444
<212>  PRT
<213>  Parachlamydia acanthamoebae

<400>  11

Ile Ala Ser Pro Leu Lys Ser Ser Val Lys Glu Val Leu Val Lys Lys
1               5                   10                  15

Thr Phe Val Leu Asp Thr Asn Val Ile Leu His Asp Pro Glu Ala Ile
            20                  25                  30

Ile Lys Phe Pro Arg Asn Arg Val Val Ile Pro Val Ala Val Leu Glu
        35                  40                  45

Glu Leu Asp Lys Met Lys Arg Leu Pro Asn Asp Leu Gly Lys Asn Ser
    50                  55                  60

Arg Ala Phe Phe Arg Phe Leu Asp Ser Leu Ala Ser Glu Gly Lys Gly
65                  70                  75                  80

Asp Leu His Asn Gly Ile Ser Leu Ser Asn Asp Ser Asp Val Arg Ile
                85                  90                  95

Ala Leu Glu Ile Lys Lys Asp Tyr Lys Gly Asp Phe Ser Leu Ser Thr
            100                 105                 110

Thr Asp Asn Lys Ile Ile Met Ala Ala Tyr Leu Leu Phe Glu Arg Ser
        115                 120                 125

Glu Asn Val Val Phe Val Ser Lys Asp Phe Ala Ala Arg Ile Lys Ala
    130                 135                 140

Glu Ala Ile Gly Leu Glu Ala Glu Asp Tyr Glu Asn Leu Lys Tyr Ala
145                 150                 155                 160

Tyr Gln Thr Met Tyr Arg Gly Asn Arg Arg Val Glu Val Thr Lys His
                165                 170                 175

Asp Val Asp Ser Phe Phe Lys Asp Gly Phe Ile Lys Leu Pro Asp Leu
            180                 185                 190

Asp Cys His Pro Asn Glu Tyr Leu Val Met Thr Ser Pro Glu Asn Ser
        195                 200                 205

Ser Ala Val Gly Lys Tyr Asp Pro Val Arg Lys Gln Val Glu Pro Leu
    210                 215                 220

Leu Lys Ser Val Asn Met Trp Gly Ile Lys Pro Arg Asn Val Glu Gln
225                 230                 235                 240

Arg Cys Ala Val Asp Leu Leu Leu Arg Asp Asp Ile Lys Leu Val Thr
                245                 250                 255

Leu Leu Gly Pro Ala Gly Thr Gly Lys Thr Leu Leu Ala Leu Ala Cys
            260                 265                 270

Gly Leu Arg Lys Val Phe Asp Glu Gly Ile Tyr Ser Arg Ile Leu Val
        275                 280                 285

Ser Arg Pro Val Ile Pro Leu Gly Arg Asp Ile Gly Tyr Leu Pro Gly
    290                 295                 300

Thr Lys Glu Glu Lys Leu Phe His Trp Met Gln Pro Ile Tyr Asp Asn
305                 310                 315                 320

Leu Glu Phe Leu Cys Glu Ser Pro Ser Gly Gln Thr Thr Asp Thr Leu
                325                 330                 335

Arg Trp Val Thr Glu Ser Lys Lys Val Glu Met Glu Ala Val Thr Tyr
            340                 345                 350

Ile Arg Gly Arg Ser Leu Pro Lys Met Tyr Ile Ile Val Asp Glu Ala
        355                 360                 365

Gln Asn Leu Thr Pro His Glu Val Lys Thr Ile Ile Ser Arg Ala Gly
    370                 375                 380

Glu Gly Thr Lys Val Ile Leu Thr Gly Asp Pro Thr Gln Ile Asp His
385                 390                 395                 400

Pro Tyr Leu Asp Lys Asp Ser Asn Gly Leu Thr Tyr Ala Val Gly Arg
                405                 410                 415

Phe Ala Asp Gln Lys Ile Tyr Gly Asn Met Phe Leu Glu Lys Thr Glu
            420                 425                 430

Arg Ser Glu Leu Ala Ala Leu Ala Ala Glu Ile Leu
        435                 440

<210>  12
<211>  464
<212>  PRT
<213>  Prachlamydia acanthamoebae

<400>  12

Glu Ile Cys Asn Phe His Lys Ala Pro Thr Arg Lys Asn Met Arg Lys
1               5                   10                  15

Cys Leu Asn Phe Phe Ser Ala Ile Arg Glu Glu Lys Val Gln Lys Gly
            20                  25                  30

Thr Lys Ile Ser Ser Glu Glu Glu Asn Ala Gln Glu Lys Arg Lys Ala
        35                  40                  45

Leu Leu Val Ser Ala Phe Asn Gly Asn Asp Gln Arg Ala Ile Cys Glu
    50                  55                  60

Glu His Leu Asp Glu Leu Glu Leu Leu Ala Glu Thr Tyr Gly Val Glu
65                  70                  75                  80

Ile Val Ala Lys Val Pro Cys Leu Ile Arg Lys Val Asp Ala Ala Thr
                85                  90                  95

Phe Val Thr Gln Gly Lys Leu Glu Glu Leu Ile Thr Leu Ser Lys Glu
            100                 105                 110

Cys Ala Ala Asp Leu Ile Ile Phe Asp Asp Glu Ile Ser Pro Ser Gln
        115                 120                 125

Gln Arg Asn Leu Glu Lys Ala Phe Gln Leu Thr Val Met Asp Arg Thr
    130                 135                 140

Glu Val Ile Leu Glu Val Phe Ala Gln Arg Ala Lys Thr Lys Glu Ala
145                 150                 155                 160

Arg Leu Gln Ile Glu Tyr Ala Arg Val Lys Tyr Gln Ala Pro Arg Leu
                165                 170                 175

Lys Arg Leu Trp Ser His Leu Ser Arg Gln His Gly Ser Gly Gly Ala
            180                 185                 190

Gly Gly Gly Ala Tyr Leu Lys Gly Glu Gly Glu Lys Gln Ile Glu Ile
        195                 200                 205

Asp Arg Arg Leu Ile Lys Lys Arg Leu Glu Gln Leu Gln Ser Glu Ile
    210                 215                 220

Gln Glu Val Lys Glu His Arg Glu Ile Gln Arg Val Ala Arg Thr Arg
225                 230                 235                 240

Ser Ala Ile Pro Val Phe Ala Leu Val Gly Tyr Thr Asn Ala Gly Lys
                245                 250                 255

Ser Thr Leu Leu Asn Ala Leu Thr Asp Ala Asp Val Phe Val Glu Asp
            260                 265                 270

Lys Leu Phe Ala Thr Leu Asp Thr Thr Thr Arg Lys Phe Pro Leu Ser
        275                 280                 285

Thr Gly Gln Glu Val Leu Val Thr Asp Thr Val Gly Phe Ile Arg Lys
    290                 295                 300

Leu Pro His Leu Leu Val Ala Ala Phe Lys Ser Thr Leu Glu Glu Ala
305                 310                 315                 320

Ile Gln Ala Asp Ile Leu Leu His Val Ile Asp Ala Thr His Pro Met
                325                 330                 335

Ala Leu Glu Gln Ala Arg Thr Thr Ile Gln Val Leu Lys Glu Leu Gly
            340                 345                 350

Val Gly Asp Lys Pro Ile Ile Thr Val Leu Asn Lys Ile Asp Gln Ala
        355                 360                 365

Glu Gln Asn Glu Leu Thr Thr Arg Leu Arg Leu Glu Tyr Pro Arg Thr
    370                 375                 380

Val Pro Ile Ser Ala Leu His Arg Arg Gly Phe Glu Glu Leu Glu Glu
385                 390                 395                 400

Met Met Leu Arg Glu Leu Ser Gln Gln Arg Lys Thr Leu Asn Leu Arg
                405                 410                 415

Ile Pro Gln Ser Glu Tyr Ala Leu Val Ser Glu Ile Arg Arg Ser Gly
            420                 425                 430

Asn Val Ile Ser Gln Asp Tyr Glu Glu Asn Asp Val Leu Ile Glu Val
        435                 440                 445

Asp Leu Pro Val Ala Leu Ala Asn Lys Leu Lys Asn Tyr Val Ile Ser
    450                 455                 460

<210>  13
<211>  401
<212>  PRT
<213>  Parachlamydia acanthamoebae

<400>  13

Pro Asn Gly Gly Asn Gln Met Ala Ala Lys Glu Ser Phe Lys Arg Ser
1               5                   10                  15

Lys Pro His Val Asn Ile Gly Thr Ile Gly His Val Asp His Gly Lys
            20                  25                  30

Thr Thr Leu Thr Ala Ala Ile Thr Lys Val Leu Ala Glu Lys Gly Gly
        35                  40                  45

Ala Ile Phe Arg Ser Tyr Asp Ser Ile Asp Lys Thr Pro Glu Glu Arg
    50                  55                  60

Ala Arg Gly Ile Thr Ile Asn Ser Thr His Val Glu Tyr Glu Thr Glu
65                  70                  75                  80

Asn Arg His Tyr Ala His Val Asp Cys Pro Gly His Ala Asp Tyr Val
                85                  90                  95

Lys Asn Met Ile Thr Gly Ala Ala Gln Met Asp Gly Ala Ile Leu Val
            100                 105                 110

Val Ala Ala Thr Asp Gly Ala Met Pro Gln Thr Arg Glu His Ile Leu
        115                 120                 125

Leu Ala His Gln Met Gln Val Pro Ala Ile Val Val Phe Leu Asn Lys
    130                 135                 140

Val Asp Met Leu Ser Glu Gly Asp Glu Glu Leu Leu Asp Leu Val Glu
145                 150                 155                 160

Met Glu Ile His Glu Leu Leu Glu Ala Lys Gly Tyr Lys Asp Ala Pro
                165                 170                 175

Val Ile Arg Gly Ser Gly Leu Arg Ala Leu Glu Gly Asp Pro Lys Tyr
            180                 185                 190

Val Glu Ala Ile His Lys Leu Met Asp Thr Val Asp Ala Phe Ile Pro
        195                 200                 205

Glu Pro Ala Arg Glu Ile Asp Lys Ala Phe Leu Met Pro Ile Glu Asp
    210                 215                 220

Val Phe Ser Ile Ser Gly Arg Gly Thr Val Ala Thr Gly Arg Val Glu
225                 230                 235                 240

Arg Gly Met Val Lys Leu Gly Asp Lys Leu Gln Leu Val Gly Leu Gly
                245                 250                 255

Asp Thr Arg Asp Val Val Val Thr Gly Leu Glu Met Phe Asn Lys Thr
            260                 265                 270

Leu Asp Glu Ala Arg Ala Gly Glu Asn Val Gly Ile Leu Leu Arg Gly
        275                 280                 285

Val Asp Lys Asn Gln Ile Gln Arg Gly Met Val Leu Ala Ala Pro Gly
    290                 295                 300

Ala Cys Thr Pro His Thr Lys Phe Lys Gly Pro Val Tyr Ile Gln Thr
305                 310                 315                 320

Lys Glu Glu Gly Gly Arg His Lys Pro Phe Phe Thr Gly Tyr Arg Pro
                325                 330                 335

Gln Leu Tyr Ile Arg Thr Thr Asp Val Thr Gly Thr Val Glu Leu Pro
            340                 345                 350

Lys Asp Val Glu Met Val Met Pro Gly Asp Asn Val Glu Met Ile Ile
        355                 360                 365

Glu Leu Ile Gln Pro Val Ala Val Glu Lys Gly Met Arg Phe Ala Ile
    370                 375                 380

Arg Glu Gly Gly Arg Thr Ile Gly Ala Gly Thr Val Ser Glu Ile Ile
385                 390                 395                 400

Lys

<210>  14
<211>  280
<212>  PRT
<213>  Parachlamydia acanthamoebae

<400>  14

Thr Met Ser Thr Pro Val Thr Ala Ala Met Ile Lys Glu Leu Arg Glu
1               5                   10                  15

Arg Thr Gly Ile Gly Met Gly Ala Cys Lys Lys Ala Leu Glu Glu Ala
            20                  25                  30

Asn Gly Asp Met Glu Leu Ala Ile Thr Asn Leu Arg Lys Ser Gly Ala

35                  40                  45

Ala Ser Ala Val Lys Lys Glu Gly Arg Thr Thr Asn Glu Gly Met Ile
    50                  55                  60

Gly Glu Ala Gln Asn Asp Lys Ala Ile Ala Leu Val Glu Val Asn Ala
65                  70                  75                  80

Glu Thr Asp Phe Val Val Lys Asn Glu Arg Phe Gln Gln Phe Val Gln
                85                  90                  95

Ala Ile Ala Lys Glu Ile Ala Asp Thr Asn Pro Val Ser Leu Glu Ala
            100                 105                 110

Phe Leu Gln Gln Lys Tyr Ser Gln Asp Ser Asn Met Thr Ile Asp Asp
        115                 120                 125

Leu Arg Ser Ser Leu Val Gln Ala Ile Gly Glu Asn Ile Gln Ile Lys
    130                 135                 140

Arg Leu Lys Val Leu Pro Lys Lys Ala Asn Thr Ser Val Gly Val Tyr
145                 150                 155                 160

Ser His Leu Gly Gly Lys Met Val Thr Val Val Glu Ile Thr Gly Ser
                165                 170                 175

Asn Glu Gln Glu Ala Leu Ala Lys Asp Ile Ala Met His Val Ala Ala
            180                 185                 190

Ala Ala Pro Glu Tyr Val Ser Pro Glu Gln Val Pro Ser Glu Val Leu
        195                 200                 205

Glu Lys Glu Lys Glu Ile Ala Arg Ser Gln Val Val Gly Lys Pro Asp
    210                 215                 220

Phe Val Ala Asp Lys Ile Ile Ala Gly Lys Leu Asn Tyr Phe Tyr Asp
225                 230                 235                 240

Thr Ala Cys Leu Val Cys Gln Lys Tyr Ile Lys Asp Asp Ser Val Lys
                245                 250                 255

Ile Glu Asp Leu Val Lys Gln Lys Gly Lys Asp Leu Gln Leu Thr Ser
            260                 265                 270

Phe Glu Arg Trp Thr Val Ser Gln
        275                 280

<210>  15

<211>  274
<212>  PRT
<213>  Parachlamydia acanthamoebae

<400>  15

Ser Thr Arg Leu Ile Leu Phe Pro Ile Arg Arg Arg Asn Leu Phe Met
1               5                   10                  15

Ile Lys Lys Ser Arg Ile Trp Ala Leu Val Ala Leu Ser Ser Leu Phe
            20                  25                  30

Leu Gln Ala Gly Ala Leu Ser Ala Asp Gln Thr Ala Thr Gln Gln Glu
        35                  40                  45

Ser Lys Pro Ala Gln Ala Ala Pro Gln Glu Ile Asp Ile Met Lys Leu
    50                  55                  60

Ser Glu Ala Phe Gly Asn Phe Ile Gly Arg Asn Leu Asn Ala Pro Gly
65                  70                  75                  80

Ile Lys Phe Asp Leu Glu Ser Ile Ile Lys Gly Met Arg Asp Gly Ala
                85                  90                  95

Ala Asn Lys Pro Ser Pro Leu Thr Asp Gln Glu Tyr Glu Thr Met Met
            100                 105                 110

Thr Gln Leu Gln Ala Lys Ala Tyr Thr Glu Leu Ala Ser Glu Asn Leu
        115                 120                 125

Lys Ala Ala Asn Gln Phe Leu Lys Asp Asn Leu Lys Thr Ala Asn Leu
    130                 135                 140

Val Glu Ile Glu Pro Gly Lys Leu Gln Tyr Val Ile Val Glu Ala Gly
145                 150                 155                 160

His Glu Pro Thr Val Thr Glu His Ala Ser Pro Leu Val Lys Tyr Thr
                165                 170                 175

Gly Lys Tyr Ile Asp Gly Ser Val Phe Gly Ser Ser Asp Asp Thr Gly
            180                 185                 190

Gly Pro Ile Thr Val Pro Leu Asp Gln Thr Ile Pro Gly Phe Ser Lys
        195                 200                 205

Gly Ile Leu Gly Met Lys Glu Gly Glu Lys Arg Lys Leu Phe Val His
    210                 215                 220

Pro Asp Leu Gly Tyr Gly Thr Ser Gly His Leu Ser Pro Asn Ser Leu
225                 230                 235                 240

Leu Ile Phe Asp Val Glu Val Ile Lys Ser Asp Asn Ser Ala Glu Asp
                245                 250                 255

Lys Ser Lys Pro Leu Pro Asp Phe Ser Asp Glu Asn Ser Asp Lys Asn
            260                 265                 270

Ser Arg

<210>  16
<211>  598
<212>  PRT
<213>  Parachlamydia acanthamoebae

<400>  16

Lys His Ile Lys Lys Leu Leu Lys Gln Val Asn Lys Asn Ile Glu Ser
1               5                   10                  15

Glu Glu Phe Asp Met Pro Asp Pro Ile Lys Gly Phe Ala Gln Val Ala
            20                  25                  30

Tyr Ala Leu Asn Lys Ala Ala Asp Tyr Val Lys Gln Ser Thr Thr Ala
        35                  40                  45

Leu Gly His Gln Ile Ser Asn Val Leu Gly Gln Lys Thr Val Pro Val
    50                  55                  60

Glu Lys Lys Ile Gln Asp Gln Thr Pro Lys Met Glu Thr Gln Ala Pro
65                  70                  75                  80

Thr Arg Lys Val Arg Phe Ala Val Ser Glu Pro Thr Lys Glu Thr Leu
                85                  90                  95

Glu Asn Gln Thr Val Lys Lys Thr Met Thr Ala Phe Gln Ser Phe Phe
            100                 105                 110

Ser Gln Ile Lys Gln His Phe Glu Ser Ser Tyr Glu Leu Ile Lys Thr
        115                 120                 125

Lys Asp Glu Leu Lys Gln Glu Val Thr Gln Ile Leu Asn Glu Asn Gln
    130                 135                 140

Ser Lys Pro Phe Asn Glu Leu Thr Ala His Glu Gln Gly Ile Ile Lys
145                 150                 155                 160

Leu Tyr Ile Ser Ser Ala Asp Lys Ser Ile Leu Asp Thr Leu Arg Thr
                165                 170                 175

Leu Ala Asp Glu Lys Asn Pro Lys Thr Leu Asp Leu Leu Leu Ser Leu
            180                 185                 190

Pro Pro Glu Tyr Gln Ser Lys Phe Phe Ala Lys Leu Ser Pro Asp Thr
        195                 200                 205

Gly Val Lys Met Arg Asp Phe Leu Ile Gln Gln Thr Lys Ser Ser Arg
    210                 215                 220

Gln Asp Asp Val Phe Gln Leu Leu Asn Ser Thr Pro Leu Pro Phe Tyr
225                 230                 235                 240

Lys Ser Val Leu Asn Ser Leu Leu Thr Pro Asn Tyr Thr Glu Lys Met
                245                 250                 255

Thr Asn Pro Thr Thr Ser Gln Arg Asp Leu Val Ser His Thr Ile Arg
            260                 265                 270

Asp Ser Ile Ile Asn Leu Cys His Ala Asp Thr Glu Ser Leu Val Lys
        275                 280                 285

Asn Gln Asn Leu Leu Leu Asn Val Pro Ala Asn His Leu Thr Ser Met
    290                 295                 300

Leu Lys Glu Val Trp Glu Glu Asn Pro Glu Ala Ala Leu His Thr Val
305                 310                 315                 320

Gln Val Leu Ser Ser Arg Ile Asp Glu Ser Pro Glu Thr Ala Lys Gly
                325                 330                 335

Ile Ser Gln Phe Val Gln Asn Leu Asn Glu Lys Gln Gln Asn Thr Leu
            340                 345                 350

Phe Glu Met Thr Leu Glu His Pro Pro Arg Gly Ile Ser Gln Ile Leu
        355                 360                 365

Leu Ala Leu Pro Ser Ser Val Ser Val Ser Met Ala Glu Met Met Ile
    370                 375                 380

Asn Ser Gly Ala Leu Ser Asp Asn Asp Leu Ala Asp Ile Ala Ser His
385                 390                 395                 400

Leu Ile Thr Asn Glu Val Lys Thr Leu Asp Ser Leu Gln Thr Phe Leu
                405                 410                 415

Val Lys Asp Asn Val Ala Met Lys Phe Val Ser Met Leu Gln Arg Lys
            420                 425                 430

Glu Asn Glu Lys Ser Leu Asn Tyr Ile Trp Gly His Ile Pro Asn Asn
        435                 440                 445

Phe Glu Leu Thr His Ser Asn Leu Glu Thr Lys Glu Ile Ile Val Pro
    450                 455                 460

Asn Pro Glu Asn Phe Leu Asn Ile His Gln Thr Ile Leu Lys Asp Ile
465                 470                 475                 480

Thr His His Leu Glu Thr Asn Pro Pro Thr Pro Ala Met Lys Lys Val
                485                 490                 495

Tyr Gln His Leu Gln Asn Glu Ile Asp Arg Lys Phe Pro Ser Ala Asn
            500                 505                 510

His Glu Asn Gly Lys Ile Ala Val Leu Gly Leu Tyr Leu Ile Lys Met
        515                 520                 525

Gln Asn Ala Met Leu Val Asn Pro Gln Gln Phe Asp Asp Ser Phe Val
    530                 535                 540

Leu Ile Asp Lys Gln Ala Pro Asn Ala Ile Glu Ile Ala Lys Ile Leu
545                 550                 555                 560

Thr Lys Phe Ala Leu Gly Glu Lys Phe Glu Ala Ser Ser Asn Tyr Ala
                565                 570                 575

Phe Met Asn Pro Phe Phe Glu Asp Thr Thr Gln Leu Arg Asn Gln Met
            580                 585                 590

Phe Gln Gly Leu Thr Gln
        595

<210>  17
<211>  399
<212>  PRT
<213>  Parachlamydia acanthamoebae

<400>  17

His Met Asn Ile Asn Ser Thr Pro Pro Asp Val Thr Thr Gln Gly Tyr
1               5                   10                  15

Asn Phe Asp Pro Ser Ile Phe Ala Lys Phe Glu Lys Ala Met Ser Gln
            20                  25                  30

Ala Glu Asp Ser Ser Glu Gly Gly Ala Pro Pro Leu Thr Gly Glu Gln
        35                  40                  45

Ile Lys Lys His Val Val Asp Ser Lys Gly Lys Ala Val Thr Pro Val
    50                  55                  60

Gly Ile Ser Asp Ser Gln Ser Asn Gln Ala Val Ser Ala Thr Ala Ser
65                  70                  75                  80

Ser Ala Ser Pro Gly Val Ala Leu Asp Ala Gln Gly Asn Lys Val Ala
                85                  90                  95

Val Val Ser Thr Val Met Gln Asp Val Ile Glu Glu Ala Ser Thr Ala
            100                 105                 110

Met Asp Lys Phe Ser Ser Ala Tyr Val Ser Gly Thr Asn Gln Ser Leu
        115                 120                 125

Ile Leu Asn Asn Pro Ala Asp Thr Pro Thr Leu Val Ala Pro Thr Gly
    130                 135                 140

Thr Gly Ala Leu Pro Thr Gly Ser Lys Gly His Gly Asn Pro Phe Leu
145                 150                 155                 160

Thr Ala Ser Phe Val Val Ala Leu Ile Glu Val Met Ala Glu Leu Ile
                165                 170                 175

Lys Val Gln Ser Gln Gln Arg Leu Val Glu Thr Lys Leu Asp Ile Met
            180                 185                 190

Ser Asn Gln Trp Thr Ile Asp Leu Ala Lys Arg Met Ala Ser Asp Ile
        195                 200                 205

Met Gln Ser Ala Lys Thr Glu Ala Met Met His Tyr Met Leu Ala Ala
    210                 215                 220

Ser Ala Gly Val Gln Leu Gly Met Ala Val Ala Asn Gly Ala Met Gly
225                 230                 235                 240

Ala Leu Ala Met Lys Lys Ser Ile Gly Asp Tyr Asn Lys Gln Thr Lys
                245                 250                 255

Ala Leu Gln Thr Lys Phe Asp Glu Leu Asn Ala Pro Ala Lys Ser Arg
            260                 265                 270

Ala Pro Gly Ser Asp Ala Lys Val Gly Thr Glu Asp Gln Leu Met Lys
        275                 280                 285

Ala Lys Phe Lys Leu Asp Glu His Arg Ala Asn Lys Trp Ser Asn Ile
    290                 295                 300

Ser Thr Ser Phe Arg Met Arg Met Phe Pro Met Asp Thr Ala Lys Asp
305                 310                 315                 320

Ile Thr Asp Ser Thr Ser Lys Met Tyr Glu Asn Ile Val Gln Gly Ile
                325                 330                 335

Met Lys Val Lys Lys Ala Glu Tyr Asp Ala Asp Ile Lys Leu Ala Glu
            340                 345                 350

Gly Tyr Gln Glu Ile Ala Arg Lys Ile Gly Ala Asp Ala Ser Ala Ser
        355                 360                 365

Leu Lys Asp Ile Glu Asp Lys Val Lys Ser Ile Ala Glu Ala Val Gln
    370                 375                 380

Gln Met Ile Thr Lys Leu Tyr Ser Ala Phe Asn Tyr Arg Ile His
385                 390                 395

<210>  18
<211>  232
<212>  PRT
<213>  Parachlamydia acanthamoebae

<400>  18

Cys Leu Lys Asn Glu Val Met Met Lys Lys Ile Trp Leu Val Ala Ile
1               5                   10                  15

Ser Leu Met Met Gly Val Ala Gln Met Ala Met Gly Asp Glu Thr Ala
            20                  25                  30

Ser Ser Thr Lys Lys Tyr Glu Val Arg Asp Phe Asp His Leu Leu Gly
        35                  40                  45

Lys Val Lys Gly Leu Asn Asp Asp Leu Leu Lys Met His Phe Lys Leu
    50                  55                  60

Tyr Gln Gly Tyr Val Asn Asn Thr Asn Thr Leu Leu Gln Lys Ile Gly
65                  70                  75                  80

Glu Leu Asp Gln Thr Gly Lys Ser Gln Ser Pro Glu Phe Ala Gly Phe
                85                  90                  95

Lys His Met Leu Gly Trp Glu Phe Asp Gly Met Leu Leu His Glu Tyr
            100                 105                 110

Tyr Phe Glu Asn Leu Gly Gly Gln Thr His Gln Leu Lys Gln Asp Asp
        115                 120                 125

Pro Leu Phe Leu Lys Met Val Gln Asp Phe Gly Gly Tyr Asp Gln Trp

130                 135                 140

Lys Ser Asp Phe Gln Ala Thr Gly Ala Ile Arg Gly Ile Gly Trp Val
145                 150                 155                 160

Ile Thr Tyr Val Asp Pro Lys Gln Gly Arg Leu Val Asn Thr Trp Ile
                165                 170                 175

Asn Glu His Asp Val Gly His Leu Ser Gly Gly Lys Pro Leu Leu Val
            180                 185                 190

Met Asp Val Phe Glu His Ala Tyr Ile Thr Gln Phe Gly Leu Asp Arg
        195                 200                 205

Ala Lys Tyr Ile Gln Val Phe Phe Asp Asn Ile Asp Trp Asn Ala Val
    210                 215                 220

Ser Gln Arg Tyr Lys Lys Thr Leu
225                 230

<210>  19
<211>  235
<212>  PRT
<213>  Parachlyamydia acanthamoebae

<400>  19

Leu Lys Leu Ile Leu Lys Ser Lys Asn Leu Lys Ser Arg Lys Gln Ile
1               5                   10                  15

His Met Gln Arg Ser Leu Gly Gly Arg Asn Ile Arg Arg Ser Leu Ser
            20                  25                  30

Ile Ile Ser Glu Leu Ser Met Arg Thr Ser His Lys Ser Leu Thr Phe
        35                  40                  45

Leu Leu Arg Arg Met Ala Met Lys Lys Leu Ser Leu Ile Leu Phe Phe
    50                  55                  60

Ala Leu Cys Ala Phe Val Phe Asn Ser Gly Tyr Ala Gln Cys Gly Pro
65                  70                  75                  80

Ser Gly Cys Gly Val Pro Ala Ser Gly Gly Phe Glu Gly Gly Ser Ser
                85                  90                  95

Phe Ala Ala Pro Gln Gly Gly Glu Gly Gly Glu Ala Gly Ala Cys Gly
            100                 105                 110

Glu Tyr Ala Val Gly Asp Cys Tyr Cys Leu Phe Val Lys Tyr Glu Pro
        115                 120                 125

Arg Tyr Cys Asn Lys Tyr Arg Cys Glu Trp Asp Thr Gln Ser Tyr Gln
    130                 135                 140

Val Gln Arg Cys Arg Gln Val Pro Glu Thr Tyr Gln Lys Thr Cys Cys
145                 150                 155                 160

Arg Tyr Val Pro Gln Tyr Tyr Gln Val Asp Cys Val Arg Tyr Val Pro
                165                 170                 175

Gln Tyr Tyr Cys Thr Thr Glu Thr Arg Gln Val Pro Arg Tyr Val Cys
            180                 185                 190

Asp Arg Glu Cys Lys Tyr Val Pro Lys Tyr Tyr Tyr Lys Arg Ile Cys
        195                 200                 205

Asn Arg Asn Pro Val Pro Pro Ala Pro Ala Pro Thr Thr Val Thr Thr
    210                 215                 220

Pro Ala Pro Ala Ser Cys Cys Gly Ser Gln Tyr
225                 230                 235


<210>  20
<211>  224
<212>  PRT
<213>  Parachlamydia acanthamoebae

<400>  20

Val Tyr Gln Ser Leu Ser Lys Gly Phe Ser Met Ser Thr Lys Val Glu
1               5                   10                  15

Gln Lys Gly Ala Ser Ser Gln Arg Leu Lys Asp Glu Leu Lys Glu Leu
            20                  25                  30

Lys Ser Lys Lys Gly Lys Asn Gln Glu Ser Ser Arg Ile His Glu Ile
        35                  40                  45

Ala Lys Gln Leu Ala Ala Glu Lys Glu Trp Ser Ser Ala Ile Glu Ala
    50                  55                  60

Ile Gln Leu Ile Thr Asp Glu Lys Glu Arg Asn Ile Leu Ile Ser Asp
65                  70                  75                  80

Thr Ile Glu Gly Tyr Leu Leu Pro Ala Lys Glu Ile Asp Gln Ala Lys
                85                  90                  95

Lys Phe Ala Lys Leu Leu Thr Pro Thr Pro Glu Met Glu Pro Phe Val
            100                 105                 110

Trp Ile Arg Ile Ala Leu Ala Ser Lys Asp Pro Glu Gln Ala Leu Lys
        115                 120                 125

Leu Ala Lys Gln Leu Pro Ser Pro Ile Ser Arg Asn Phe Ala Phe Ile
    130                 135                 140

His Ile Met Glu Tyr Tyr Leu Ser Asn Lys Glu Lys Ser Lys Ile Asp
145                 150                 155                 160

Glu Leu Arg Lys Gln Met Leu Glu Asn Leu Lys Thr Ile Tyr Asp His
                165                 170                 175

Lys Ser Arg Ser Phe Ile Leu Arg Glu Leu Ala Ile Ser Leu Phe Tyr
            180                 185                 190

Ala Tyr Gly Glu Lys Glu Gln Ala Lys Glu Ile Ala Lys Leu Ile Pro
        195                 200                 205

Asp Glu Asp Met Arg Ser Lys Val Leu Lys Lys Val Glu Ala Thr Lys
    210                 215                 220

<210>  21
<211>  329
<212>  PRT
<213>  Parachlamydia acanthamoebae

<400>  21

Thr Asn Ile Phe Lys Gln Lys Lys Tyr Val Ser Tyr Leu Thr Ile Ala
1               5                   10                  15

Leu Gly Leu Ser Val Ala Pro Ser Leu Ser Ala Phe Ser Tyr Asp Asn
            20                  25                  30

Ser Ser Ser Ser Asn Pro Ser Ser Asn Tyr Ser Asn Pro Ser Ser Asn
        35                  40                  45

Pro Gly Ser Ala Arg Asp Ser Tyr Ser Gly Ser Ser Ser Lys Asp Ser
    50                  55                  60

Tyr Ser Asn Pro Gly Ser Ser Gly Ser Arg Asp Ser Asn Ser Gly Ser
65                  70                  75                  80

Ser Gly Ser Arg Asp Ser Ser Ser Asn Pro Gly Ser Ser Ser Ser Arg
                85                  90                  95

Asp Ser Asp Ser Ser Pro Gly Ser Ser Ser Ser Gly Gly Ser Ser Ser
            100                 105                 110

Asn Pro Gly Ser Ser Ser Ser Gly Ser Asn Asp Ser Phe Ser Asn Tyr
        115                 120                 125

Ser Ser Gln Arg Asp Val Asn Pro Asn Ser Ser Ser Tyr Tyr Asn Pro
    130                 135                 140

Ser Gly Ser Ser Tyr Ser Asn Ala Lys Asp Pro Tyr Ser Gly Ser Ser
145                 150                 155                 160

Ser Ala Thr Tyr Ser Glu Thr Ser Gly Asp Gln Glu Leu Ala Arg Arg
                165                 170                 175

Ile Arg Glu Lys Ile Ser Ser Gly Trp Phe Ser Arg Gly Tyr Asp Arg
            180                 185                 190

Val Ser Val Gln Val Asn Asn Gly Ala Val Ile Ile Gln Gly Val Val
        195                 200                 205

Lys Ser Gln Ala Asp Lys Asp Lys Val Glu Lys Glu Ile Arg Asp Ile
    210                 215                 220

Asp Gly Val Lys Ser Leu Thr Ser His Val Asn Val Glu Glu Ser His
225                 230                 235                 240

Ala Arg Gly His Glu Glu Arg Gln Phe Leu Gln Asp Arg Ala Ala Thr
                245                 250                 255

Ser Glu Asp Gln Gln Leu Asn Lys Lys Ile Arg Asp Asn Val Ser Lys
            260                 265                 270

Gly Trp Leu Trp Asp Ser Tyr Lys Asp Val Ser Leu Asn Thr Ser Asn
        275                 280                 285

Gly Val Val Thr Leu Asp Gly Thr Val Gly Ser Glu Ser Asp Lys Gln
    290                 295                 300

Asp Leu Ile Asn Glu Val Gln Lys Val Glu Gly Val Lys Ser Val Lys
305                 310                 315                 320

Ser Asn Leu Arg Ile Glu Ser Arg Tyr
                325

<210>  22
<211>  338
<212>  PRT
<213>  Parachlamydia acanthamoebae

<400>  22

Trp Ser Val Ser Met Gly Pro Asn Leu Ser Val Thr Ser Asp Asp Ala

1               5                   10                  15

Asp Arg Tyr Arg Ser Glu Gly Pro Asp Arg Lys Leu Leu Lys Asp Thr
            20                  25                  30

Phe Lys Val Val His Lys Asp Asp Ala Lys Glu Asp Ser Gln Lys Lys
        35                  40                  45

Arg Lys Asn Val Ala Asp Lys Gly Lys Phe Thr Lys Ala Leu Asp Ala
    50                  55                  60

Ala Asn Leu Lys Ala Glu Gln Glu Glu Glu Ala Gln Ser Lys Pro Leu
65                  70                  75                  80

Ser Pro Ser Leu Phe Asp Leu Ala Arg Gly Arg Lys Gly Ala Gly Asp
                85                  90                  95

Lys Gly Ile Glu Asp Lys Gly Ala Lys Glu Gln Ala Val Gln Asn Gln
            100                 105                 110

Gly Glu Glu Glu Phe Glu Gln Ser Arg Ser Gln Ile Ala Gln Arg Pro
        115                 120                 125

Tyr Phe Val Pro His Ala Glu Lys Lys Pro Glu Val Lys Met Glu Asp
    130                 135                 140

Val Ser Ile Asn Gln Asn Asp Thr Glu Asp Phe Gly Ala Gly Val Asp
145                 150                 155                 160

Ser Lys Lys Lys Asn Gly Gln Asn Pro Val Gln Glu Leu Pro Asn Gln
                165                 170                 175

Pro Leu Val Asn Pro Met Val Val Lys Thr Glu Leu Val Ser Ile Tyr
            180                 185                 190

Thr Pro Thr Pro Pro Ile Val Gln Pro Val Asn Met Ile Asp Arg Ile
        195                 200                 205

Lys Glu Ile Val Asp Gln Ile Gly Gln Ile Asp Leu Tyr Ser Ile Lys
    210                 215                 220

Glu Gly Gly Lys Thr Glu Thr Ala Ile Thr Leu Asn Gly Ile Asn Lys
225                 230                 235                 240

Met Phe Asp Gly Ala Arg Val Val Val Ser Ser Tyr Asp Thr Ala Arg
                245                 250                 255

Asn Glu Phe Asn Ile Thr Phe Glu Asn Leu Thr Gln Gln Ala Lys Asn

260                 265                 270

Leu Leu Asp Leu Pro Lys Asn Gln Glu Ala Leu Met Gln Thr Leu Ala
        275                 280                 285

Gln Arg Gly Glu Trp Ile Val His Gln Val Thr Thr Thr Thr Ile Met
    290                 295                 300

Glu Asn Arg Pro Tyr Leu Asp Asp Thr Gln Leu Ala Lys Asp Asp His
305                 310                 315                 320

Gln Arg Asp Glu Asp Glu Ser Gln Gly Gln Gly Arg Gln Lys Arg Gln
                325                 330                 335

Lys Gly

<210>  23
<211>  590
<212>  PRT
<213>  Parachlamydia acanthamoebae

<400>  23

Met Ser Asn Lys Phe Lys Arg Ala Trp Glu Glu Ser Asn Ile Val Asp
1               5                   10                  15

Asp Val Met Phe Asp Glu Lys Asp Ala Gln Ala Phe Lys Ala Met Leu
            20                  25                  30

Asp Gln Lys Gly Gly Asp Val Ser Ile Glu Glu Pro Thr Ala Met Ile
        35                  40                  45

Pro Gly Thr Val Leu Lys Gly Arg Ile Val Glu Ile Thr Lys Asp His
    50                  55                  60

Val Val Val Asp Val Gly Leu Lys Ser Glu Gly Leu Val Pro Met Glu
65                  70                  75                  80

Glu Phe Ser Asp Pro Ser Gln Ile Val Leu Asp Gly Glu Val Glu Val
                85                  90                  95

Leu Leu Asp Gln Ala Glu Asp Asp Asn Gly Gln Ile Val Leu Ser Arg
            100                 105                 110

Glu Lys Ala Glu Arg Leu Arg Gln Trp Glu Tyr Ile Leu Gln His Cys
        115                 120                 125

Glu Glu Gly Ser Ile Val Lys Gly Arg Val Leu Arg Lys Val Lys Gly
    130                 135                 140

Gly Leu Met Val Asp Ile Gly Met Glu Ala Phe Leu Pro Gly Ser Gln
145                 150                 155                 160

Ile Asp Asn Lys Arg Ile Lys Asn Leu Asp Asp Tyr Ile Asp Lys Thr
                165                 170                 175

Tyr Glu Phe Lys Ile Leu Lys Ile Asn Ile Glu Arg Lys Asn Val Val
            180                 185                 190

Val Ser Arg Arg Glu Leu Leu Glu Ala Glu Arg Val Ser Lys Lys Ala
        195                 200                 205

Glu Val Leu Glu Arg Ile Gln Glu Gly Asp Ile Arg Glu Gly Thr Val
    210                 215                 220

Lys Asn Ile Thr Asp Phe Gly Val Phe Leu Asp Leu Asp Gly Ile Asp
225                 230                 235                 240

Gly Leu Leu His Ile Thr Asp Met Thr Trp Lys Arg Ile Lys His Pro
                245                 250                 255

Ser Glu Met Val Gln Leu Gly Asp Lys Leu Lys Val Lys Ile Leu Ser
            260                 265                 270

Ile Asp Lys Glu Lys Gly Arg Val Ala Leu Gly Leu Lys Gln Lys Glu
        275                 280                 285

Pro Asn Pro Trp Asp Gln Ile Glu Gln Lys Tyr Pro Pro Gly Thr Arg
    290                 295                 300

Val Lys Gly Lys Ile Val Asn Leu Leu Pro Tyr Gly Ala Phe Ile Glu
305                 310                 315                 320

Ile Glu Pro Gly Ile Glu Gly Leu Ile His Val Ser Glu Met Ser Trp
                325                 330                 335

Val Lys Asn Val Thr Asp Pro Ser Asp Val Val Lys Lys Gly Asp Glu
            340                 345                 350

Val Glu Ala Ile Val Leu Ser Val Gln Lys Asp Glu Gly Lys Ile Ser
        355                 360                 365

Leu Gly Ile Lys Gln Thr Glu His Asn Pro Trp Asp Asp Val Gln Asn
    370                 375                 380

Lys Tyr Gln Val Gly Thr Asn Val Lys Ala Glu Ile Lys Ser Leu Thr
385                 390                 395                 400

Asn Tyr Gly Ala Phe Val Glu Leu Glu Pro Gly Val Glu Gly Leu Ile
                405                 410                 415

His Ile Ser Asp Leu Ser Trp Ile Lys Lys Val Ser His Pro Ser Glu
            420                 425                 430

Val Leu Arg Lys Gly Asp Val Val Asp Ala Val Ile Leu Ser Val Asp
        435                 440                 445

Lys Asp Ser Lys Lys Ile Thr Leu Gly Val Lys Gln Leu Ser Asp Asn
    450                 455                 460

Pro Trp Glu Ser Ile Glu Lys Thr Met Pro Val Gly Thr Leu Val Lys
465                 470                 475                 480

Gly Val Val Ser Lys Ile Thr Ala Phe Gly Ala Phe Val Glu Leu Glu
                485                 490                 495

Ser Gly Ile Glu Gly Leu Ile His Val Thr Glu Leu Ser Asp Lys Ala
            500                 505                 510

Phe Gly Lys Val Glu Asp Val Val Ala Lys Gly Asp Glu Val Ile Ala
        515                 520                 525

Lys Val Ile Lys Leu Asp Pro Glu Tyr Lys Lys Ile Ala Leu Ser Ile
    530                 535                 540

Lys Glu Tyr Leu Ile Asp Gln Asn Gln Cys Asn His Asp Asp Ile Val
545                 550                 555                 560

Val Ala Pro Ser Lys Arg Lys Lys Lys Asp Ala Glu Glu Gly Arg Lys
                565                 570                 575

Asp Asp Asp Glu Asn Thr Glu Ser Asp Glu Ser Asp Glu Ser
            580                 585                 590

<210>  24
<211>  440
<212>  PRT
<213>  Parachlamydia acanthamoebae

<400>  24

Asp Val Leu Ser Asn Lys Leu Pro Lys Tyr Glu Val Met Val Ser Asn
1               5                   10                  15

Asn Gln Thr Glu Val Ser Asn Glu His Val Lys Ile Ser Leu Lys Lys
            20                  25                  30

Glu Asp Gly Cys Leu Val Lys Leu Asp Val His Val Thr Pro Gln Ala
        35                  40                  45

Thr Gln Ala Ala Tyr Gln Glu Ala Ile Lys Ala Ile Ser Lys Glu Val
    50                  55                  60

Ser Met Pro Gly Phe Arg Lys Gly Lys Val Pro Ser Ala Met Ile Leu
65                  70                  75                  80

Gln Asn Phe His Pro His Val Glu Lys Glu Trp Arg Asp Ile Leu Leu
                85                  90                  95

Asn Arg Ala Leu Arg Glu Phe Met Asp Ile Thr Arg Ile Tyr Pro Phe
            100                 105                 110

Thr Lys Glu Ser Val Gln Lys Ala Leu Val Lys Asn Val Ser Leu Glu
        115                 120                 125

Ala Gly Ala Asp Leu Leu Ile Asp Phe Glu Ser Ala Pro Glu Ile Pro
    130                 135                 140

Glu Val Asp Pro Lys Ser Leu Glu Leu Lys Ser Ala Gln Lys Lys Asp
145                 150                 155                 160

Ile Thr Glu Ala Asp Ile Asp Lys Ala Leu Glu Asn Val Arg Ile His
                165                 170                 175

His Ala Asp Trp Asn Val Val Thr Asp Arg Pro Val Gln Glu Gly Asp
            180                 185                 190

Thr Val Val Val Asp Ala Cys Ser Val Glu Asn Pro Glu Gln Val Tyr
        195                 200                 205

Tyr Ala Asp Ala Ser Phe Lys Val Asp Glu Gln Asn Met Ala Glu Trp
    210                 215                 220

Phe Lys Thr Ala Ile Val Gly His Lys Thr Gly Glu Ser Val Glu Ala
225                 230                 235                 240

Gln Ala Ala Asp Gln Glu Pro Pro Val His Val Arg Ile Thr Ile Lys
                245                 250                 255

Glu Ile Lys Thr Ala Ile Leu Pro Glu Leu Asn Asp Glu Phe Ala Lys
            260                 265                 270

Lys Ser Gly Ala Glu Ser Leu Glu Asp Met Arg Lys Lys Ile Glu Asp
        275                 280                 285

Gln Leu Asn Lys Asn Ala Glu Glu Glu Ser Lys Phe Val Leu Arg Glu
    290                 295                 300

Gln Ile Ser Pro Ile Leu Leu Glu Lys Tyr Pro Phe Glu Val Pro Ala
305                 310                 315                 320

Ser Ile Val Lys Gln Glu Leu Lys Arg Ile Ile Thr Gln Lys Thr Glu
                325                 330                 335

Glu Leu Lys Glu Thr Gly Leu Ser Ser Ser Glu Ala Ser Lys Lys Ile
            340                 345                 350

Glu Glu Met Arg Glu Gln Ile Glu Lys Glu Cys Thr Asp Ser Ile Arg
        355                 360                 365

Leu Ile Phe Leu Glu Asn Glu Leu Ile Gln Lys Phe Asn Val Gln Val
    370                 375                 380

Ser Asp Glu Glu Val Val Arg Glu Leu Phe Ala Gln Ile Tyr Lys Tyr
385                 390                 395                 400

Ser Gly Ser Leu Thr Asp Ala Arg Gly Leu Leu Asn Ser Pro Glu Met
                405                 410                 415

His Asn Arg Val Arg Gln Leu Leu Met Ser Lys Lys Ile Lys Asp Phe
            420                 425                 430

Leu Val Glu Gln Ala Gln Lys Asn
        435                 440

<210>  25
<211>  542
<212>  PRT
<213>  Parachlamydia acanthamoebae

<400>  25

Ile Ile Tyr Glu Lys Leu Lys Glu Lys Asn Asn Met Ser Ser Thr Pro
1               5                   10                  15

Lys Gln Ile Ile Phe Glu Glu Glu Ala Arg Glu Leu Leu Leu Ser Gly
            20                  25                  30

Ile Lys Lys Leu Ala Asp Val Val Ala Phe Thr Leu Gly Pro Lys Gly
        35                  40                  45

Arg Asn Val Gly Leu Glu Lys Ser Trp Gly Thr Pro Thr Ile Thr Asn
    50                  55                  60

Asp Gly Ser Ser Ile Val Lys Glu Ile Asn Val Gln Cys Val Tyr Glu
65                  70                  75                  80

Asn Met Gly Val Ser Met Gly Lys Glu Val Val Gln Lys Ile Lys Glu
                85                  90                  95

Lys Cys Gly Asp Gly Thr Thr Thr Gly Thr Leu Leu Leu Ser Ala Leu
            100                 105                 110

Val Glu Asn Gly Val Lys Leu Ile Ala Ser Gly Ala Ser Pro Ile Gly
        115                 120                 125

Ile Lys Arg Gly Ile Asp Lys Ala Val Glu Ala Ile Val Lys Ala Ile
    130                 135                 140

Ser Thr Ser Ala Ile Pro Val Lys Ser Ser Gln Glu Val Arg His Ile
145                 150                 155                 160

Ala Thr Ala Ser Ala Ser Gly Asn Glu Glu Ile Gly Asn Leu Ile Ala
                165                 170                 175

Glu Ala Met Glu Lys Val Gly Lys Ser Gly Val Ile Thr Ile Glu Glu
            180                 185                 190

Ala Lys Gly Ile Glu Thr Thr Ile Glu Ile Val Glu Gly Met Gln Phe
        195                 200                 205

Asp Arg Gly Tyr Ile Ser Ser Tyr Phe Cys Thr Asn Thr Asp Lys Met
    210                 215                 220

Ile Ala Asp Met Ala Asn Pro Gln Ile Leu Leu Val Asp Arg Lys Ile
225                 230                 235                 240

Gly Ser Ile His Glu Leu Leu Pro Val Leu Gln Ala Thr Ala Ser Ala
                245                 250                 255

Gly Lys Glu Leu Leu Ile Val Ala Glu Asp Ile Glu Gly Asp Ala Leu
            260                 265                 270

Ser Thr Leu Val Val Asn Lys Leu Arg Gly Thr Leu Lys Val Val Ala
        275                 280                 285

Val Lys Ser Pro Gly Phe Gly Asp Arg Arg Lys Ala Met Leu Gln Asp
    290                 295                 300

Leu Ala Val Leu Thr Gly Ala Thr Val Ile Ser Glu Asp Ala Gly Met
305                 310                 315                 320

Ser Leu Lys Glu Ile Pro Ala Ser Ala Leu Gly Ser Ala Glu Lys Ile
                325                 330                 335

Ile Val Ser Lys Glu Asn Thr Val Ile Ile His Gly Ser Gly Ser Gln
            340                 345                 350

Glu Asp Ile Asp Ala Arg Val Lys Gln Ile Glu Asn Glu Ile Glu Ile
        355                 360                 365

Thr Lys Ser Ser Tyr Asp Lys Glu Lys Leu Glu Glu Arg Lys Ala Lys
    370                 375                 380

Leu Ser Gly Gly Val Ala Val Ile Arg Val Gly Ala Ala Thr Glu Pro
385                 390                 395                 400

Glu Leu Lys Gln Lys Lys Gln Val Phe Glu Asp Ser Leu Asn Ser Thr
                405                 410                 415

Lys Ala Ala Leu Glu Glu Gly Ile Val Pro Gly Gly Gly Val Ala Leu
            420                 425                 430

Leu Arg Ala Lys Ser Ala Ile Gly Gln Leu Lys Leu Val Gly Asp Glu
        435                 440                 445

Ala Met Gly Ala Thr Ile Val Glu Lys Ala Cys Glu Thr Pro Leu Lys
    450                 455                 460

Gln Ile Val Ser Asn Ala Gly Leu Asp Gly Ser Val Ile Leu Ala Glu
465                 470                 475                 480

Val Leu Lys Ala Pro Tyr Asn Phe Gly Tyr Asn Val Val Ser Gln Lys
                485                 490                 495

Leu Glu Asp Leu Val Ala Ser Gly Val Ile Asp Pro Ala Lys Val Val
            500                 505                 510

Lys Asn Ala Leu Ile Tyr Ala Ala Ser Val Ala Gly Ile Val Leu Ile
        515                 520                 525

Ser Glu Ala Leu Ile Ala Asp Ala Pro Glu Asp Asp Glu Glu
    530                 535                 540

<210>  26
<211>  324
<212>  PRT
<213>  Parachlamydia acanthamoebae

<400>  26

Glu Met Lys Ala Ile Val Val Ser Gln Tyr Gly Asp Pro Glu Val Leu

1               5                   10                  15

Ser Tyr Gln Asp Ala Asp Ile Gly Lys Pro Gly Lys Gly Gln Val Leu
            20                  25                  30

Val Arg Leu Ala Tyr Ala Gly Val Asn Phe Val Glu Ile Tyr Gln Arg
        35                  40                  45

Arg Gly Thr Tyr Pro Arg Thr Leu Pro Tyr Ile Pro Gly Ser Glu Ala
    50                  55                  60

Ser Gly Ile Ile Glu Ala Val Gly Glu Gly Val Glu Asp Phe Lys Val
65                  70                  75                  80

Gly Asp His Val Ala Tyr Val His Gln Pro Gly Ala Tyr Ala Glu Ala
                85                  90                  95

Ser Ile Ile Asn Ala Lys Ser Leu Ile Pro Leu Pro Ser Ser Phe Ser
            100                 105                 110

Leu Glu Gln Gly Ala Ala Phe Pro Leu Gln Gly Met Thr Ala His Tyr
        115                 120                 125

Leu Leu His Glu Phe Arg Lys Pro Lys Gln Gly Asp Val Val Leu Ile
    130                 135                 140

His Ala Ala Ala Gly Gly Met Gly Leu Leu Leu Val Gln Trp Ala Arg
145                 150                 155                 160

His Leu Gly Ala Arg Val Ile Gly Thr Val Ser Thr Glu Glu Lys Ala
                165                 170                 175

Lys Ala Ala Arg Gln Ala Gly Ala Thr Asp Val Ile Leu Tyr Asn Glu
            180                 185                 190

Lys Asp Phe Ala Val Glu Thr Lys Arg Leu Thr Asp Gly His Gly Ala
        195                 200                 205

Asp Leu Ile Ile Asp Gly Val Gly Lys Thr Thr Phe Lys Gly Asn Leu
    210                 215                 220

Glu Ala Ala Ala Leu Arg Gly His Ile Val Ile Tyr Gly Ala Ala Ser
225                 230                 235                 240

Gly Pro Ala Glu Pro Ile Pro Pro Asn Ala Leu Met Val Arg Ser Leu
                245                 250                 255

Thr Val Ser Gly Gly Ser Leu Pro Asn Tyr Leu Leu Thr Arg Asp Glu

260                 265                 270

Met Leu Gln Arg Ala His Ser Val Ile Lys Gly Ile Gln Glu Gly Trp
        275                 280                 285

Leu Lys Leu Thr Ile Asp Lys Val Ile Pro Leu Asp Gln Ala Ala Glu
    290                 295                 300

Ala His Arg Leu Leu Glu Asn Arg Gln Thr Ile Gly Lys Ile Val Leu
305                 310                 315                 320

Lys Ile Lys Ala

<210>  27
<211>  208
<212>  PRT
<213>  Parachlamydia acanthamoebae

<400>  27

Gly Lys Met Ala Phe Asn Phe Leu Lys Asn Ser Phe Glu Lys Met Lys
1               5                   10                  15

Asn Ala Leu Thr His Ala Gly Ser Leu Leu Gly Asn Lys Ile Arg Ala
            20                  25                  30

Leu Phe Gln Gly Pro Ile Asp Asp Glu Thr Trp Gly Lys Leu Glu Gln
        35                  40                  45

Leu Leu Tyr Glu Ala Asp Leu Gly Val Gln Thr Ala Ile Glu Leu Thr
    50                  55                  60

Glu Lys Val Lys Lys Leu Ser Gln Gln Asn Ser Ser Leu Lys Gly Asp
65                  70                  75                  80

Glu Leu Leu Ser Ser Val Arg Thr Glu Ile Leu Gln Val Leu Asn Gln
                85                  90                  95

His Ala Ser Pro Leu Arg Glu Ile Ser Ser Ser Ser Glu Pro Leu Val
            100                 105                 110

Ile Leu Ile Val Gly Val Asn Gly Asn Gly Lys Thr Thr Ser Thr Ala
        115                 120                 125

Lys Leu Ala Lys Phe Phe Gln Ser Gln Gly Lys Lys Val Leu Val Ala
    130                 135                 140

Ala Ala Asp Thr Phe Arg Ala Ala Ala Val Glu Gln Leu Asp Val Trp
145                 150                 155                 160

Ala Gln Lys Leu Gly Ile Asp Ile Val Lys Gly Ala Pro Lys Ser Asp
                165                 170                 175

Pro Ala Ala Val Val Phe Asp Ala Leu Ser Ala Ala Lys Ala Arg Gln
            180                 185                 190

Ala Glu Val Val Ile Val Asp Thr Ala Gly Arg Leu Gln Thr Lys Asn
        195                 200                 205

<210>  28
<211>  252
<212>  PRT
<213>  Parachlamydia acanthamoebae

<400>  28

Asn Arg Arg Lys Gln Phe Asp Lys Ile Asp Ser Ile Val Leu Ala Tyr
1               5                   10                  15

Leu Tyr Trp Pro Asn Ser Ile Gly Leu Glu Lys Glu Val Phe Met Asn
            20                  25                  30

Arg Asp Val Leu Glu Thr Asn Trp Val Gln Val Arg Glu Ile Leu Arg
        35                  40                  45

Glu Lys Phe Ser Asn Leu Ser Glu Asp Asp Ile Arg Gln Ile Asn Gly
    50                  55                  60

Arg Tyr Asp Gln Leu Val Ala Lys Leu Gln Gln Lys Tyr Gly Tyr Ser
65                  70                  75                  80

Lys Glu Glu Ala Glu Asp Arg Ile Arg Ser Trp Asn Phe Asp Arg Met
                85                  90                  95

Ala Ala Gly Pro Arg Asp Ala Ile Arg Asp Glu Lys Leu Arg Arg Glu
            100                 105                 110

Asp Ala Asn Thr Ile Arg Arg Glu Asp Thr Ala Arg Arg Glu Asp Arg
        115                 120                 125

Val Arg Arg Glu Glu Asp Asn Ser Thr Leu Phe Lys Trp Leu Leu Gly
    130                 135                 140

Leu Gly Ile Pro Leu Leu Leu Leu Ala Thr Tyr Phe Leu Ser Ser Gly
145                 150                 155                 160

Thr Arg Thr Pro Glu Val Thr Arg Thr Pro Thr Ala Thr Gln Glu Gln
                165                 170                 175

Phe Val Val Glu Thr Pro Ala Asp Arg Ala Ile Ser Ser Ser Leu Arg
            180                 185                 190

Asn Ala Phe Leu Ser Gln Pro Asn Leu Ala Ser Glu Leu Gln Thr Val
        195                 200                 205

Gln Ile Thr Thr His Asn Gly Val Val Thr Leu Ser Gly Ser Val Ser
    210                 215                 220

Ser Arg Glu Ile Ser Asp Phe Met Thr Thr Ser Ala Gln Asn Ala Ser
225                 230                 235                 240

Gly Val Asn Gln Val Ile Asn Asn Leu Gln Ile Arg
                245                 250

<210>  29
<211>  153
<212>  PRT
<213>  Parachlamydia acanthamoebae

<400>  29

Phe Phe Leu Thr Pro Ile Leu Lys Tyr Leu Arg Arg Val Ile Met Lys
1               5                   10                  15

Lys Val Ile Tyr Leu Leu Pro Ala Leu Gly Met Leu Phe Thr Ala Cys
            20                  25                  30

Glu Lys Lys Glu Thr Pro Thr Gly Pro Thr Pro Pro Asn Pro Ser Ser
        35                  40                  45

Tyr Tyr Thr Ala Asp Ala Asp Ser Ala Leu Asp Ala Thr Ser Thr Lys
    50                  55                  60

Ser Lys Asp Ala Ser Ile Thr Asp Thr His Pro Gly Asp Gln Ala Asp
65                  70                  75                  80

Ser Tyr Ala Asp Arg Pro Trp Leu Arg Lys Ile Arg Gln Ala Ile Ala
                85                  90                  95

Glu Asp Lys Asp Leu Glu Ser Gln Ala Gly Asn Ile Lys Ile Val Ile
            100                 105                 110

Ile Lys Gly Thr Ile Asn Leu Lys Gly Thr Val Pro Asn Glu Lys Ala
        115                 120                 125

Lys Ala Asp Leu Val Lys Lys Val Lys Gln Val Val Gly Asp Arg Lys
    130                 135                 140

Val Glu Asp Gln Thr Glu Val Ser Lys
145                 150

<210>  30
<211>  235
<212>  PRT
<213>  Parachlamydia acanthamoebae

<400>  30

Asn Gln Phe Val Ile Pro Thr Ile Leu Glu Leu Lys His Cys Ser Cys
1               5                   10                  15

Ala Pro Ser Ile Lys Ile Met Asn Glu Leu Val Phe Lys Asp Thr Leu
            20                  25                  30

Phe Ile His Ile Lys Ser Ser Arg Asp Leu Asp Leu Phe Leu Leu Lys
        35                  40                  45

Lys Ile Glu Lys Lys Cys Arg Ile Asn Leu Trp Ser Asn Phe Lys Tyr
    50                  55                  60

Leu Gln Leu Arg Glu Val Phe Met Ile Lys Thr Arg His Ile Phe Pro
65                  70                  75                  80

Leu Phe Ala Ile Ala Ala Ser Cys Val Leu Phe Asn Pro Ser Thr Leu
                85                  90                  95

Arg Ala Ala Asn Ile Ser Ser Val Gln Ala Gln Ala Asp Glu Gln Asn
            100                 105                 110

Phe Tyr Lys Gln Ile Glu Asp Leu Ile Ala Lys Asp Gln Lys His Leu
        115                 120                 125

Gln Glu Val Gln Asp Arg Ala Tyr Ser Arg Thr Glu Ala Pro Met Trp
    130                 135                 140

Ala Thr Lys Val Glu Leu Glu Gln Ala Ile Ile Met Leu Asp Val Lys
145                 150                 155                 160

Lys Val Leu Tyr Ala Asn Phe Lys Asn Thr Pro Ala Ile Gln Ser Pro
                165                 170                 175

Leu Val Arg Glu Lys Leu Leu Ser Leu Met Arg Lys Asp Asn Ile Thr
            180                 185                 190

Met Ser Asp Leu Ala Glu Leu Gln Ser Leu Thr Glu Arg Glu Lys Gln
        195                 200                 205

His Leu Arg Asp Glu Ser Ala Gln Ile Gln Ser Gln Gln Thr Gln Pro

210                 215                 220

Gln Gln Pro Pro Val Pro Ala Gly Ser Ala Gln
225                 230                 235

<210>  31
<211>  171
<212>  PRT
<213>  Parachlamydia acanthamoebae

<400>  31

Leu Ile Leu Leu Asn Leu Tyr Lys Glu Ile Lys Met Lys Ser Lys Leu
1               5                   10                  15

Leu Ile Leu Pro Ala Leu Ser Ile Met Leu Val Gly Cys Glu Gln His
            20                  25                  30

Val Thr Asn Pro Pro Pro Pro Lys Ser Pro Thr Gln Gln Asn Ser Ser
        35                  40                  45

Ile Leu Asp Ala Ala Pro Ser Glu Thr Asn Thr Asn Arg Asn Leu Gln
    50                  55                  60

Asp Arg Arg Ala Ser Asp Asp Ala Asp Asn Thr Ser Arg Asn Val Arg
65                  70                  75                  80

Asp Arg Ile Glu Gly Ala Leu Thr Pro Phe Asp Gln Ser Glu Ser Val
                85                  90                  95

Gly Asp Arg Ile Thr Leu Gln Ser Ile Arg Lys Ala Leu Val Glu Asn
            100                 105                 110

Lys Ile Leu Ser Thr Tyr Ala Lys Asn Ile Lys Val Ile Thr Ala Asn
        115                 120                 125

Gly Val Val Thr Leu Arg Gly Pro Val Asn Ser Ala Ala Glu Lys Glu
    130                 135                 140

Ala Ile Glu Arg Ile Val Ser Leu Ile Pro Gly Val Thr Lys Val Asp
145                 150                 155                 160

Asn Gln Leu Glu Val Leu Gln Asn Ala Glu Lys
                165                 170

<210>  32
<211>  182
<212>  PRT
<213>  Parachlamydia acanthamoebae

<400>  32

Val Gln Ser Asn Lys Leu Lys Glu Lys Met Glu Ala Ala Leu Glu His
1               5                   10                  15

Leu Lys Asn Asp Leu Asn Ser Ile Arg Thr Gly Gln Ala Asn Pro Gly
            20                  25                  30

Met Leu Asp Gly Ile Thr Val Glu Val Tyr Gly Ser Gln Met Arg Leu
        35                  40                  45

Arg Asp Ile Ala Thr Ile Asn Ser Pro Glu Ala Arg Gln Leu Leu Ile
    50                  55                  60

Thr Pro Phe Asp Ala Gly Thr Ala Gly Ala Ile Gly Lys Ser Ile Glu
65                  70                  75                  80

Arg Ala Asn Leu Gly Phe Met Pro Ile Val Asp Gly Asn Ala Val Arg
                85                  90                  95

Ile Lys Ile Pro Pro Met Asp Glu Ser Leu Arg Lys Glu Met Ala Lys
            100                 105                 110

Leu Cys His Lys Lys Gln Glu Glu Ala Lys Val Ala Ile Arg Asn His
        115                 120                 125

Arg Arg Asp Ala Asn Asp Leu Val Arg Lys Gln Lys Ala Ala Gly Glu
    130                 135                 140

Leu Pro Glu Asp Gln Met Lys Lys Gln Glu Lys Glu Ile Gln Glu Leu
145                 150                 155                 160

Thr Asp Lys Tyr Cys Lys Leu Ala Asp Glu Ile Ala Ala Lys Lys Glu
                165                 170                 175

Lys Glu Ile Ser Thr Ile
            180

<210>  33
<211>  95
<212>  PRT
<213>  Parachlamydia acanthamoebae

<400>  33

Gln Glu Val Ile Met Val Asn Lys Asp Thr Leu Gln Gly Gln Trp Lys
1               5                   10                  15

Gln Leu Gln Gly Lys Ile Lys Glu Lys Trp Gly Lys Leu Thr Asp Asp
            20                  25                  30

Glu Leu Thr Lys Ile Asn Gly Arg Arg Glu Met Leu Ile Gly Lys Leu
        35                  40                  45

Gln Glu Lys Tyr Gly Met Ala Lys Asp Arg Ala Glu Lys Glu Val Gln
    50                  55                  60

Asn Leu Glu Glu Ala Phe Arg His Ser Ser Glu Ser Ser Glu His Glu
65                  70                  75                  80

His Glu Glu Lys Glu His Ala His Gly Ser Ser Lys Arg Arg Phe
                85                  90                  95

<210>  34
<211>  133
<212>  PRT
<213>  Parachlamydia acanthamoebae

<400>  34

Ile Lys Ser Asn Arg Leu Val Tyr Lys Glu Lys Arg Met Ala Leu Lys
1               5                   10                  15

Lys Thr Glu Val Ala Lys Phe Lys Lys Arg Leu Glu Glu Met Arg Asp
            20                  25                  30

His Leu Thr Arg Met Leu Lys Gly Ser Thr Glu Glu Val Lys Lys Pro
        35                  40                  45

Asp Glu Ala Thr Gly Tyr Ser Gln His Gln Ala Asp Gln Gly Thr Asp
    50                  55                  60

Asp Phe Asp Arg Thr Ile Asn Leu Glu Val Thr Thr Arg Glu Tyr Ala
65                  70                  75                  80

Ile Leu Arg Gln Ile Asp Arg Ala Leu Glu Lys Ile His Asp Asn Thr
                85                  90                  95

Tyr Gly Ile Cys Asp Val Thr Gly Glu Glu Ile Pro Leu Ala Arg Leu
            100                 105                 110

Glu Ala Val Pro Tyr Ala Ser Met Thr Val Lys Ala Gln Glu Lys Leu
        115                 120                 125

Glu Lys Gly Leu Ile
    130

<210>  35
<211>  146
<212>  PRT
<213>  Parachlamydia acanthamoebae

<400>  35

Glu Ser Ala Lys Lys Ala Cys Lys Lys Ile Gly Arg Ala Ile Tyr Leu
1               5                   10                  15

Leu Ile Ser Ile Ser Leu Gln Ala Leu Leu Ala Arg Cys Lys Pro Lys
            20                  25                  30

Leu Arg Arg Asn Pro Met Ala Gln Thr Glu Glu Leu Lys Ala Thr Gln
        35                  40                  45

Ala Lys Asn Thr Leu Lys Pro Leu Gly Asp Arg Val Leu Val Arg Arg
    50                  55                  60

Leu Glu Ala Glu Glu Lys Leu Lys Gly Gly Ile Ile Leu Pro Asp Thr
65                  70                  75                  80

Ala Lys Lys Lys Gln Glu Gln Ala Glu Val Val Ala Ile Gly Thr Gly
                85                  90                  95

Lys Lys Asp Lys Asp Gly Asn Leu Ile Pro Pro Pro Val Lys Ile Gly
            100                 105                 110

Asp Ile Val Leu Met Glu Lys Tyr Ser Gly Gln Glu Val Thr Leu Gly
        115                 120                 125

Asp Gln Glu Tyr Val Ile Val Arg Gly Asp Asp Leu Ile Ala Ile Ile
    130                 135                 140

Gln Asn
145


<210>  36
<211>  147
<212>  PRT
<213>  Parachlamydia acanthamoebae

<400>  36

Ile Met Ala Arg Asp Leu Leu Pro Asp Leu Ile Ser Asp Arg Pro Phe
1               5                   10                  15

Thr Gln Leu Ala Asn Leu Trp Pro Asn Leu Phe Ala Gly Asn Val Leu
            20                  25                  30

Ser Asp Asp Phe Ser Ile Lys Gly Val Arg Ile Tyr Glu Glu Asn Asn
        35                  40                  45

Gln Leu His Val Glu Val Pro Val Pro Gly Leu Ser Leu Asp Asp Ile
    50                  55                  60

Glu Val Ser Leu Asn Lys Gly Val Leu Trp Ile Lys Gly Glu Leu Lys
65                  70                  75                  80

Glu Glu Glu Lys Asp Lys Lys Arg Asn Phe Tyr Arg Phe Ser Lys Arg
                85                  90                  95

Ser Tyr Ser Ser Ser Ile Pro Leu Pro Ala Gln Ile Asp Glu Lys Gln
            100                 105                 110

Glu Pro Gln Ala Val Tyr Glu Asp Gly Ile Leu Lys Val Ser Leu Gln
        115                 120                 125

Leu Ala Lys His Ala Glu Thr Lys Lys Ile Lys Val Lys Ser Gly Asn
    130                 135                 140

Lys Lys Lys
145

<210>  37
<211>  107
<212>  PRT
<213>  Parachlamydia acanthamoebae

<400>  37

Glu Leu Lys Thr Ala Leu Glu Glu Lys Trp Gly Val Lys Ala Ala Ala
1               5                   10                  15

Ala Ala Ala Val Met Ala Val Ala Pro Ala Ala Ala Ala Pro Ala Ala
            20                  25                  30

Ala Glu Ser Thr Asp Phe Gln Ile Thr Leu Glu Lys Val Thr Asp Asp
        35                  40                  45

Lys Lys Ile Ser Ala Ile Lys Val Val Arg Glu Ile Thr Gly Leu Gly
    50                  55                  60

Leu Lys Glu Ala Lys Glu Leu Val Glu Ser Thr Pro Lys Val Ile Lys
65                  70                  75                  80

Glu Thr Ala Pro Lys Ala Glu Ala Glu Glu Ile Lys Lys Lys Val Glu
                85                  90                  95

Ala Ala Gly Cys Lys Val Thr Leu Lys Gly Leu
            100                 105

<210>  38
<211>  129
<212>  PRT
<213>  Parachlamydia acanthamoebae

<400>  38

Pro Lys Val Val Ser Met Asn Cys Ile Val Lys Ser Lys Arg His Val
1               5                   10                  15

Thr Leu Ile Glu Met Met Ile Val Met Phe Leu Ile Ala Leu Ile Thr
            20                  25                  30

Gly Val Val Ala Tyr Asn Tyr Arg Gly Ala Leu Asp Glu Gly Lys Val
        35                  40                  45

Phe Lys Thr Lys Ala Gly Ile Glu Arg Leu Glu Thr Ile Leu Asn Leu
    50                  55                  60

Gln Val Ser Glu Asn Pro Ala Leu Leu Asn Asp Ile Gly Asn Asn Trp
65                  70                  75                  80

Gln Gly Ile Val Ala Lys Ser Pro Leu Val Gln Asn Pro Ala Ala Leu
                85                  90                  95

Ile Arg Asp Gly Trp Gly Glu Glu Tyr Lys Val Thr Val Asp Asn Gly
            100                 105                 110

Val Ile His Val Arg Ser Glu Lys Leu Glu Gln Tyr Gln Lys Ser Ser
        115                 120                 125

Lys

<210>  39
<211>  175
<212>  PRT
<213>  Parachlamydia acanthamoebae

<400>  39

Val Trp Gly Gly Asp Met Thr Gln Asn Ala Phe Tyr Arg Leu Phe Leu
1               5                   10                  15

Lys Glu Ile Ser Asp Leu Tyr Ser Ala Glu Asn Gln Leu Val Glu Ala
            20                  25                  30

Leu Pro Lys Met Ala Glu Ala Ala Thr Thr Pro Glu Leu Lys Glu Ala
        35                  40                  45

Leu Glu Arg His Leu Ser Glu Thr Lys Lys His Val Ala Arg Leu Asp
    50                  55                  60

Ser Ile Phe Ser Met Leu Asn Glu Asn Ser Phe Lys Glu Thr Cys Glu
65                  70                  75                  80

Ala Met Glu Gly Leu Ile Tyr Glu Ala Asn Glu Ile Ile Gln Asn Glu
                85                  90                  95

Ala Pro Ser Val Val Lys Asp Ala Ala Leu Ile Gly Ala Thr Gln Arg
            100                 105                 110

Ile Glu His Tyr Glu Ile Ala Gly Tyr Gly Val Ala Lys Thr Phe Ala
        115                 120                 125

Thr Gln Leu Glu Leu Tyr Asp Ile Ala Glu Leu Leu Lys Glu Ser Leu
    130                 135                 140

Glu Glu Glu Gly Asn Ala Asp Lys His Leu Thr Val Ile Ala Glu Gly
145                 150                 155                 160

Gly Phe Phe Thr Thr Gly Ile Asn Lys Arg Ala Lys Asn Ile Ser
                165                 170                 175

<210>  40
<211>  98
<212>  PRT
<213>  Parachlamydia acantamoebae

<400>  40

Ile Met Ala Asn Ile Gln Pro Leu Ala Asp Arg Val Leu Val Lys Arg
1               5                   10                  15

Thr Lys Ala Ser Thr Ser Lys Gly Gly Ile Leu Leu Pro Asp Ser Ala
            20                  25                  30

Gln Glu Lys Pro Lys Glu Gly Ile Val Val Ala Val Gly Pro Gly Lys
        35                  40                  45

Ile Asn Glu Asn Gly Arg His Glu Pro Val Ser Leu Glu Val Gly Ala
    50                  55                  60

Arg Val Leu Phe Ser Ser Tyr Ala Gly Thr Glu Val Lys Ser Pro Lys
65                  70                  75                  80

Asp Glu Glu Thr Tyr Leu Ile Leu Asn Ala Asp Asp Ile Leu Gly Val
                85                  90                  95

Phe Ala

<210>  41
<211>  129
<212>  PRT

<213>  Parachlamydia acanthamoebae

<400>  41

Pro Lys Val Val Ser Met Asn Cys Ile Val Lys Ser Lys Arg His Val
1               5                   10                  15

Thr Leu Ile Glu Met Met Ile Val Met Phe Leu Ile Ala Leu Ile Thr
            20                  25                  30

Gly Val Val Ala Tyr Asn Tyr Arg Gly Ala Leu Asp Glu Gly Lys Val
        35                  40                  45

Phe Lys Thr Lys Ala Gly Ile Glu Arg Leu Glu Thr Ile Leu Asn Leu
    50                  55                  60

Gln Val Ser Glu Asn Pro Ala Leu Leu Asn Asp Ile Gly Asn Asn Trp
65                  70                  75                  80

Gln Gly Ile Val Ala Lys Ser Pro Leu Val Gln Asn Pro Ala Ala Leu
                85                  90                  95

Ile Arg Asp Gly Trp Gly Glu Glu Tyr Lys Val Thr Val Asp Asn Gly
            100                 105                 110

Val Ile His Val Arg Ser Glu Lys Leu Glu Gln Tyr Gln Lys Ser Ser
        115                 120                 125

Lys

<210>  42
<211>  91
<212>  PRT
<213>  Parachlamydia acanthamoebae

<400>  42

Gly Ser Asn Trp Ser Ser His Asn Phe Phe Arg Val Leu Tyr Leu Ile
1               5                   10                  15

Leu Asn Ser Leu Phe Leu Lys Thr Ile Thr Glu Val Asn Met Ala Thr
            20                  25                  30

Glu Arg Thr Leu Ser Ile Ile Lys Pro Asp Ala Val Gly Asn Asn His
        35                  40                  45

Ile Gly Asp Ile Ile Ala Arg Phe Glu Lys Ala Gly Ile Arg Ile Val
    50                  55                  60

Ala Ala Lys Met Lys His Leu Ser Gln Ala Asp Ala Glu Gly Phe Tyr

65                  70                  75                  80

Ala Val His Lys Glu Arg Pro Phe Ser Val Thr
                85                  90

<210>  43
<211>  164
<212>  PRT
<213>  Parachlamydia acanthamoebae

<400>  43

Arg Asn Val Met Tyr Ser Tyr Leu Cys Ile Leu Leu Thr Val Ile Ser
1               5                   10                  15

Thr Ser Leu Phe Ala His Gln Pro Asn Thr Thr Glu Gln Gln Thr Leu
            20                  25                  30

Ser Ile Ile Lys Pro Asp Ala Val Ser Ser Lys His Ile Gly His Ile
        35                  40                  45

Ile Ser Arg Phe Glu Asp Asn Gly Leu Arg Val Ala Ala Val Lys Met
    50                  55                  60

Thr Lys Leu Ser Lys Glu Gln Ala Gly Gln Phe Tyr Ala Val His Lys
65                  70                  75                  80

Glu Arg Pro Phe Tyr Thr Asp Leu Val Glu Phe Met Ser Ser Gly Pro
                85                  90                  95

Val Val Ile Leu Val Leu Lys Gly Glu Asp Ala Val Ala Lys Asn Arg
            100                 105                 110

Ala Leu Met Gly Ala Thr Asp Pro Ser Lys Ala Glu Lys Asn Thr Leu
        115                 120                 125

Arg Ala Asp Phe Ala Gln Ser Val Thr Lys Asn Ala Val His Gly Ser
    130                 135                 140

Asp Ser Val Glu Asn Ala Glu Lys Glu Ile Thr Phe Phe Phe Asn Pro
145                 150                 155                 160

Ser Glu Ile Tyr

<210>  44
<211>  193
<212>  PRT
<213>  Parachlamydia acanthamoebae

<400>  44

Lys Phe Asn Phe Leu Gln Glu Ala Ile Met Thr Lys Ala Val Phe Gly
1               5                   10                  15

Leu Ala Lys Asn Glu Ser Gln Ala Gln Arg Ile Val Gln Lys Leu Gln
            20                  25                  30

Ala Ser Gly Phe Asp Thr Asp Glu Ile Ser Val Leu Phe Ala Asp Arg
        35                  40                  45

Thr His Glu Gly Lys Leu Thr Asp Val Ser Thr Asn Lys Gly Ala Ile
    50                  55                  60

Gly His Glu Lys His Thr Lys Ala Pro Glu Gly Ala Ala Thr Gly Ala
65                  70                  75                  80

Val Ser Gly Gly Ile Ile Gly Gly Ser Ile Gly Ile Leu Ala Gly Ile
                85                  90                  95

Gly Ala Leu Ala Ile Pro Gly Leu Gly Pro Phe Ile Ala Ala Gly Pro
            100                 105                 110

Ile Met Ala Ala Leu Ala Gly Ser Gly Ile Gly Gly Ser Leu Gly Met
        115                 120                 125

Leu Ile Gly Ala Leu Val Gly Met Gly Ile Pro Glu Tyr Glu Ala Lys
    130                 135                 140

Arg Tyr Glu Asn Ser Leu Lys Ala Gly Gly Ile Leu Ile Ser Val His
145                 150                 155                 160

Thr Asn Asn Ala Glu Asp Val Asp Arg Ala Ser Glu Leu Leu Arg Lys
                165                 170                 175

Glu Gly Ala Glu Asp Ile Ser Thr Ser Tyr Glu Lys Ser Glu Arg Ser
            180                 185                 190

Ser

<210>  45
<211>  186
<212>  PRT
<213>  parachlamydia acanthamoebae

<400>  45

Lys Glu Gly Ile Met Lys Asn Gln Asp Leu Thr Gln Leu Phe Ile Asp
1               5                   10                  15

Glu Leu Ala Asp Met Tyr Asn Ser Glu Asn Gln Ile Leu Lys Ala Leu
            20                  25                  30

Pro Lys Leu Ile Lys Ala Ala Ser Leu Pro Asp Leu Lys Glu Ala Leu
        35                  40                  45

Thr Thr His Leu His Glu Thr Glu Glu Gln Val Glu Arg Ile Glu Lys
    50                  55                  60

Ile Phe Ser Leu Met Asn Leu Pro Val Ser Asp Lys Ile Cys Glu Ala
65                  70                  75                  80

Met Arg Gly Leu Ile Lys Glu Ala Asp Asp Leu Thr Gln Asn Lys Ser
                85                  90                  95

Lys Ser Ala Thr Leu Asp Ala Ala Ile Ile Ser Ala Ala Gln Lys Val
            100                 105                 110

Glu His Tyr Glu Ile Ala Ser Tyr Gly Thr Leu Arg Ser Phe Ala Lys
        115                 120                 125

His Leu Asp Leu Lys Ser Glu Ile Thr Asp Leu Leu Gln Glu Thr Leu
    130                 135                 140

Asp Glu Glu Gly Ser Ala Asp Lys Lys Leu Thr Lys Ile Ala Asp Gly
145                 150                 155                 160

Ser Phe Leu Ser Ser Gly Val Asn Lys Glu Ala Val Glu Glu Glu Phe
                165                 170                 175

Ala His Ser Ser Ser His Arg Lys Arg Lys
            180                 185

<210>  46
<211>  217
<212>  PRT
<213>  Parachlamydia acanthamoebae

<400>  46

Glu Gly Ser Leu Phe His Asn Phe Phe Asn Pro Ile Cys Arg Cys Phe
1               5                   10                  15

Ile Leu His Arg Ser Ala Ser Ile Leu Lys Val Lys Thr Ile Met Ala
            20                  25                  30

Glu Lys Lys Asn Gln Leu Phe Pro Gly Arg Glu Pro Glu Arg Pro Leu
        35                  40                  45

Glu Cys Thr Glu Cys Lys Lys Pro Ile Ala Val Arg Tyr Thr Glu Ile

50                  55                  60

Ile Gly Asn Ser Ile Asn Asn Ile Ser Met Cys Ala Asp Cys Pro Glu
65                  70                  75                  80

Leu Lys Lys Lys Leu Tyr Gly Val Gln Ser His Lys Glu Gly Gly Val
                85                  90                  95

Ser Val Gly Asp Gly Gly Ala Asn Leu Ala Cys Gly Asn Cys Gly Thr
            100                 105                 110

Thr Leu Glu Glu Val Arg Ile Gly Ser Arg Met Gly Cys Ser Thr Cys
        115                 120                 125

Tyr Asp Val Phe Gly Glu Ala Leu Leu Ala Asp Met Val Ser Ser Asn
    130                 135                 140

Lys Ile Pro Ala Arg Leu Ala Ser Asn Lys Lys Ser Ile Pro Phe His
145                 150                 155                 160

Ile Gly Arg Thr Pro Gln Glu Ala Pro Ile Ile Asn Ser Ser Leu Arg
                165                 170                 175

Leu Leu Ala Leu Asn Glu Ala Leu Asn Glu Thr Leu Gln Arg Glu Asp
            180                 185                 190

Tyr Glu Gln Ala Ala Leu Leu Arg Asp Gln Ile Lys Glu Leu Thr Lys
        195                 200                 205

Asp Ala Glu Glu Gly His Asp Glu Lys
    210                 215

<210>  47
<211>  127
<212>  PRT
<213>  Parachlamydia acanthamoebae

<400>  47

Phe Lys Lys Tyr Tyr Asn Lys Thr Tyr His Ile Pro Leu Arg Arg Cys
1               5                   10                  15

Val Met Phe Pro Arg Lys Phe Leu Phe Leu Ser Leu Phe Ala Leu Ser
            20                  25                  30

Cys Ser Ser Leu Ala Phe Ser Thr Gln Ser Thr Leu Leu Ala Arg Gly
        35                  40                  45

Gly Glu His Glu Gly Gly Gly His His Glu Gly Gln His His Glu Met
    50                  55                  60

Asn His His Glu Asn Gly Gln Arg Phe His Asp Glu Gly His Gly Asn
65                  70                  75                  80

Glu Phe Arg His Gly Tyr Gly His Glu Gly Glu Trp Asn Asn Asn Arg
                85                  90                  95

His Gly Val Phe Val Asp Glu Ser Asn Pro Ala Pro Val Val Val Glu
            100                 105                 110

Pro Ser Asp Asp Val Pro Tyr Asn Ser Ser Asn Gly Tyr Gly Asn
        115                 120                 125

<210>  48
<211>  189
<212>  PRT
<213>  Parachlamydia acanthamoebae

<400>  48

Val Arg Asn Gly Asp Gln Leu Glu Glu Lys Thr Met Arg Lys Leu Leu
1               5                   10                  15

Ala Tyr Gly Leu Cys Leu Phe Leu Leu Thr Ser Cys Asn Ser Ser Glu
            20                  25                  30

Gln Lys Ala Glu Asp Pro His His Ile Lys Lys Ala Asn Ala Val Val
        35                  40                  45

Asn Pro Thr Glu Gly Tyr Lys Thr Trp Gly Asn Ile Thr Phe Thr Glu
    50                  55                  60

Thr Lys Glu Gly Val Gln Ile Val Ala Asn Val Gln Gly Leu Pro Ala
65                  70                  75                  80

Gly Lys His Gly Phe His Ile His Glu Phe Gly Asp Cys Ser Ala Pro
                85                  90                  95

Asp Ala Ser Ser Ala Gly Ala His Tyr Asp Pro Thr His His Lys His
            100                 105                 110

Gly Gly Pro Asp Asp Leu Asp Arg His Val Gly Asp Leu Gly Asn Leu
        115                 120                 125

Glu Ala Asn Glu Asn Gly His Ala Leu Tyr Glu Arg Leu Asp Lys Thr
    130                 135                 140

Ile Thr Leu Asn Gly Pro Asn Ser Ile Val Gly Lys Ser Ile Val Ile
145                 150                 155                 160

His Glu Asp Glu Asp Asp Phe Lys Ser Gln Pro Ala Gly Asn Ser Gly
                165                 170                 175

Lys Arg Val Ala Cys Gly Leu Ile Leu Pro Leu Glu Glu
            180                 185

<210>  49
<211>  219
<212>  PRT
<213>  Parachlamydia acanthamoebae

<400>  49

Thr Leu Leu Asp Ala Met Val Ile Met Glu Lys Phe Glu Lys Gly Gln
1               5                   10                  15

Asp Lys Ile Gln Lys Ile Cys Asp Thr Leu Arg Lys Glu Thr Leu Glu
            20                  25                  30

Pro Ala Arg Gln Glu Ala Gln Glu Ile Ile Ala Glu Ala His Ala Lys
        35                  40                  45

Ala Ala Lys Ile Ile Lys Glu Ala Glu Gln Gln Ala Val Thr Leu His
    50                  55                  60

Ala Gln Ala Arg Lys Ser Ile Glu Gln Glu Arg Asn Val Phe Gln Ser
65                  70                  75                  80

Ser Leu Glu Gln Ala Ala Arg Gln Gly Leu Glu Ser Leu Arg Gln Ser
                85                  90                  95

Ile Glu His Lys Leu Phe Asn Glu Glu Leu Glu Gly Leu Leu Glu Lys
            100                 105                 110

Gln Thr Ala Asp Pro Lys Leu Ile Ala Asn Val Val Asn Ala Ile Val
        115                 120                 125

Glu Ala Val Gln Lys Glu Gly Ile Ser Ser Asn Leu Ser Ala Val Ile
    130                 135                 140

Ser Lys Arg Val Ser Pro Glu Gln Val Asn Ala Leu Leu Leu Glu Asn
145                 150                 155                 160

Val Lys Ser Lys Leu Asn Glu Lys Gly Val Thr Leu Gly Asn Phe Ser
                165                 170                 175

Gly Gly Ala Glu Val Lys Leu His Gly Lys Arg Leu Thr Ile Asp Ile
            180                 185                 190

Thr Asp Gln Thr Leu Lys Glu Leu Leu Ala Gly Tyr Ala Arg Lys Asp
        195                 200                 205

Phe Arg Gln Leu Ile Phe Gly Ile Lys Gly Gly
    210                 215

<210>  50
<211>  277
<212>  PRT
<213>  Parachlamydia acanthamoebae

<400>  50

Gln Met Gln Glu Gln Pro Lys Arg Cys Ser Glu Phe Arg Glu Gly Gly
1               5                   10                  15

Arg Arg Met Ala Thr Ala Ala Asp Lys Ile Ile Lys Lys Glu Leu Asp
            20                  25                  30

Lys Leu Gly Ser Glu Ile Thr Lys Glu Gln Arg Lys Ser Tyr Glu Asp
        35                  40                  45

Val Leu Asn Lys Ile Phe Lys Asp Gly Gln Ser Pro Gln Gln Ala Met
    50                  55                  60

Gly Leu Ser Asp Glu Thr Leu Glu Ala Phe Tyr Gly Tyr Ala Tyr Asn
65                  70                  75                  80

Leu Tyr Gly Ser Gly Asn Tyr Leu Asp Ala Gln Arg Ile Phe Gln Phe
                85                  90                  95

Leu Ile Asn Leu Asp Ala Ser Arg Ser Lys Tyr Ala Leu Gly Leu Ala
            100                 105                 110

Ala Ser Met His Met Gln Lys Asn Tyr Gln Ala Ala Ile Glu Ala Tyr
        115                 120                 125

Met Leu Ala Ala Phe Tyr Asp Gln Glu Asn Pro Val Pro Phe Phe His
    130                 135                 140

Ile Ala Asp Cys Tyr Ile Lys Leu Asp Asn Pro Tyr Gly Ala Leu Leu
145                 150                 155                 160

Ala Leu Asp Asp Ala Ile Arg Leu Gly Phe Tyr Pro Arg Tyr Thr Leu
                165                 170                 175

Leu Arg Ser Lys Ala Ile Leu Met Arg Lys Lys Ile Arg Asp Asp Leu
            180                 185                 190

Gly Leu Pro Asp Thr Pro Pro Pro Ile Lys Glu Tyr Leu Ile Leu Gln

195                 200                 205

Gly Cys Asp Val Glu Gly Leu Asp Ile Glu Lys Leu Phe Leu Pro Gly
    210                 215                 220

Ser Glu Gly Glu Glu Glu Lys Phe Glu Glu Ala Val Lys Lys Ala Glu
225                 230                 235                 240

Gly Thr Ser Gln Glu Arg Glu Lys Ala Gln Met Ile Val Gln Glu Ser
                245                 250                 255

Ser Glu Ser Gly Asp Val Asp Leu Glu Pro Gly Arg Gln Trp Asp Pro
            260                 265                 270

Pro Lys Leu Glu Leu
        275

<210>  51
<211>  277
<212>  PRT
<213>  Parachlamydia acanthamoebae

<400>  51

Gly Val Glu Ile Met Asp Ile Asn Ser Gln Leu Pro Ser Asn His Ser
1               5                   10                  15

Asn Pro Ser Ile Pro Phe Asn Gln Pro Ser Gln Ser Val Glu Phe Asp
            20                  25                  30

Arg Leu Met Ala Gln Ser Gln Ser Asn Asn Leu Pro Phe Leu Thr Gly
        35                  40                  45

Ile Leu Ser Leu Glu Asn Leu Lys Lys Ile Glu Leu Gly Glu Thr Asp
    50                  55                  60

His Val Glu Ile Arg Met Thr His Asp Leu Leu Glu Phe Val Glu Ala
65                  70                  75                  80

Ile Lys Asn Asn Thr Ala Val Met Ile Gln Thr Ser His Ala Asn Thr
                85                  90                  95

Pro Val Phe Lys Phe Ser Lys Glu Ile Asn Arg Leu Ile His Asp Gln
            100                 105                 110

His Val His Glu Phe Thr Val Ser Leu Gly Asp Gln Thr Lys Gly Tyr
        115                 120                 125

His Arg Asn Ala Val Gln Thr Glu Lys Tyr Val Leu Glu Asp Met Arg
    130                 135                 140

Ala Leu Asp Ala His Phe Leu Glu Glu Leu Thr Leu Phe Phe Gln Gly
145                 150                 155                 160

Asn His Gln Asn Ser Gln Asp Ser Tyr Lys Glu Gln Gln Lys Ser Ile
                165                 170                 175

Ala Ser Asp Tyr Gln Lys Ser Gln Gln Arg Leu Gly Lys Met Arg Glu
            180                 185                 190

Arg Ala Leu Leu Asn Gln Glu Lys Glu Glu Asp His Pro Lys Ile Glu
        195                 200                 205

Met Glu Lys Glu Lys Val Pro Leu Pro Glu Lys Ala Val Gly Glu Gly
    210                 215                 220

Thr Leu Phe Asp Gln Ile Val Asp Lys Gln Glu Arg Ala Ser Ile Glu
225                 230                 235                 240

Ala Gln Glu Gln Lys Glu Gln Thr Ala Glu Val Glu Leu Lys Lys Thr
                245                 250                 255

Ala Lys Ala Lys Asn Leu Gln Ile Asp Gly Asp Asn Ile Ser Met Ala
            260                 265                 270

Lys Arg Glu Gly Ser
        275

<210>  52
<211>  584
<212>  PRT
<213>  Parachlamydia acanthamoebae

<400>  52

Asn Ile Lys Tyr Val Ile Asn Leu Tyr Leu Val Val Val Phe Phe Ser
1               5                   10                  15

Cys His Ala Leu Leu Ala Ser Asp Ser Leu Leu Arg Gln Glu Met Val
            20                  25                  30

Glu Asp Leu His Phe Ile Arg Lys Asn Phe Gln Ile Lys Tyr Val Pro
        35                  40                  45

Ala Asp Trp Lys Leu Leu Gln Phe Gly Trp Asn Leu Asn Gln Gln Phe
    50                  55                  60

Asp Asn Ala Glu Ala Glu Ile Thr Ser Leu Ser Ser Pro Thr Leu Lys
65                  70                  75                  80

Asp Tyr His Lys Ile Leu Asn Lys Leu Phe Thr Ser Ala Ala Asp Tyr
                85                  90                  95

His Val Asn Val Ser Phe Tyr Ser Thr Glu Met Ala Ser Leu Pro Phe
            100                 105                 110

His Leu Gln Gly Ala Glu Gly Arg Tyr Phe Ile Thr Trp Ile Asn Glu
        115                 120                 125

Glu Lys Leu Pro Glu Glu Cys Leu Asp Trp Ser Ile Gly Asp Glu Val
    130                 135                 140

Ile Ala Phe Asp Gly Gln Asp Ile His Glu Phe Val Gln Asn Leu Arg
145                 150                 155                 160

Arg Ser Glu His Asp Arg His Asn Glu Lys Thr Asp Gln Arg Phe Ala
                165                 170                 175

Glu Arg Ser Leu Thr Leu Arg Ala Gly Met Phe Ala Glu Asp Val Pro
            180                 185                 190

Gln Gly Asn Val Glu Val Asp Leu Ile His Lys Lys Thr Gly Glu Gln
        195                 200                 205

Lys Ser Tyr Val Leu Thr Trp Thr Tyr His Pro Glu Gln Val Lys Glu
    210                 215                 220

Ile Pro Leu Pro Ser Leu Val Ala Arg Gly Ile Ser Glu Lys Gln Pro
225                 230                 235                 240

Phe Phe Gln Arg Pro Tyr Tyr Lys Lys Met Met Leu Thr Pro Leu Tyr
                245                 250                 255

Ala Lys Phe Asn Gln Pro Glu Ser Arg Val Val Glu Asp Val His Asn
            260                 265                 270

Leu Gly Ala Arg Lys Ser Phe Val Pro Pro Leu Gly Ser Ile Val Trp
        275                 280                 285

Gln Ser Pro Pro Asp Ser Pro Phe His Ala Tyr Ile Cys Glu Thr Pro
    290                 295                 300

Ser Arg Gln Arg Ile Gly Tyr Ile Arg Ile Ala Thr Tyr Asp Gly Thr
305                 310                 315                 320

Asp Asp Glu Val Val Glu Phe Ala Lys Leu Ile Asp Tyr Phe Asn Pro
                325                 330                 335

Arg Thr Gln Ala Leu Val Val Asp Gln Val Asn Asn Pro Gly Gly Ser
            340                 345                 350

Val Phe Tyr Ser Tyr Gly Leu Leu Ser Leu Leu Thr Asp Gln Pro Leu
        355                 360                 365

Glu Leu Pro Lys Tyr Lys Met Ala Ile Thr Gln Glu Glu Val Met Glu
    370                 375                 380

Ala Leu Glu Thr Leu Asp Thr Leu Lys Glu Ile Thr Asn Asp Glu Glu
385                 390                 395                 400

Ala Ile Asp Val Cys Gly Pro Ser Leu His Gly Tyr Ala Val Ser Phe
                405                 410                 415

Glu Leu Val Gln Ser Val Ile Gln Tyr Phe Gln Phe Val Leu Ser Glu
            420                 425                 430

Trp Glu Glu Gly Lys Tyr Val Thr Ser Pro Val His Leu Leu Val Asn
        435                 440                 445

Thr Ile Ala Pro Ser Pro Phe Val Thr Tyr Asp Lys Pro Met Val Val
    450                 455                 460

Leu Val Asn Glu Phe Asp Ile Ser Cys Gly Asp Ser Phe Pro Cys Ile
465                 470                 475                 480

Leu Gln Asp Asn Gln Arg Ala Leu Ile Phe Gly Thr Arg Thr Ala Gly
                485                 490                 495

Ala Gly Gly Thr Val Leu Gly Gly Gly His Pro Asn Arg Leu Gly Ile
            500                 505                 510

Ala Gly Tyr Ser Tyr Thr Ala Ser Leu Leu Glu Arg His Thr Asp Gly
        515                 520                 525

Thr Pro Ile Glu Asn Leu Gly Val Thr Pro Asp Val Ser Tyr Ser Leu
    530                 535                 540

Thr Pro Glu Asp Phe Gln Asn Gly Tyr Lys Gly Tyr Lys Ala Ala Leu
545                 550                 555                 560

Gln Glu Ala Ile Asp Gly Leu Leu Lys Asn Thr Arg Pro Ser Ser Ser
                565                 570                 575

Ala Cys Pro Leu Arg Asp Arg His
            580

<210>  53
<211>  628
<212>  PRT
<213>  Parachlamydia acanthamoebae

<400>  53

Arg Leu Gly Ser Asn Thr Lys Pro Gln Phe Asn Gln Lys Leu Arg Ser
1               5                   10                  15

Asn Ser His Gln Pro Arg Ala Asn Ser Lys Cys Gly Lys Gly Asp Ser
            20                  25                  30

Glu Asn Asp Cys Lys Gln Ile Glu Lys Ser Thr Lys Asp Val Lys Lys
        35                  40                  45

Gly Val Phe Ile Met Arg Lys Gln Leu Gly Val Ile Thr Ser Leu Leu
    50                  55                  60

Phe Leu Cys Ala Thr Ala Phe Leu Gly Ala Ala His Ser Ser Pro Asn
65                  70                  75                  80

Asn Tyr Gly Trp Gly Gly Gly Tyr Pro Val Asp Glu Gln Gln Ser Tyr
                85                  90                  95

Gly Gln Gly Tyr Glu Gln Ser Tyr Ala Pro Gly Pro Gln Gly Ser Tyr
            100                 105                 110

Ala Pro Ala Tyr Glu Gln Ser Ser Arg Pro Arg Gln Ala Cys Pro Gln
        115                 120                 125

Pro Ala Cys Pro Gln Pro Arg Thr Cys Pro Ala Pro Arg Gln Ala Cys
    130                 135                 140

Pro Gln Pro Ala Pro Val Cys Lys Pro Val Cys Val Pro Pro Gln Pro
145                 150                 155                 160

Ala Cys Cys Glu Glu Pro Leu Cys Lys Thr Ile Thr Pro Cys Arg His
                165                 170                 175

Gly Asn Gln Asn Lys Leu Val Cys His Asp Gly Ile Thr Val Thr Ala
            180                 185                 190

Arg Asn Pro Lys Met Cys Met Leu Gly Asp Gln Tyr Pro Leu Glu Phe
        195                 200                 205

Asp Ile Gln Ala Cys Asp Asp Val Cys Asn Val Val Val Thr Thr His
    210                 215                 220

Leu Pro Glu Gly Val Ser Phe Val Arg Ser Val Pro Glu Ala Lys Val
225                 230                 235                 240

Asp Gly Asn Lys Leu Val Trp Glu Ile Gly Ser Met Glu Lys Gly Gln
                245                 250                 255

Cys Val Pro Ala Lys Val Trp Val Lys Cys Glu Cys Glu Gly Glu Leu
            260                 265                 270

Cys Ala Cys Phe Cys Ala Thr Ala Ser Pro Val Arg Phe Cys Ser Leu
        275                 280                 285

Leu Cys Ala Lys Pro Ile Leu Thr Cys His Lys Cys Gly Pro Glu Glu
    290                 295                 300

Val Cys Pro Gly Asp Gln Val Asn Tyr Thr Ile Thr Val Thr Asn Arg
305                 310                 315                 320

Gly Ser Cys Ala Ala Glu Asp Val Val Val Thr Asp Asn Val Pro Glu
                325                 330                 335

Gly Leu Glu His Ser Ser Cys Leu Arg Thr Leu Cys Tyr Lys Leu Gly
            340                 345                 350

Thr Leu Glu Pro Cys Gln Thr Lys Lys Ile Asn Leu Cys Phe Thr Ala
        355                 360                 365

Val Lys Arg Gly Gln Val Cys Asn Thr Ala Val Val Thr Ala Cys Asn
    370                 375                 380

Ala Asp Thr Val Ser Cys Gln Trp Cys Thr Asn Val Cys Lys Glu Cys
385                 390                 395                 400

Ile Glu Ile Thr Lys Val Gly Pro Lys Glu Val Pro Ile Gly Lys Asn
                405                 410                 415

Ala Asp Tyr Gln Ile Thr Val Thr Asn Pro Gly Asp Lys Asp Leu Thr
            420                 425                 430

Glu Val Thr Ile Thr Asp Cys Ala Pro Ser Ser Thr Ser Ile Val Ser
        435                 440                 445

Ala Asn Gly Ala Thr Ile Arg Gly Asn Gln Ala Val Trp Arg Leu Lys
    450                 455                 460

Glu Leu Lys Pro Gly Glu Lys Val Thr Leu Pro Ile Thr Leu Thr Thr
465                 470                 475                 480

Cys Thr Pro Gly Cys Trp Thr Asn Arg Val Thr Val Thr Asn Cys Gln
                485                 490                 495

Asn Cys Thr Ala Cys Ala Glu Ala Thr Thr Arg Trp Arg Gly Arg Pro
            500                 505                 510

Ala Leu Asn Met Cys Ile Thr Asp Thr Glu Asp Pro Ile Cys Ile Gly
        515                 520                 525

Glu Ser Thr Thr Tyr Cys Ile Thr Val Thr Asn Gln Gly Ser Glu Ala
    530                 535                 540

Asp Ser Asn Val Ala Val Val Val Arg Phe Pro Lys Glu Val Thr Pro
545                 550                 555                 560

Thr Ala Ala Val Gly Asp Ser Ala Gly Thr Val Ser Gly Gln Thr Val
                565                 570                 575

Thr Phe Ala Pro Val Ala Asn Phe Ala Pro Arg Gln Thr Leu Lys Phe
            580                 585                 590

Arg Ile Asp Ala Arg Ala Lys Glu Ser Gly Asp Ala Arg Ile Ile Ala
        595                 600                 605

Glu Val Ser Ser Asp Ser Ile Lys Thr Pro Ile Val Gln Gln Glu Ser
    610                 615                 620

Thr Ile Val Asn
625


<210>  54
<211>  395
<212>  PRT
<213>  Parachlamydia acanthamoebae

<400>  54

Pro Asp Trp Asn Ile Gln Gln Gly Leu Ser Asp Ser Arg Val Asn Val
1               5                   10                  15

Thr Leu Glu Ala Met Lys Gln Val Gln Gln Glu Thr Lys Glu Glu Leu
            20                  25                  30

Thr Ala Glu Gln Ala Glu Ser Lys Leu Ala Phe Gln Glu Ser Gln Lys
        35                  40                  45

Glu Ile Ser Asn Pro Phe Ala Ala Arg Leu Thr Lys Lys Glu Lys Pro
    50                  55                  60

Leu Ser Ala Gln Arg Gly Arg Val Gln Lys Ser Gly Lys Met Gly Glu

65                  70                  75                  80

Lys Ala Asp Arg Leu Leu Pro Leu Gln Ala Ile Lys Asp Ala Ala Ser
                85                  90                  95

Gln Phe Gln Gly Arg Asn Pro Glu Leu Lys Ala Gln Ile Leu Val Leu
            100                 105                 110

Leu Arg Glu Gln Ile Lys Pro Asp Asp Ser Lys Glu Glu Ile Leu Arg
        115                 120                 125

Lys Ile Leu Glu Phe Tyr Pro Asp Val Ser Leu Ala Asp Glu Ala Leu
    130                 135                 140

Glu Phe Leu Leu Glu Thr Thr Asp Gly Glu Leu Tyr Gln Lys Ile Lys
145                 150                 155                 160

Glu Ile Lys Glu Glu Phe Ser Gln Gln Asn Asp Arg Glu Ile Ala Ala
                165                 170                 175

Gly Arg Asn Ile Ser Ala Gln Ala Arg Gln Ala Ala Asp Ala Gly Leu
            180                 185                 190

Gly Thr Pro Thr Thr Leu Arg Asp Met Tyr Arg Asp Ile Thr Gly Thr
        195                 200                 205

Pro Arg Asp Ser Thr Ser Leu Phe Gln Glu Leu Ser Gln Arg Tyr Pro
    210                 215                 220

Phe Asn Glu Leu Lys Lys Val Val Ser Phe Leu Leu His Ser Leu Gly
225                 230                 235                 240

Ser Asp Leu Lys Ser Lys Gly Pro Ser Ile Pro Arg Gly Gln Leu His
                245                 250                 255

Arg Leu Ile Thr Glu Thr Arg Ser Leu Gln Ala Ile Leu Gly Val Tyr
            260                 265                 270

Arg Phe Phe Gln Gly Arg Met Asn Leu Met His Ser Leu Phe Ser Lys
        275                 280                 285

Glu Gly Leu Asp Phe Pro Glu Gln Leu Thr Phe Glu Met Met Ala Lys
    290                 295                 300

Gln Phe Met Ser Leu Ala Ala Glu Arg Tyr Pro Ser Ala Asp Lys Val
305                 310                 315                 320

Leu Gln Arg Ala Val Lys Leu Gly Ile Glu Asp Trp Ile Leu Ala Lys

325                 330                 335

Ile Ile Ala Phe Ser Gln Phe Arg Asp Ala Val Arg Glu Val Ala Met
            340                 345                 350

Asn Gln Ile Tyr Lys Ser Leu Gln His Arg Asp Glu Leu Leu Leu Ala
        355                 360                 365

Ile Ile Glu Ala Leu Glu Asp Leu Glu Asp Asp Leu Glu Glu Gln Glu
    370                 375                 380

Glu Lys Glu Gln Glu Glu Glu Glu Asp Lys Glu
385                 390                 395

<210>  55
<211>  427
<212>  PRT
<213>  Parachlamydia acanthamoebae

<400>  55

Lys Lys Trp His Val Ile Leu Tyr Glu Cys Ala Leu Ala Ile Ala Gly
1               5                   10                  15

Val Cys Ala Leu Pro Trp Leu Ile Tyr Gln Ala Ile Phe Lys Lys Lys
            20                  25                  30

Tyr Arg Lys Ser Phe Trp Lys Arg Leu Gly Trp Gly Phe Pro Ala Ile
        35                  40                  45

Gln Lys Glu Gln Arg Thr Val Ile Trp Met His Ala Val Ser Val Gly
    50                  55                  60

Glu Thr Lys Ala Ile Ile Gly Leu Ala Arg Leu Phe Lys Glu Arg Phe
65                  70                  75                  80

Ser Asn Ser Leu Leu Ile Ile Ser Ser Ile Thr Glu Thr Gly His Glu
                85                  90                  95

Glu Ala Gln Arg Ser Leu Pro Phe Ala Asp His His Val Tyr Leu Pro
            100                 105                 110

Phe Asp Phe Gly Trp Met Ile Lys Pro Ile Ile Lys Arg Ile Ser Pro
        115                 120                 125

Asp Ile Val Ile Leu Ser Glu Thr Asp Phe Trp Phe Asn Phe Leu His
    130                 135                 140

Gln Ala Lys Gln Ser Gly Ala Phe Leu Ser Val Val Asn Gly Lys Leu
145                 150                 155                 160

Ser Glu Arg Ser Leu Lys Arg Tyr Gln Met Leu Gly Ser Phe Ser Leu
                165                 170                 175

Phe Ser Leu Phe Asp Leu Phe Cys Leu Gln Asn Thr Gln Tyr Gln Asn
            180                 185                 190

Arg Phe Ser Ser Leu Asn Ile Pro Leu Thr Lys Leu Ser Val Thr Gly
        195                 200                 205

Asn Leu Lys Cys Gly Thr Met Leu Pro Arg Leu Ser Glu Gln Glu Ile
    210                 215                 220

Val Asp Trp Arg Glu Lys Leu Gly Phe Ser Thr Gln Asp Leu Val Leu
225                 230                 235                 240

Thr Ile Gly Ser Thr His Asp Pro Glu Glu Arg Glu Leu Leu Glu Gln
                245                 250                 255

Leu Gln Pro Leu Leu Gln Lys Phe Pro Gln Leu Lys Ile Leu Leu Val
            260                 265                 270

Pro Arg His Pro Glu Arg Cys Glu Gln Val Ala Gln Leu Leu His Gln
        275                 280                 285

Ser Asp Ile Pro Tyr Glu Arg Tyr Ser Ala Asn Ala Ser Lys Glu Gln
    290                 295                 300

Ala Arg Val Leu Leu Val Asp Ala Met Gly Val Leu Leu Lys Cys Tyr
305                 310                 315                 320

Gln Leu Ser Asp Val Ala Ile Val Ala Gly Ser Phe Ile Glu Lys Val
                325                 330                 335

Gly Gly His His Ile Leu Glu Pro Ser Tyr Tyr Glu Val Pro Val Leu
            340                 345                 350

Phe Gly Pro His Met His Ser Gln Arg Glu Phe Glu Ala Leu Cys Leu
        355                 360                 365

Glu His Gly Ala Gly Leu Gln Val Asn Tyr Glu Asp Ile Ala His Ser
    370                 375                 380

Val Glu Met Leu Leu Asn Asp Glu Ala Leu Arg Lys Ser Ile Gly Lys
385                 390                 395                 400

Lys Gly Phe Gln Leu Met Leu Glu Leu Gln Gly Ala His Glu Val Thr
                405                 410                 415

Phe Lys Thr Leu Gln Gln His Leu Lys Arg Ile
            420                 425

<210>  56
<211>  30
<212>  DNA
<213>  Artificial

<220>
<223>  Primer forward Pac1 P. acanthamoebae

<400>  56
cagcatatga attttaaatc ttcctttaca                                        30

<210>  57
<211>  34
<212>  DNA
<213>  Artificial

<220>
<223>  Primer rv Pac1 P. acanthamoebae

<400>  57
gatgagctct taatcgactt taatagaaac gaag                                   34

<210>  58
<211>  33
<212>  DNA
<213>  Artificial

<220>
<223>  Primer forward Pac4 P. acanthamoebae

<400>  58
gatcatatgg ctataagttt atattcaaat caa                                    33

<210>  59
<211>  33
<212>  DNA
<213>  Artificial

<220>
<223>  Primer Pac4 reverse P. acanthamoebae

<400>  59
gatggatcct taggtattag acgttgcagg ttt                                    33

<210>  60
<211>  34
<212>  DNA
<213>  Artificial

<220>
<223>  Primer Pac12 forward P. acanthamoebae

<400>  60
gatcatatga cttacaataa taatatcaat gtat                                   34

<210>  61
<211>  32
<212>  DNA
<213>  Artificial

<220>
<223>  Primer Pac12 reverse P. acanthamoebae

<400>  61
gatggatccc tacggaattt gatcggaacg tt                                    32


<210>  62
<211>  33
<212>  DNA
<213>  Artificial

<220>
<223>  Primer Pac18 forward P. acanthamoebae

<400>  62
gattcatgag tccagatcca atcaaaggtt tcg                                   33


<210>  63
<211>  32
<212>  DNA
<213>  Artificial

<220>
<223>  Primer Pac18 reverse P. acanthamoebae

<400>  63
gatctcgagc tgtgtaagtc cttggaacat ct                                    32


<210>  64
<211>  31
<212>  DNA
<213>  Artificial

<220>
<223>  Primer Pac26 forward P. acanthamoebae

<400>  64
gaacatatga atattaatag tactccacct g                                     31


<210>  65
<211>  35
<212>  DNA
<213>  Artificial

<220>
<223>  Primer Pac26 reverse P. acanthamoebae

<400>  65
gtcggatcct tagtgaatac gataattaaa tgcgc                                 35


<210>  66
<211>  27

<212>  DNA
<213>  Artificial

<220>
<223>  Primer Pac35 forward P. acanthamoebae

<400>  66
gatcatatgc aacgatccct tgggggt                                          27


<210>  67
<211>  31
<212>  DNA
<213>  Artificial

<220>
<223>  Primer Pac35 reverse P. acanthamoebae

<400>  67
gatggatcct tagtattggc taccgcagca a                                     31


<210>  68
<211>  36
<212>  DNA
<213>  Artificial

<220>
<223>  Primer Pac123 forward P. acanthamoebae

<400>  68
gactcatgag taaaaaatgg catgtgattt tatacg                                36


<210>  69
<211>  32
<212>  PRT
<213>  Artificial

<220>
<223>  Primer Pac123 reverse P. acanthamoebae

<400>  69

Gly Ala Cys Cys Thr Cys Gly Ala Gly Ala Ala Thr Gly Cys Gly Thr
1               5                   10                  15


Thr Thr Ala Ala Gly Ala Thr Gly Cys Thr Gly Thr Thr Gly Cys Ala
            20                  25                  30